# EUROPÄISCHE PATENTSCHRIFT

(11) **EP 1 654 253 B1**
(45) Veröffentlichungstag und Bekanntmachung des Hinweises auf die Patenterteilung: **03.01.2007**
(21) Anmeldenummer: 04763915.8
(22) Anmeldetag: 09.08.2004
(51) Int. Cl.: C07D 403/04, A61K 31/404

(54) **SUBSTITUIERTE 3-PYRROLIDIN-INDOL-DERIVATE**
SUBSTITUTED 3-PYRROLIDIN-INDOLE DERIVATIVES
DERIVES DE 3-PYRROLIDIN-INDOLE SUBSTITUE

(30) Priorität: 13.08.2003 DE 10337184
(43) Veröffentlichungstag der Anmeldung: 10.05.2006
(73) Patentinhaber: Grünenthal GmbH, 52078 Aachen (DE)
(72) Erfinder: SUNDERMANN, Corinna, 52066 Aachen (DE); SUNDERMANN, Bernd, 52066 Aachen (DE); BIJSTERVELD, Edward, NL-6503 CB Nijmegen (NL)
(86) Internationale Anmeldenummer: PCT/EP2004/008889
(87) Internationale Veröffentlichungsnummer: WO 2005/019208

(56) Entgegenhaltungen:
- WO-A-02/051837
- WO-A-20/04043949
- DE-A- 19 615 232
- LISA MATZEN, CHRISTOPH VAN AMSTERDAM, WILFRIED RAUTENBERG, HARTMUT E. GREINER, JÜRGEN HARTING ET AL.: "5-HT Reuptake Inhibitors with 5-HT1B/1D Antagonistic Activity: A New Approach toward Efficient Antidepressants" J. MED. CHEM., Bd. 43, 2000, Seiten 1149-1157, XP002308490

## Beschreibung

Die Erfindung betrifft substituierte 3-Pyrrolidin-Indol-Derivate, Verfahren zu deren Herstellung, Arzneimittel enthaltend diese Verbindungen sowie die Verwendung dieser Stoffe zur Herstellung von Arzneimitteln, vorzugsweise zur Behandlung von Schmerzen und/oder Depressionen.

Depression ist eine Störung der Affektivität, bei der ein depressives Syndrom im Vordergrund steht, wobei depressiv mit Verstimmung verbunden bzw. traurig gestimmt heißt. Die zur Therapie verwendeten Antidepressiva sind auch wichtige Adjuvantien für die Schmerztherapie (in Analgesics - from Chemistry and Pharmacology to Clinical Application, 265-284, Wiley VCH, 2002), insbesondere bei chronischen Schmerzzuständen, da die andauernde Schmerzbelastung zu einer depressiven Verstimmung der Patienten führen kann. Dies ist besonders häufig bei Krebs-Schmerzpatienten der Fall (Berard, Int. Med. J. 1996, 3/4, 257-259). Da es bisher keine Schmerzmittel mit einer klinisch relevanten antidepressiven Wirkkomponenten gibt, müssen die Antidepressiva als Zusatzmedikation zur Analgetika-Gabe hinzugefügt werden. Da chronische Schmerzpatienten häufig eine Vielzahl von verschiedenen Medikamenten benötigen, führt die zusätzliche Gabe des Antidepressivums zu einer weiteren Belastung des Organismus. Aus diesem Grund und zur Erhöhung der Akzeptanz wäre eine analgetisch wirksame Substanz mit einer antidepressiven Wirkkomponente von besonderem Vorteil.
Grundlage der antidepressiven Wirkung ist die Wiederaufnahmehemmung von Serotonin.
Aus der Strukturklasse der 3-Pyrrolidin-Indole sind bereits verschiedene Derivate aus der Literatur bekannt.
WO9311106 beschreibt 5-substituierte 3-Pyrrolidin-Indol-Derivate, wobei der Rest am Pyrrolidin-Stickstoff eine Alkylkette oder Aryl bedeutet.
WO02051837 beschreibt 3-Pyrrolidin-Indol-Derivate als 5-HT₆-Liganden, bei denen der Rest am Pyrrolidin-Stickstoff eine Alkylkette, in der ein Kohlenstoff auch durch Sauerstoff oder Stickstoff ersetzt sein kann, Cycloalkyl, Cycloheteroaryl, Aryl oder Heteroaryl bedeutet.
WO9410171 offenbart 3-Pyrrolidin-Indol-Derivate mit Alkyl, Aryl und Alkylaryl als Stickstoffsubstituenten des Pyrrolidin als analgetisch wirksame Verbindungen, die jedoch in 5-Position substituiert sind.
US3109844 offenbart 3-Pyrrolidin-Indol-Derivate, die einen substituierten Alkyrest am Pyrrolidin-Stickstoff tragen.
W002079190 beschreibt 3-Pyrrolidin-Indol-Derivate, die einen speziell substituierten Alkylrest (einen gesättigten Stickstoffheterocyclus) am Pyrrolidin-Stickstoff tragen. Bei diesen 3-Pyrrolidin-Indol-Derivaten handelt es sich um Chemokin-Antagonisten. WO02079151 beschreibt 3-Pyrrolidin-Indol-Derivate, die am Pyrrolidin-Stickstoff eine Alkylkette, die durch einen cyclischen Rest unterbrochen sein kann, wobei es sich um Chemokin-Antagonisten handelt.
WO0214317 offenbart 3-Pyrrolidin-Indol-Derivate, bei denen ein Pyrazolrest über eine Alkylkette, die auch Heteroatome enthalten kann, mit dem Pyrrolidin-Stickstoff verknüpft ist.
WO0143740 offenbart 3-Pyrrolidin-Indol-Derivate, bei denen ein Arylrest über eine Alkylkette, die durch ein Heteroatom unterbrochen ist, mit dem Pyrrolidin-Stickstoff verknüpft ist.
WO9958525 und W09910346 offenbaren 3-Pyrrolidin-Indol-Derivate, bei denen ein Dihydroindolrest oder ein 3,4-Dihydro-1H-benzo[1,2,6]thiadiazin-2,2-dioxid über eine Alkylkette mit dem Pyrrolidin-Stickstoff verknüpft ist. US5891875 beschreibt 3-Pyrrolidin-Indol-Derivate, bei denen ein substituierter Morpholinrest über eine Methylcarbonylgruppe mit dem Pyrrolidin-Stickstoff verknüpft ist.
US5792760 offenbart 3-Pyrrolidin-Indol-Derivate, bei denen ein N-Benzyl-N-(3-1H-indol-3-yl-propionyl)-acetamid-Rest über eine Ethylamin-Gruppe mit dem Pyrrolidin-Stickstoff verknüpft ist.
WO9740038 offenbart Verbindungen, bei denen der Pyrrolidin-Stickstoff mit einer Alkylkette oder einem Cycloalkylring, einem Phenylrest oder einem Stickstoffsubstituenten über eine Alkylaminkette, eine Alkylamidkette oder eine Amidfunktion verknüpft ist.

Die Mehrzahl der aufgeführten Verbindungen sind als Serotonin-Rezeptor-Liganden oder als Serotonin-Wiederaufnahme-Hemmer beschrieben.

Die der Erfindung zugrundeliegende Aufgabe bestand in dem zur Verfügung stellen einer neuen Strukturklasse antidepressiv wirksamer Substanzen, die sich darüber hinaus insbesondere zur Behandlung von Schmerzen eignen.

Überraschenderweise wurde nun gefunden, dass substituierte 3-Pyrrolidin-Indol - Derivate der allgemeinen Formel I, die zusätzlich zur Serotonin-Wiederaufnahme-Hemmung auch eine Noradrenalin-Wiederaufnahme-Hemmung sowie eine Natriumkanal-Bindung (BTX-Bindungsstelle) aufweisen, eine ausgeprägte antidepressive und auch analgetische Wirkung besitzen.

Gegenstand der Erfindung sind daher substituierte 3-Pyrrolidin-Indol - Derivate der allgemeinen Formel I, 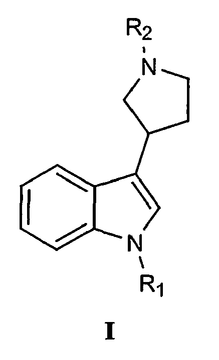 worin
der Rest R¹ für H; C₁₋₈-Alkyl, jeweils gesättigt oder ungesättigt, verzweigt oder unverzweigt, unsubstituiert oder einfach oder mehrfach substituiert; oder über C₁₋₃-Alkyl gebundenes Aryl, C₃₋₈-Cycloalkyl oder Heteroaryl, jeweils einfach oder mehrfach substituiert oder unsubstituiert; CH₂CONR³R⁴ steht;
der Rest R² für COR⁵; SO2R⁶; CSNHR⁷; CONHR⁸ steht
die Reste R³ und R⁴ unabhängig voneinander für C₁₋₈ Alkyl, jeweils gesättigt oder ungesättigt, verzweigt oder unverzweigt, unsubstituiert oder einfach oder mehrfach substituiert stehen;
oder
die Reste R³ und R⁴ zusammen CH₂CH₂OCH₂CH₂, oder (CH₂)₃₋₆ bedeuten;
der Rest R⁵ für C₁₋₈-Alkyl, verzweigt oder unverzweigt, gesättigt oder ungesättigt, substituiert oder unsubstituiert; Aryl, substituiert oder unsubstituiert; Heteroaryl, substituiert oder unsubstituiert; Cycloalkyl oder Heterocyclyl, jeweils gesättigt oder ungesättigt, substituiert oder unsubstituiert;
oder 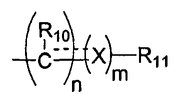 mit n = 1, 2, 3; m = 0, 1; X = O, NH und 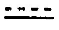 in der Bedeutung einer Einfachbindung oder einer Doppelbindung, steht
der Rest R⁶ für Aryl, jeweils substituiert oder unsubstituiert, Heteroaryl, jeweils substituiert oder unsubstituiert, C₁₋₈- Alkyl, jeweils gesättigt oder ungesättigt, verzweigt oder unverzweigt, unsubstituiert oder einfach oder mehrfach substituiert; C₃₋₈ Cycloalkyl, substituiert oder unsubstituiert, gesättigt oder ungesättigt; oder über C₁₋₃-Alkyl gebundenes Aryl, C₃₋₈-Cycloalkyl oder Heteroaryl, jeweils einfach oder mehrfach substituiert oder unsubstituiert steht
der Rest R⁷ für Aryl, jeweils substituiert oder unsubstituiert, Heteroaryl, jeweils substituiert oder unsubstituiert, C₁₋₈- Alkyl, jeweils gesättigt oder ungesättigt, verzweigt oder unverzweigt, unsubstituiert oder einfach oder mehrfach substituiert; C₃₋₈ Cycloalkyl, substituiert oder unsubstituiert, gesättigt oder ungesättigt; oder über C₁-₃-Alkyl gebundenes Aryl, C₃₋₈-Cycloalkyl oder Heteroaryl, jeweils einfach oder mehrfach substituiert oder unsubstituiert steht,
der Rest R⁸ für C₁₋₈-Alkyl, verzweigt oder unverzweigt, substituiert oder unsubstituiert, gesättigt oder ungesättigt; C₃₋₈-Cycloalkyl, substituiert oder unsubstituiert, gesättigt oder ungesättigt; trifluormethyl- oder nitrosubstituiertes Phenyl, Pyrrolyl, Indolyl, Furyl, Benzofuranyl, Thienyl (Thiophenyl), Benzothienyl, Pyrazolyl, Imidazolyl, Thiazolyl, Oxazolyl, Chromenyl, Oxadiazolyl, Isoxazoyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, steht,
R¹⁰ für H; C₁₋₈-Alkyl, verzweigt oder unverzweigt, substituiert oder unsubstituiert, gesättigt oder ungesättigt; Aryl oder Heteroaryl, jeweils substituiert oder unsubstituiert, Benzyl oder Phenethyl, jeweils substituiert oder unsubstituiert, steht
R¹¹ für H; C₁₋₈-Alkyl verzweigt oder unverzweigt, substituiert oder unsubstituiert, gesättigt oder ungesättigt; Aryl oder Heteroaryl, jeweils substituiert oder unsubstituiert, oder über C₁₋₃-Alkyl gebundenes Aryl, C₃₋₈-Cycloalkyl oder Heteroaryl, jeweils einfach oder mehrfach substituiert oder unsubstituiert steht, mit der Maßgabe, dass wenn R² Cyclohexyl bedeutet, der Cyclohexylring nicht in 4-Position mit NR^{x}R^{y} und R^{z} substituiert sein darf,
wobei R^{x} und R^{y} unabhängig voneinander für H; C₁₋₈-Alkyl oder C₃₋₈-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, stehen,
oder die Reste R^{x} und R^{y} zusammen einen Ring bilden und CH₂CH₂OCH₂CH₂, CH₂CH₂NRⁿCH₂CH₂ oder (CH₂)₃₋₆ bedeuten, wobei Rⁿ H; C₁₋₈-Alkyl oder C₃₋₈-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, bedeutet;
und
R^{Z} für C₁₋₈-Alkyl oder C₃₋₈-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte C₁₋₄-Alkyl-Gruppe gebundenem Aryl, C₃₋₈-Cycloalkyl oder Heteroaryl, jeweils unsubstituiert oder einfach oder mehrfach substituiert, steht;

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Bevorzugt sind substituierte 3-Pyrrolidin-Indol-Derivate, worin der Rest R¹ für H; C₁₋₈-Alkyl, jeweils gesättigt oder ungesättigt, verzweigt oder unverzweigt, unsubstituiert oder einfach oder mehrfach substituiert; Benzyl oder Methylnaphthyl, jeweils einfach oder mehrfach substituiert oder unsubstituiert, besonders bevorzugt Benzyl unsubstituiert oder einfach oder mehrfach mit CF₃, Br, F, CN, OCH₃ oder CH₃ substituiert; CH₂CONR³R⁴ steht und die übrigen Reste die oben angegebene Bedeutung haben.

Weiterhin bevorzugt sind substituierte 3-Pyrrolidin-Indol-Derivate worin der Rest R² für COR⁵ steht und R⁵ 4-Propylphenyl, 3,4-Dimethoxyphenyl, 2-Methyl-4-trifluormethylphenyl-3-pyridin, Ethenylphenyl, 2,3-Difluorphenyl, 4-tert.-Butylphenyl, 2-Ethoxyphenyl, 3-Fluor-4-trifluormethylphenyl, 2,3-Dimethylphenyl, Phenoxyethyl, Phenoxymethyl, 3,4-Dichlorphenyl, 4-Trifluormethylsulfanylphenyl, 2,5-Dimethoxyphenyl, 2-Chlor-4-nitrophenyl, 2-Chlorphenyl, 4-Methyl-N-phenethyl-benzylsulfonamid, 2-Chlor-5-fluor-3-methylphenyl, 3-(2-Chlorphenyl)-5-methylisoxazol, 5-tert.-Butyl-2-methylfuran, Benzo[1,2,5]oxadiazol, Phenylpropyl, 2-Methylsulfanyl-3-pyridin, 2-Chlor-5-trifluormethylphenyl, Methoxymethyl, 4-Methylphenyl, Biphenyl, 4-Chlorbenzyl, 2,3,4,5,6-Pentafluorphenyl, 3-Chlorphenyl, 4-Trifluormethylphenyl, 2,6-Dlfluor-3-methylphenyl, 2-Methylphenyl, 4-Fluorphenyl, 6-Chlorchromen, 2-Chlor-6-fluorphenyl, Ethylcyclopentyl, 1-(4-Chlorphenyl)-5-trifluormethylpyrazol, 2,4-Dichlorphenyl, 2,3-Dimethylphenyl, 3-Nitro-4-methylphenyl, 4-Brom-3-methylphenyl, (4-Chlorphenoxy)methyl, 4-Chlorphenyl, 5-Methylisoxazol, 3-Methoxyphenyl, 2-Chlorphenylethenyl, 2-Chlor-4-fluor-3-methylphenyl, 2-Fluorphenyl, 3-Difluormethylsulfanylphenyl, 2-Fluor-3-chlorphenyl, Cyclopropylphenyl, 1-Phenyl-5-propylpyrazol, 2,6-Difluorphenyl, Benzo[1,3]-dioxol, 4-Bromphenyl, 3-Chlorthiophenyl, 2-, 3- oder 4-Pyridin, Phenyl oder 3,4-Difluorphenyl bedeutet und die übrigen Reste die oben angegebene Bedeutung haben.

Ebenfalls bevorzugt sind substituierte 3-Pyrrolidin-Indol-Derivate worin der Rest R² für SO₂R⁶ steht und R⁶ 2,4,6-Dimethylphenyl, 4-Nitrophenyl, Benzyl, 4-Propylphenyl, 4-Chlorphenyl, 2-Trifluormethylphenyl, 2,4-Difluorphenyl, 3-Trifluormethylphenyl bedeutet und die übrigen Reste die oben angegebene Bedeutung haben.

Weiterhin bevorzugt sind substituierte 3-Pyrrolidin-Indol-Derivate worin der Rest R² für CSNHR⁷ steht und der Rest R⁷ 3-Trifluormethylphenyl bedeutet und die übrigen Reste die oben angegebene Bedeutung haben.

Außerdem bevorzugt sind substituierte 3-Pyrrolidin-Indol-Derivate, worin der Rest R² für CONHR⁸ steht und der Rest R⁸ 3,4-Dichlorbenzyl bdeutet und die übrigen Reste die oben angegebene Bedeutung haben.

Besonders bevorzugt sind substituierte 3-Pyrrolidin-Indol-Derivate ausgewählt aus der Gruppe
- (4-Propyl-phenyl)-{3-[1-(4-trifluormethyl-benzyl)-1H-indol-3-yl]-pyrrolidin-1-yl}-methanon
- (3,4-Dimethoxy-phenyl)-{3-[1-(3-methyl-butyl)-1H-indol-3-yl]-pyrrolidin-1-yl}-methanon
- [3-(1-Butyl-1H-indol-3-yl)-pyrrolidin-1-yl]-(2-methyl-6-trifluormethyl-pyridin-3-yl)-methanon
- 1-{3-[1-(3-Methoxy-benzyl)-1H-indol-3-yl]-pyrrolidin-1-yl}-3-phenyl-propenon
- (2,3-Difluor-phenyl)-{3-[1-(4-trifluormethyl-benzyl)-1H-indol-3-yl]-pyrrolidin-1-yl}-methanon
- (4-tert-Butyl-phenyl)-{3-[1-(3-methyl-butyl)-1H-indol-3-yl]-pyrrotidin-1-yl}-methanon
- {3-[1-(4-Brom-benzyl)-1H-indol-3-yl]-pyrrolidin-1-yl}-(4-propyl-phenyl)-methanon
- (2-Ethoxy-phenyl)-{3-[1-(3-methoxy-benzyl)-1H-indol-3-yl]-pyrrolidin-1-yl}-methanon
- [3-(1-Ethyl-1H-indol-3-yl)-pyrrolidin-1-yl]-(3-fluor-4-trifluormethyl-phenyl)-methanon
- (2,3-Dimethyl-phenyl)-[3-(1-ethyl-1H-indol-3-yl)-pyrrolidin-1-yl]-methanon
- 1-{3-[1-(3-Methoxy-benzyl)-1H-indol-3-yl]-pyrrolidin-1-yl}-2-phenoxy-propan-1-on
- 2-(3-Chlor-phenoxy)-1-[3-(1-ethyl-1H-indol-3-yl)-pyrrolidin-1-yl]-ethanon
- 4-{3-[1-(2-Phenoxy-propionyl)-pyrrolidin-3-yl]-indol-1-ylmethyl}-benzonitril
- N,N-Dimethyl-2-{3-[1-(2,4,6-trimethyl-benzensulfonyl)-pyrrolidin-3-yl]-indol-1-yl}-acetamid
- (3,4-Dichlor-phenyl)-[3-(1-ethyl-1H-indol-3-yl)-pyrrolidin-1-yl]-methanon
- N,N-Dimethyl-2-{3-[1-(3-trifluormethyl-phenylthiocarbamoyl)-pyrrolidin-3-yl]-indol-1-yl}-acetamid
- 3-(1-Dimethylcarbamoylmethyl-1H-indol-3-yl)-pyrrolidin-1-carbonsäure 3,4-dichlor-benzylamid
- [3-(1H-Indol-3-yl)-pyrrolidin-1-yl]-(4-trifluormethylsulfanyl-phenyl)-methanon
- 2-(3-{1-[2-(2,5-Dimethoxy-phenyl)-acetyl]-pyrrolidin-3-yl}-indol-1-yl)-N,N-dimethyl-acetamid
- (2-Chlor-4-nitro-phenyl)-{3-[1-(3-fluor-benzyl)-1H-indol-3-yl]-pyrrolidin-1-yl}-methanon
- N,N-Dimethyl-2-{3-[1-(pyridin-2-carbonyl)-pyrrolidin-3-yl]-indol-1-yl}-acetamid
- Cyclobutyl-[3-(1H-indol-3-yl)-pyrrolidin-1-yl]-methanon
- N,N-Diethyl-2-[3-(1-pentanoyl-pyrrolidin-3-yl)-indol-1-yl]-acetamid
- 3-[1-(4-Nitro-benzensulfonyl)-pyrrolidin-3-yl]-1H-indol
- 2-[3-(1-Cyclopentanecarbonyl-pyrrolidin-3-yl)-indol-1-yl]-N,N-diethyl-acetamid
- 1-[3-(1-Methyl-1H-indol-3-yl)-pyrrolidin-1-yl]-3-phenyl-propenon
- (2-Chlor-phenyl)-[3-(1H-indol-3-yl)-pyrrolidin-1-yl]-methanon
- [3-(1-Ethyl-1H-indol-3-yl)-pyrrolidin-1-yl]-pyridin-3-yl-methanon
- N,N-Dimethyl-2-(3-{1-[3-phenyl-2-(toluene-4-sulfonylamino)-propionyl]-pyrrolidin-3-yl}-indol-1-yl)-acetamid
- N,N-Dimethyl-2-[3-(1-phenylmethanesulfonyl-pyrrolidin-3-yl)-indol-1-yl]-acetamid
- 4-{3-[1-(2-Chlor-6-fluor-3-methyl-benzoyl)-pyrrolidin-3-yl]-indol-1-ylmethyl}-benzonitril
- [3-(2-Chlor-phenyl)-5-methyl-isoxazol-4-yl]-[3-(1-ethyl-1H-indol-3-yl)-pyrrolidin-1-yl]-methanon
- 2-{3-[1-(5-tert-Butyl-2-methyl-furan-3-carbonyl)-pyrrolidin-3-yl]-indol-1-ylmethyl}-benzonitril
- 4-{3-[1-(Benzo[1,2,5]oxadiazole-5-carbonyl)-pyrrolidin-3-yl]-indol-1-ylmethyl}-benzonitril
- 1-[3-(1-Ethyl-1H-indol-3-yl)-pyrrolidin-1-yl]-3-phenyl-propan-1-on
- 1-{3-[1-(3-Methoxy-benzyl)-1H-indol-3-yl]-pyrrolidin-1-yl}-3-phenyl-propan-1-on
- [3-(1-Methyl-1H-indol-3-yl)-pyrrolidin-1-yl]-(2-methylsulfanyl-pyridin-3-yl)-methanon
- (2-Chlor-5-trifluormethyl-phenyl)-[3-(1-ethyl-1H-indol-3-yl)-pyrrolidin-1-yl]-methanon
- (2-Chlor-4-nitro-phenyl)-{3-[1-(4-trifluormethyl-benzyl)-1H-indol-3-yl]-pyrrolidin-1-yl}-methanon
- (2-Chlor-pyridin-3-yl)-[3-(1-methyl-1H-indol-3-yl)-pyrrolidin-1-yl]-methanon
- 2-Methoxy-1-{3-[1-(3-methoxy-benzyl)-1H-indol-3-yl]-pyrrolidin-1-yl}-ethanon
- {3-[1-(3-Fluor-benzyl)-1H-indol-3-yl]-pyrrolidin-1-yl}-(2-methyl-6-trifluormethyl-pyridin-3-yl)-methanon
- {3-[1-(3-Methoxy-benzyl)-1H-indol-3-yl]-pyrrolidin-1-yl}-p-tolyl-methanon
- (2-Methylsulfanyl-pyridin-3-yl)-[3-(1-prop-2-ynyl-1H-indol-3-yl)-pyrrolidin-1-yl]-methanon
- Biphenyl-4-yl-[3-(1-methyl-1H-indol-3-yl)-pyrrolidin-1-yl]-methanon
- 2-(4-Chlor-phenyl)-1-{3-[1-(3-methoxy-benzyl)-1H-indol-3-yl]-pyrrolidin-1-yl}-ethanon
- [3-(1-Methyl-1H-indol-3-yl)-pyrrolidin-1-yl]-pentafluorphenyl-methanon
- [3-(1-Ethyl-1H-indol-3-yl)-pyrrolidin-1-yl]-(4-trifluormethyl-phenyl)-methanon
- N,N-Dimethyl-2-{3-[1-(4-propyl-benzensulfonyl)-pyrrolidin-3-yl]-indol-1-yl}-acetamid
- (2-Chlor-4-nitro-phenyl)-{3-[1-(3-methoxy-benzyl)-1H-indol-3-yl]-pyrrolidin-1-yl}-methanon
- 3-{3-[1-(4-Chlor-benzensulfonyl)-pyrrolidin-3-yl]-indol-1-ylmethyl}-benzonitril
- 1-{3-[1-(3-Methoxy-benzyl)-1H-indol-3-yl]-pyrrolidin-1-yl}-3,3-dimethyl-butan-1-on
- 2-(4-Chlor-phenyl)-1-[3-(1-methyl-1H-indol-3-yl)-pyrrolidin-1-yl]-ethanon
- (2,6-Difluor-3-methyl-phenyl)-{3-[1-(3-methoxy-benzyl)-1H-indol-3-yl]-pyrrolidin-1-yl}-methanon
- [3-(1-Ethyl-1H-indol-3-yl)-pyrrolidin-1-yl]-o-tolyl-methanon
- [3-(1-Ethyl-1H-indol-3-yl)-pyrrolidin-1-yl]-(4-fluor-phenyl)-methanon
- [3-(1-Butyl-1H-indol-3-yl)-pyrrolidin-1-yl]-(6-chlor-2H-chromen-3-yl)-methanon
- (2-Chlor-6-fluor-phenyl)-[3-(1-naphthalen-2-ylmethyl-1H-indol-3-yl)-pyrrolidin-1-yl]-methanon
- 2-{3-[1-(3-Cyclopentyl-propionyl)-pyrrolidin-3-yl]-indol-1-yl}-N,N-dimethyl-acetamid
- [3-(2-Chtor-phenyl)-5-methyl-isoxazol-4-yl]-{3-[1-(3-methoxy-benzyl)-1H-indol-3-yl]-pyrrolidin-1-yl}-methanon
- [1-(4-Chlor-phenyl)-5-trifluormethyl-1H-pyrazol-4-yl]-{3-[1-(3-fluor-benzyl)-1H-indol-3-yl]-pyrrolidin-1-yl}-methanon
- [3-(1-Allyl-1H-indol-3-yl)-pyrrolidin-1-yl]-p-tolyl-methanon
- {3-[1-(4-Fluor-benzyl)-1H-indol-3-yl]-pyrrolidin-1-yl}-pyridin-2-yl-methanon
- (3,4-Dichtor-phenyl)-{3-[1-(4-triftuormethyl-benzyl)-1H-indol-3-yl]-pyrrolidin-1-yl}-methanon
- 1-Butyl-3-[1-(2,5-dimethoxy-benzensulfonyl)-pyrrolidin-3-yl]-1H-indol
- 1-[3-(1-Allyl-1H-indol-3-yl)-pyrrolidin-1-yl]-3,3-dimethyl-butan-1-on
- 4-{3-[1-(2-Methoxy-acetyl)-pyrrolidin-3-yl]-indol-1-ylmethyl}-benzonitril
- (2,4-Difluor-phenyl)-[3-(1-ethyl-1H-indol-3-yl)-pyrrolidin-1-yl]-methanon
- (2,3-Dimethyl-phenyl)-{3-[1-(4-trifluormethyl-benzyl)-1H-indol-3-yl]-pyrrolidin-1-yl}-methanon
- [3-(1-Butyl-1H-indol-3-yl)-pyrrolidin-1-yl]-(4-methyl-3-nitro-phenyl)-methanon
- (4-Brom-3-methyl-phenyl)-{3-[1-(3-methoxy-benzyl)-1H-indol-3-yl]-pyrrolidin-1-yl}-methanon
- 1-[3-(1-Butyl-1H-indol-3-yl)-pyrrolidin-1-yl]-2-(4-chlor-phenoxy)-ethanon
- 2-(4-Chlor-phenyl)-1-{3-[1-(3-fluor-benzyl)-1H-indol-3-yl]-pyrrolidin-1-yl}-ethanon
- (4-Chlor-phenyl)-{3-[1-(4-trifluormethyl-benzyl)-1H-indol-3-yl]-pyrrolidin-1-yl}-methanon
- [3-(1-Benzyl-1H-indol-3-yl)-pyrrolidin-1-yl]-(5-methyl-isoxazol-3-yl)-methanon
- 1-[3-(1-Benzyl-1H-indol-3-yl)-pyrrolidin-1-yl]-2-(4-chlor-phenoxy)-ethanon
- [3-(1-Naphthalen-2-ylmethyl-1H-indol-3-yl)-pyrrolidin-1-yl]-(4-trifluormethylphenyl)-methanon
- (3-Methoxy-phenyl)-{3-[1-(4-trifluormethyl-benzyl)-1H-indol-3-yl]-pyrrolidin-1-yl}-methanon
- 1-[3-(1-Butyl-1H-indol-3-yl)-pyrrolidin-1-yl]-3-(2-chlor-phenyl)-propenon
- 1-[3-(1-Methyl-1H-indol-3-yl)-pyrrolidin-1-yl]-2-phenyl-butan-1-on
- (2-Chlor-6-fluor-3-methyl-phenyl)-[3-(1-methyl-1H-indol-3-yl)-pyrrolidin-1-yl]-methanon
- 2-{3-[1-(3-Methoxy-benzoyl)-pyrrolidin-3-yl]-indol-1-yl}-N,N-dimethyl-acetamid
- 2-{3-[1-(2-Fluor-benzoyl)-pyrrolidin-3-yl]-indol-1-yl}-N,N-dimethyl-acetamid
- (3-Difluormethylsulfanyl-phenyl)-[3-(1-naphthalen-2-ylmethyl-1H-indol-3-yl)-pyrrolidin-1-yl]-methanon
- Benzo[1,2,5]oxadiazol-5-yl-{3-[1-(3-fluor-benzyl)-1H-indol-3-yl]-pyrrolidin-1-yl}-methanon
- o-Tolyl-{3-[1-(4-trifluormethy)-benzyl)-1H-indol-3-yl]-pyrrolidin-1-yl}-methanon
- N,N-Dimethyl-2-{3-[1-(2-trifluormethyl-benzensulfonyl)-pyrrolidin-3-yl]-indol-1-yl}-acetamid
- 1-[3-(1-Butyl-1H-indol-3-yl)-pyrrolidin-1-yl]-2-phenoxy-propan-1-on
- (6-Chlor-2-fluor-3-methyl-phenyl)-[3-(1-naphthalen-2-ylmethyl-1H-indol-3-yl)-pyrrolidin-1-yl]-methanon
- [3-(1-Allyl-1H-indol-3-yl)-pyrrolidin-1-yl]-(2-methyl-6-trifluormethyl-pyridin-3-yl)-methanon
- [3-(1-Butyl-1H-indol-3-yl)-pyrrolidin-1-yl]-(4-propyl-phenyl)-methanon
- 2-{3-[1-(4-Chlor-benzoyl)-pyrrolidin-3-yl]-indol-1-yl}-N,N-dimethyl-acetamid
- 1-{3-[1-(3-Fluor-benzyl)-1H-indol-3-yl]-pyrrolidin-1-yl}-3-phenyl-propan-1-on
- {3-[1-(2-Fluor-benzyl)-1H-indol-3-yl]-pyrrolidin-1-yl}-(1-phenyl-5-propyl-1H-pyrazol-4-yl)-methanon
- 3-{3-[1-(5-tert-Butyl-2-methyl-furan-3-carbonyl)-pyrrolidin-3-yl]-indol-1-ylmethyl}-benzonitril
- 3-[1-(2,4-Difluor-benzensulfonyl)-pyrrolidin-3-yl]-1-(2-fluor-benzyl)-1H-indol
- 2-{3-[1-(2,6-Difluor-benzoyl)-pyrrolidin-3-yl]-indol-1-yl}-N,N-dimethyl-acetamid
- 1-[3-(1-Butyl-1H-indol-3-yl)-pyrrolidin-1-yl]-butan-1-on
- 4-{3-[1-(2-Propyl-pentanoyl)-pyrrolidin-3-yl]-indol-1-ylmethyl}-benzonitril
- 2-(4-Chlor-phenoxy)-1-[3-(1-prop-2-ynyl-1H-indol-3-yl)-pyrrolidin-1-yl]-ethanon
- [3-(1-Methyl-1H-indol-3-yl)-pyrrolidin-1-yl]-pyridin-3-yl-methanon
- 3-(3-[1-(Benzo[1,3]dioxole-5-carbonyl)-pyrrolidin-3-yl]-indol-1-ylmethyl)-benzonitril
- [3-(1-Butyl-1H-indol-3-yl)-pyrrolidin-1-yl]-(2-chlor-5-trifluormethyl-phenyl)-methanon
- (3-Chlor-phenyl)-{3-[1-(4-trifluormethyl-benzyl)-1H-indol-3-yl]-pyrrolidin-1-yl}-methanon
- 2-{3-[1-(4-Brom-benzoyl)-pyrrolidin-3-yl]-indol-1-yl}-N,N-dimethyl-acetamid
- 3-Phenyl-1-[3-(1-prop-2-ynyl-1H-indol-3-yl)-pyrrolidin-1-yl]-propenon
- 3-{3-[1-(3-Chlor-2-fluor-benzoyl)-pyrrolidin-3-yl]-indol-1-ylmethyl}-benzonitril
- 3-{3-[1-(2-Phenyl-cyclopropanecarbonyl)-pyrrolidin-3-yl]-indol-1-ylmethyl}-benzonitril
- 3-{3-[1-(3-Chlor-thiophen-2-carbonyl)-pyrrolidin-3-yl]-indol-1-ylmethyl}-benzonitril
- 1-Prop-2-ynyl-3-[1-(2-trifluormethyl-benzensulfonyl)-pyrrolidin-3-yl]-1H-indol
- 1-(2-Fluor-benzyl)-3-[1-(3-trifluormethyl-benzensulfonyl)-pyrrolidin-3-yl]-1H-indol
- [3-(1-Benzyl-1H-indol-3-yl)-pyrrolidin-1-yl]-(6-chlor-2H-chromen-3-yl)-methanon
- [3-(1-Benzyl-1H-indol-3-yl)-pyrrolidin-1-yl]-(3,4-difluor-phenyl)-methanon
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Die Ausdrücke "C₁₋₈-Alkyl" und " C₁₋₃-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1 bis 8 bzw. 1 bis 3 C-Atomen, d.h. C₁₋₈-Alkanyle, C₂₋₈-Alkenyle und C₂₋₈-Alkinyle bzw. C₁₋₃-Alkanyle, C₂₋₃-Alkenyle und C₂₋₃-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, isoPentyl, neo-Pentyl, n-Hexyl, 2-Hexyl, n-Heptyl, n-Octyl, 1,1,3,3-Tetramethylbutyl; Ethylenyl (Vinyl), Ethinyl, Propenyl (-CH₂CH=CH₂, -CH=CH-CH₃, -C(=CH₂)-CH₃), Propinyl (-CH-C≡CH, -C≡C-CH₃), Butenyl, Butinyl, Pentenyl, Pentinyl, Hexenyl, Hexinyl, Heptenyl, Heptinyl, Octenyl und Octinyl umfaßt.

Der Ausdruck "C₃₋₈-Cycloalkyl" bedeutet für die Zwecke dieser Erfindung cyclische Kohlenwasserstoffe mit 3 bis 8 Kohlenstoffatomen, die gesättigt oder ungesättigt, unsubstituiert oder ein- oder mehrfach substituiert sein können. Vorteilhaft ist C₃₋₈-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl enthält. Besonders bevorzugt steht Cycloalkyl für Cyclohexyl, Cyclopentyl und Cyclobutyl.

Der Ausdruck "Heterocyclyl" steht für einen 3-, 4-, 5-, 6- oder 7-gliedrigen cyclischen organischen Rest, der mindestens 1, ggf. auch 2, 3, 4 oder 5 Heteroatome enthält, wobei die Heteroatome gleich oder verschieden sind und der cyclische Rest gesättigt oder ungesättigt, aber nicht aromatisch ist und unsubstituiert oder ein- oder mehrfach substituiert sein kann. Der Heterocyclus kann auch Teil eines bi- oder polycyclischen Systems sein. Bevorzugte Heteroatome sind Stickstoff, Sauerstoff und Schwefel. Es ist bevorzugt, daß der Heterocyclyl-Rest ausgewählt ist aus der Gruppe, die Tetrahydrofuryl, Tetrahydropyranyl, Pyrrolidinyl, Piperidinyl, Piperazinyl und Morpholinyl enthält, wobei die Bindung an die Verbindung der allgemeinen Struktur I über jedes beliebige Ringglied des Heterocyclyl-Restes erfolgen kann.

Der Ausdruck "Aryl" bedeutet im Sinne dieser Erfindung aromatische Kohlenwasserstoffe, u.a. Phenyle, Naphthyle und Phenanthrenyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Vorteilhafterweise ist Aryl aus der Gruppe ausgewählt, die Phenyl, 1-Naphthyl, 2-Naphthyl, welche jeweils unsubstituiert oder ein- oder mehrfach substituiert sein können, enthält.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Heteroarylsubstituenten gleich oder verschieden sein und in jeder beliebigen und möglichen Position des Heteroaryls sein. Der Heterocyclus kann auch Teil eines bi- oder polycyclischen Systems sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Pyrazolyl, Imidazolyl, Chromenyl, Oxadiazolyl, Thiazolyl, Oxazolyl, Isoxazoyl, Pyridinyl, Pyridazinyl, Pyrimidinyl, Pyrazinyl, Pyranyl, Indolyl, Indazolyl, Purinyl, Pyrimidinyl, Indolizinyl, Chinolinyl, Isochinolinyl, Chinazolinyl, Carbazolyl, Phenazinyl, Phenothiazinyl enthält, wobei die Bindung an die Verbindungen der allgemeinen Struktur I über jedes beliebige und mögliche Ringglied des Heteroaryl-Restes erfolgen kann. Besonders bevorzugte Heteroaryl-Reste sind für die Zwecke dieser Erfindung Pyridin-2-yl, Pyridin-3-yl, Furan-2-yl, Furan-3-yl, Thien-2-yl (2-Thiophen), Thien-3-yl (3-Thiophen), Isoxazol-4-yl, lsoxazol-3-yl, Pyrazol-3-yl, Pyrazol-4-yl, Chromen-3-yl und Oxadiazol-3-yl, die jeweils unsubstituiert oder ein- oder mehrfach substituiert sein können.

Der Ausdruck "über C₁₋₃-Alkyl gebundenes Aryl, C₃₋₈-Cycloalkyl oder Heteroaryl" bedeuten für die Zwecke der vorliegenden Erfindung, daß C₁₋₃-Alkyl und C₃₋₈-Cycloalkyl, Aryl und Heteroaryl die oben definierten Bedeutungen haben und der Cycloalkyl-, Aryl- bzw. Heteroaryl-Rest über eine C₁₋₃-Alkyl-Gruppe an die Verbindung der allgemeinen Struktur I gebunden ist.

Im Zusammenhang mit "Alkyl", "Alkanyl", "Alkenyl" und "Alkinyl" versteht man unter dem Begriff "substituiert" im Sinne dieser Erfindung die Substitution eines Wasserstoffrestes durch F, Cl, Br, I, -CN, -N≡C, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, CO₂-Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, SO-Alkyl, SO₂-Alkyl, SO₂NH₂, SO₃H, PO(O-C₁₋₆-Alkyl)₂, Si(C₁₋₆-Alkyl)₃, Si(C₃₋₈-Cycloalkyl)₃, Si(CH₂-C₃₋₈-Cycloalkyl)₃, Si(Phenyl)₃, Cycloalkyl, Aryl, Heteroaryl oder Heterocyclyl, wobei unter mehrfach substituierten Resten solche Reste zu verstehen sind, die entweder an verschiedenen oder an gleichen Atomen mehrfach, z.B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfaßt - OAlkyl u.a. auch -O-CH₂-CH₂-O-CH₂-CH₂-OH. Besonders bevorzugt für die Zwecke der vorliegenden Erfindung bedeutet "Alkyl" in diesem Zusammenhang Methyl, Ethyl, Propyl, -OCH₃ oder -CN

In Bezug auf "Aryl", "Heterocyclyl", "Heteroaryl" sowie "Cycloalkyl" versteht man im Sinne dieser Erfindung unter "ein- oder mehrfach substituiert" die ein- oder mehrfache, z.B. zwei-, drei- oder vierfache, Substitution eines oder mehrerer Wasserstoffatome des Ringsystems durch F, Cl, Br, I, CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Cycloalkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Cycloalkyl, O-Aryl, O-Heteroaryl, 0-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)-C₁₋₆-Alkyl-Aryl, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, CO₂-Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, S(O)-Alkyl, S(O)-Aryl, SO₂-Alkyl, SO₂-Aryl, SO₂NH₂, SO₃H, CF₃, =O, =S; Alkyl, Cycloalkyl, Aryl, Heteroaryl und/oder Heterocyclyl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten. Für "Aryl" sind dabei besonders bevorzugte Substituenten -F, -Cl, -Br, -CF₃, -O-CH₃, -O-CH₂CH₃, Methyl, n-Propyl, Nitro, tert.-Butyl, -CN, -SCF₃, Phenyl und -SCHF₂. Für "Heteroaryl" sind besonders bevorzugte Substituenten Methyl, -CF₃, Phenyl, 2-Chlorphenyl, 4-Chlorphenyl, -SCH₃, -Cl, -CN, n-Propyl, tert.-Butyl. Für "Heterocyclyl" ist -Cl ein bevorzugter Substituent.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1λ⁶-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Acetylsalicylsäure, Hippursäure und/oder Asparaginsäure.

Die erfindungsgemäßen Substanzen eignen sich als pharmazeutische Wirkstoffe in Arzneimitteln. Ein weiterer Gegenstand der Erfindung sind daher Arzneimittel enthaltend wenigstens ein erfindungsgemäßes 3-Pyrrolidin-Indol-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen 3-Pyrrolidin-Indol-Derivat gegebenenfalls geeignete Zusatz- und/oder Hilfsstoffe, so auch auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße 3-Pyrrolidin-Indol-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen 3-Pyrrolidin-Indol-Derivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg, bevorzugt 0,05 bis 5 mg/kg wenigstens eines erfindungsgemäßen 3-Pyrrolidin-Indol-Derivats appliziert.

In einer bevorzugten Form des Arzneimittels liegt ein enthaltenes erfindungsgemäßes 3-Pyrrolidin-Indol-Derivat als reines Diastereomer und/oder Enantiomer, als Razemat oder als nicht-äquimolare oder äquimolare Mischung der Diastereomere und/oder Enantiomere vor.

Ein weiterer Gegenstand der Erfindung ist die Verwendung eines erfindungsgemäßen 3-Pyrrolidin-Indol-Derivats zur Herstellung eines Arzneimittels zur Behandlung von Schmerz, insbesondere von akutem, neuropathischem oder chronischem Schmerz.

Ein weiterer Gegenstand der Erfindung ist die Verwendung eines erfindungsgemäßen 3-Pyrrolidin-Indol-Derivats zur Herstellung eines Arzneimittels zur Behandlung von Depressionen und/oder zur Anxiolyse.

Überraschenderweise hat sich gezeigt, dass die substituierten 3-Pyrrolidin-Indol-Derivates der allgemeinen Formel I sich zur Behandlung von cardiovaskulären Erkrankungen, Harninkontinenz, Diarrhöe, Pruritus, Alkohol- und Drogenmißbrauch, Medikamentenabhängigkeit, Entzündungen, Antriebslosigkeit, Bulimie, Anorexie, Katalepsie, zur Verwendung als Lokalanästhetikum, Antiarrythmikum, Antiemetikum, Nootropikum, und zur Vigilanz- und Libidosteigerung eignen.

Gegenstand der Erfindung ist daher auch die Verwendung eines substituierten 3-Pyrrolidin-Indol-Derivates der allgemeinen Formel I zur Herstellung eines Arzneimittels zur Behandlung von cardiovaskulären Erkrankungen, Haminkontinenz, Diarrhöe, Pruritus, Alkohol- und Drogenmißbrauch, Medikamentenabhängigkeit, Entzündungen, Antriebslosigkeit, Bulimie, Anorexie, Katalepsie, zur Verwendung als Lokalanästhetikum, Antiarrythmikum, Antiemetikum, Nootropikum und zur Vigilanz- und Libidosteigerung.

Besonders bevorzugt werden die erfindungsgemäßen substituierten 3-Pyrrolidin-Indol-Derivate, die zur Herstellung eines Arzneimittels zur Behandlung von Schmerz, insbesondere von akutem, neuropathischem oder chronischem Schmerz, von Depressionen und/oder zur Anxiolyse, zur Behandlung von cardiovaskulären Erkrankungen, Harninkontinenz, Diarrhöe, Pruritus, Alkohol- und Drogenmißbrauch, Medikamentenabhängigkeit, Entzündungen, Antriebslosigkeit, Bulimie, Anorexie, Katalepsie, zur Verwendung als Lokalanästhetikum, Antiarrythmikum, Antiemetikum, Nootropikum oder zur Vigilanz- und Libidosteigerung verwendet werden, ausgewählt aus folgender Gruppe:
- (4-Propyl-phenyl)-{3-[1-(4-trifluormethyl-benzyl)-1H-indol-3-yl]-pyrrolidin-1-yl}-methanon
- (3,4-Dimethoxy-phenyl)-{3-[1-(3-methyl-butyl)-1H-indol-3-yl]-pyrrolidin-1-yl}-methanon
- [3-(1-Butyl-1H-indol-3-yl)-pyrrolidin-1-yl]-(2-methyl-6-trifluormethyl-pyridin-3-yl)-methanon
- 1-{3-[1-(3-Methoxy-benzyl)-1H-indol-3-yl]-pyrrolidin-1-yl}-3-phenyl-propenon
- (2,3-Difluor-phenyl)-{3-[1-(4-trifluormethyl-benzyl)-1H-indol-3-yl]-pyrrolidin-1-yl}-methanon
- (4-tert-Butyl-phenyl)-{3-[1-(3-methyl-butyl)-1H-indol-3-yl]-pyrrolidin-1-yl}-methanon
- {3-[1-(4-Brom-benzyl)-1H-indol-3-yl]-pyrrolidin-1-yl}-(4-propyl-phenyl)-methanon
- (2-Ethoxy-phenyl)-{3-[1-(3-methoxy-benzyl)-1H-indol-3-yl]-pyrrolidin-1-yl}-methanon
- [3-(1-Ethyl-1H-indol-3-yl)-pyrrolidin-1-yl]-(3-fluor-4-trifluormethyl-phenyl)-methanon
- (2,3-Dimethyl-phenyl)-[3-(1-ethyl-1H-indol-3-yl)-pyrrolidin-1-yl]-methanon
- 1-{3-[1-(3-Methoxy-benzyl)-1H-indol-3-yl]-pyrrolidin-1-yl}-2-phenoxy-propan-1-on
- 2-(3-Chlor-phenoxy)-1-[3-(1-ethyl-1H-indol-3-yl)-pyrrolidin-1-yl]-ethanon
- 4-{3-[1-(2-Phenoxy-propionyl)-pyrrolidin-3-yl]-indol-1-ylmethyl}-benzonitril
- N,N-Dimethyl-2-{3-[1-(2,4,6-trimethyl-benzensulfonyl)-pyrrolidin-3-yl]-indol-1-yl}-acetamid
- (3,4-Dichlor-phenyl)-[3-(1-ethyl-1H-indol-3-yl)-pyrrolidin-1-yl]-methanon
- N,N-Dimethyl-2-{3-[1-(3-trifluormethyl-phenylthiocarbamoyl)-pyrrolidin-3-yl]-indol-1-yl}-acetamid
- 3-(1-Dimethylcarbamoylmethyl-1H-indol-3-yl)-pyrrolidin-1-carbonsäure 3,4-dichlor-benzylamid
- [3-(1H-Indol-3-yl)-pyrrolidin-1-yl]-(4-trifluormethylsulfanyl-phenyl)-methanon
- 2-(3-{1-[2-(2,5-Dimethoxy-phenyl)-acetyl]-pyrrolidin-3-yl}-indol-1-yl)-N,N-dimethyl-acetamid
- (2-Chlor-4-nitro-phenyl)-{3-[1-(3-fluor-benzyl)-1H-indol-3-yl]-pyrrolidin-1-yl}-methanon
- N,N-Dimethyl-2-{3-[1-(pyridin-2-carbonyl)-pyrrolidin-3-yl]-indol-1-yl}-acetamid
- Cyclobutyl-[3-(1H-indol-3-yl)-pyrrolidin-1-yl]-methanon
- N,N-Diethyl-2-[3-(1-pentanoyl-pyrrolidin-3-yl)-indol-1-yl]-acetamid
- 3-[1-(4-Nitro-benzensulfonyl)-pyrrolidin-3-yl]-1H-indol
- 2-[3-(1-Cyclopentanecarbonyl-pyrrolidin-3-yl)-indol-1-yl]-N,N-diethyl-acetamid
- 1-[3-(1-Methyl-1H-indol-3-yl)-pyrrolidin-1-yl]-3-phenyl-propenon
- (2-Chlor-phenyl)-[3-(1H-indol-3-yl)-pyrrolidin-1-yl]-methanon
- [3-(1-Ethyl-1H-indol-3-yl)-pyrrolidin-1-yl]-pyridin-3-yl-methanon
- N,N-Dimethyl-2-(3-{1-[3-phenyl-2-(toluene-4-sulfonylamino)-propionyl]-pyrrolidin-3-yl}-indol-1-yl)-acetamid
- N,N-Dimethyl-2-[3-(1-phenylmethanesulfonyl-pyrrolidin-3-yl)-indol-1-yl]-acetamid
- 4-{3-[1-(2-Chlor-6-fluor-3-methyl-benzoyl)-pyrrolidin-3-yl]-indol-1-ylmethyl}-benzonitril
- [3-(2-Chlor-phenyl)-5-methyl-isoxazol-4-yl]-[3-(1-ethyl-1H-indol-3-yl)-pyrrolidin-1-yl]-methanon
- 2-{3-[1-(5-tert-Butyl-2-methyl-furan-3-carbonyl)-pyrrolidin-3-yl]-indol-1-ylmethyl}-benzonitril
- 4-{3-[1-(Benzo[1,2,5]oxadiazole-5-carbonyl)-pyrrolidin-3-yl]-indol-1-ylmethyl}-benzonitril
- 1-[3-(1-Ethyl-1H-indol-3-yl)-pyrrolidin-1-yl]-3-phenyl-propan-1-on
- 1-{3-[1-(3-Methoxy-benzyl)-1H-indol-3-yl]-pyrrolidin-1-yl}-3-phenyl-propan-1-on
- [3-(1-Methyl-1H-indol-3-yl)-pyrrolidin-1-yl]-(2-methylsulfanyl-pyridin-3-yl)-methanon
- (2-Chlor-5-trifluormethyl-phenyl)-[3-(1-ethyl-1H-indol-3-yl)-pyrrolidin-1-yl]-methanon
- (2-Chlor-4-nitro-phenyl)-{3-[1-(4-trifluormethyl-benzyl)-1H-indol-3-yl]-pyrrolidin-1-yl}-methanon
- (2-Chlor-pyridin-3-yl)-[3-(1-methyl-1H-indol-3-yl)-pyrrolidin-1-yl]-methanon
- 2-Methoxy-1-{3-[1-(3-methoxy-benzyl)-1H-indol-3-yl]-pyrrolidin-1-yl}-ethanon
- {3-[1-(3-Fluor-benzyl)-1H-indol-3-yl]-pyrrolidin-1-yl}-(2-methyl-6-trifluormethyl-pyridin-3-yl)-methanon
- {3-[1-(3-Methoxy-benzyl)-1H-indol-3-yl]-pyrrolidin-1-yl}-p-tolyl-methanon
- (2-Methylsulfanyl-pyridin-3-yl)-[3-(1-prop-2-ynyl-1H-indol-3-yl)-pyrrolidin-1-yl]-methanon
- Biphenyl-4-yl-[3-(1-methyl-1H-indol-3-yl)-pyrrolidin-1-yl]-methanon
- 2-(4-Chlor-phenyl)-1-{3-[1-(3-methoxy-benzyl)-1H-indol-3-yl]-pyrrolidin-1-yl}-ethanon
- [3-(1-Methyl-1H-indol-3-yl)-pyrrolidin-1-yl]-pentafluorphenyl-methanon
- [3-(1-Ethyl-1H-indol-3-yl)-pyrrolidin-1-yl]-(4-trifluormethyl-phenyl)-methanon
- 2-[3-(1-But-2-ynyl-1H-indol-3-yl)-pyrrolidin-1-yl]-N-(2,5-dimethyl-2H-pyrazol-3-yl)-acetamid
- N-(3-Cyano-4-methyl-thiophen-2-yl)-2-{3-[1-(3,5-dimethyl-benzyl)-1H-indol-3-yl]-pyrrolidin-1-yl}acetamid
- N,N-Diethyl-2-(3-{1-[(4-trifluormethyl-phenylcarbamoyl)-methyl]-pyrrolidin-3-yl}-indol-1-yl)-acetamid
- N-(3-Cyano-4-methyl-thiophen-2-yl)-2-[3-(1-ethyl-1H-indol-3-yl)-pyrrolidin-1-yl]-acetamid
- 2-{3-[1-(3,4-Difluor-benzyl)-1H-indol-3-yl]-pyrrolidin-1-yl}-N-(4-phenyl-5-trifluormethyl-thiophen-3-yl)-acetamid
- N,N-Dimethyl-2-{3-[1-(4-propyl-benzensulfonyl)-pyrrolidin-3-yl]-indol-1-yl}-acetamid
- (2-Chlor-4-nitro-phenyl)-{3-[1-(3-methoxy-benzyl)-1H-indol-3-yl]-pyrro)idin-1-yl}-methanon
- 3-{3-[1-(4-Chlor-benzensulfonyl)-pyrrolidin-3-yl]-indol-1-ylmethyl}-benzonitril
- 1-{3-[1-(3-Methoxy-benzyl)-1H-indol-3-yl]-pyrrolidin-1-yl}-3,3-dimethyl-butan-1-on
- 2-(4-Chlor-phenyl)-1-[3-(1-methyl-1H-indol-3-yl)-pyrrolidin-1-yl]-ethanon
- (2,6-Difluor-3-methyl-phenyl)-{3-[1-(3-methoxy-benzyl)-1H-indol-3-yl]-pyrrolidin-1-yl}-methanon
- [3-(1-Ethyl-1H-indol-3-yl)-pyrrolidin-1-yl]-o-tolyl-methanon
- [3-(1-Ethyl-1H-indol-3-yl)-pyrrolidin-1-yl]-(4-fluor-phenyl)-methanon
- [3-(1-Butyl-1H-indol-3-yl)-pyrrolidin-1-yl]-(6-chlor-2H-chromen-3-yl)-methanon
- (2-Chlor-6-fluor-phenyl)-[3-(1-naphthalen-2-ylmethyl-1H-indol-3-yl)-pyrrolidin-1-yl]-methanon
- 2-{3-[1-(3-Cyclopentyl-propionyl)-pyrrolidin-3-yl]-indol-1-yl}-N,N-dimethyl-acetamid
- [3-(2-Chlor-phenyl)-5-methyl-isoxazol-4-yl]-{3-[1-(3-methoxy-benzyl)-1H-indol-3-yI]-pyrrolidin-1-yl}-methanon
- [1-(4-Chlor-phenyl)-5-trifluormethyl-1H-pyrazol-4-yl]-{3-[1-(3-fluor-benzyl)-1H-indol-3-yl]-pyrrolidin-1-yl}-methanon
- [3-(1-Allyl-1H-indol-3-yl)-pyrrolidin-1-yl]-p-tolyl-methanon
- {3-[1-(4-Fluor-benzyl)-1H-indol-3-yl]-pyrrolidin-1-yl}-pyridin-2-yl-methanon
- (3,4-Dichlor-phenyl)-{3-[1-(4-trifluormethyl-benzyl)-1H-indol-3-yl]-pyrrolidin-1-yl}-methanon
- 1-Butyl-3-[1-(2,5-dimethoxy-benzensulfonyl)-pyrrolidin-3-yl]-1H-indol
- 1-[3-(1-Allyl-1H-indol-3-yl)-pyrrolidin-1-yl]-3,3-dimethyl-butan-1-on
- 4-{3-[1-(2-Methoxy-acetyl)-pyrrolidin-3-yl]-indol-1-ylmethyl}-benzonitril
- (2,4-Difluor-phenyl)-[3-(1-ethyl-1H-indol-3-yl)-pyrrolidin-1-yl]-methanon
- (2,3-Dimethyl-phenyl)-{3-[1-(4-trifluormethyl-benzyl)-1H-indol-3-yl]-pyrrolidin-1-yl}-methanon
- [3-(1-Butyl-1H-indol-3-yl)-pyrrolidin-1-yl]-(4-methyl-3-nitro-phenyl)-methanon
- (4-Brom-3-methyl-phenyl)-{3-[1-(3-methoxy-benzyl)-1H-indol-3-yl]-pyrrolidin-1-yl}-methanon
- 1-[3-(1-Butyl-1H-indol-3-yl)-pyrrolidin-1-yl]-2-(4-chlor-phenoxy)-ethanon
- 2-(4-Chlor-phenyl)-1-{3-[1-(3-fluor-benzyl)-1H-indol-3-yl]-pyrrolidin-1-yl}-ethanon
- (4-Chlor-phenyl)-{3-[1-(4-trifluormethyl-benzyl)-1H-indol-3-yl]-pyrrolidin-1-yl}-methanon
- [3-(1-Benzyl-1H-indol-3-yl)-pyrrolidin-1-yl]-(5-methyl-isoxazol-3-yl)-methanon
- 1-[3-(1-Benzyl-1H-indol-3-yl)-pyrrolidin-1-yl]-2-(4-chlor-phenoxy)-ethanon
- [3-(1-Naphthalen-2-ylmethyl-1H-indol-3-yl)-pyrrolidin-1-yl]-(4-trifluormethylphenyl)-methanon
- (3-Methoxy-phenyl)-{3-[1-(4-trifluormethyl-benzyl)-1H-indol-3-yl]-pyrrolidin-1-yl}-methanon
- 1-[3-(1-Butyl-1H-indol-3-yl)-pyrrolidin-1-yl]-3-(2-chlor-phenyl)-propenon
- 1-[3-(1-Methyl-1H-indol-3-yl)-pyrrolidin-1-yl]-2-phenyf-butan-1-on
- (2-Chlor-6-fluor-3-methyl-phenyl)-[3-(1-methyl-1H-indol-3-yl)-pyrrolidin-1-yl]-methanon
- 2-{3-[1-(3-Methoxy-benzoyl)-pyrrolidin-3-yl]-indol-1-yl}-N,N-dimethyl-acetamid
- 2-{3-[1-(2-Fluor-benzoyl)-pyrrolidin-3-yl]-indol-1-yl}-N,N-dimethyl-acetamid
- (3-Difluormethylsulfanyl-phenyl)-[3-(1-naphthalen-2-ylmethyl-1H-indol-3-yl)-pyrrolidin-1-yl]-methanon
- Benzo[1,2,5]oxadiazol-5-yl-{3-[1-(3-fluor-benzyl)-1H-indol-3-yl]-pyrrolidin-1-yl}-methanon
- o-Tolyl-{3-[1-(4-trifluormethyl-benzyl)-1H-indol-3-yl]-pyrrolidin-1-yl}-methanon
- N,N-Dimethyl-2-{3-[1-(2-trifluormethyl-benzensulfonyl)-pyrrolidin-3-yl]-indol-1-yl}-acetamid
- 1-[3-(1-Butyl-1H-indol-3-yl)-pyrrolidin-1-yl]-2-phenoxy-propan-1-on
- (6-Chlor-2-fluor-3-methyl-phenyl)-[3-(1-naphthalen-2-ylmethyl-1H-indol-3-yl)-pyrrolidin-1-yl]-methanon
- [3-(1-Allyl-1H-indol-3-yl)-pyrrolidin-1-yl]-(2-methyl-6-trifluormethyl-pyridin-3-yl)-methanon
- [3-(1-Butyl-1H-indol-3-yl)-pyrrolidin-1-yl]-(4-propyl-phenyl)-methanon
- 2-{3-[1-(4-Chlor-benzoyl)-pyrrolidin-3-yl]-indol-1-yl}-N,N-dimethyl-acetamid
- 1-{3-[1-(3-Fluor-benzyl)-1H-indol-3-yl]-pyrrolidin-1-yl}-3-phenyl-propan-1-on
- {3-[1-(2-Fluor-benzyl)-1H-indol-3-yl]-pyrrolidin-1-yl}-(1-phenyl-5-propyl-1H-pyrazol-4-yl)-methanon
- 3-{3-[1-(5-tert-Butyl-2-methyl-furan-3-carbonyl)-pyrrolidin-3-yl]-indol-1-ylmethyl}-benzonitril
- 3-[1-(2,4-Difluor-benzensulfonyl)-pyrrolidin-3-yl]-1-(2-fluor-benzyl)-1H-indol
- 2-{3-[1-(2,6-Difluor-benzoyl)-pyrrolidin-3-yl]-indol-1-yl}-N,N-dimethyl-acetamid
- 1-[3-(1-Butyl-1H-indol-3-yl)-pyrrolidin-1-yl]-butan-1-on
- 4-{3-[1-(2-Propyl-pentanoyl)-pyrrolidin-3-yl]-indol-1-ylmethyl}-benzonitril
- 2-(4-Chlor-phenoxy)-1-[3-(1-prop-2-ynyl-1H-indol-3-yl)-pyrrolidin-1-yl]-ethanon
- [3-(1-Methyl-1H-indol-3-yl)-pyrrolidin-1-yl]-pyridin-3-yl-methanon
- 3-{3-[1-(Benzo[1,3]dioxole-5-carbonyl)-pyrrolidin-3-yl]-indol-1-ylmethyl}-benzonitril
- [3-(1-Butyl-1H-indol-3-yl)-pyrrolidin-1-yl]-(2-chlor-5-trifluormethyl-phenyl)-methanon
- (3-Chlor-phenyl)-{3-[1-(4-trifluormethyl-benzyl)-1H-indol-3-yl]-pyrrolidin-1-yl}-methanon
- 2-{3-[1-(4-Brom-benzoyl)-pyrrolidin-3-yl]-indol-1-yl}-N,N-dimethyl-acetamid
- 3-Phenyl-1-[3-(1-prop-2-ynyl-1H-indol-3-yl)-pyrrolidin-1-yl]-propenon
- 3-{3-[1-(3-Chlor-2-fluor-benzoyl)-pyrrolidin-3-yl]-indol-1-ylmethyl}-benzonitril
- 3-{3-[1-(2-Phenyl-cyclopropanecarbonyl)-pyrrolidin-3-yl]-indol-1-ylmethyl}-benzonitril
- 3-{3-[1-(3-Chlor-thiophen-2-carbonyl)-pyrrolidin-3-yl]-indol-1-ylmethyl}-benzonitril
- 1-Prop-2-ynyl-3-[1-(2-trifluormethyl-benzensulfonyl)-pyrrolidin-3-yl]-1H-indol
- 1-(2-Fluor-benzyl)-3-[1-(3-trifluormethyl-benzensulfonyl)-pyrrolidin-3-yl]-1H-indol
- [3-(1-Benzyl-1H-indol-3-yl)-pyrrolidin-1-yl]-(6-chlor-2H-chromen-3-yl)-methanon
- [3-(1-Benzyl-1H-indol-3-y1)-pyrrolidin-1-yl]-(3,4-difluor-phenyl)-methanon
in Form des Razemats, der reinen Stereoisomeren, der Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weiterer Wirkstoffe.

Ein weiterer Gegenstand der Erfindung ist ein Verfahren zur Herstellung eines erfindungsgemäßen 3-Pyrrolidin-Indol-Derivats.

3-Pyrrolidin-Indolderivate der allgemeinen Formel I werden nach folgendem Schema hergestellt:

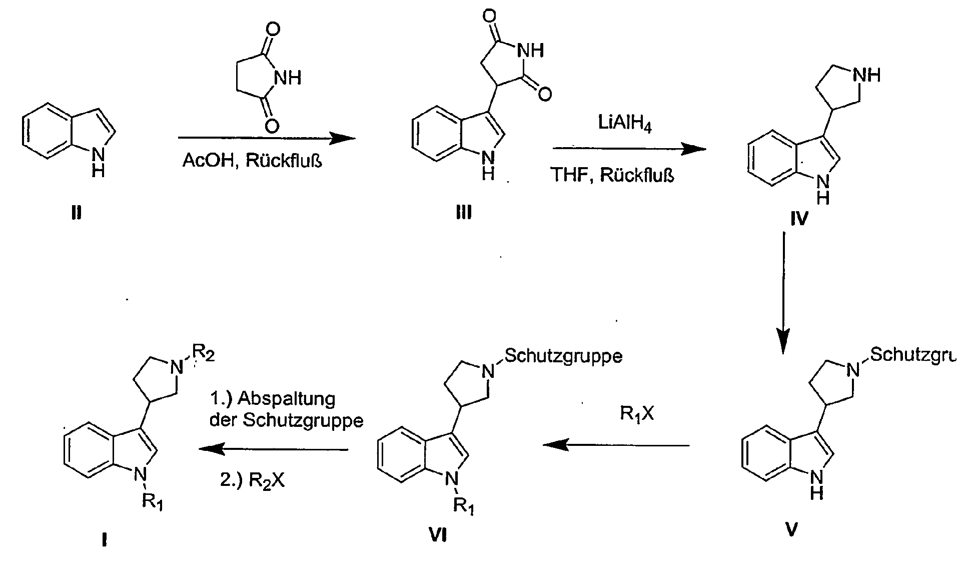

Dabei wird Indol (II) mit Maleinimid in an sich bekannter Weise unter Zugabe von Essigsäure umgesetzt und anschließend mit LiAlH₄ zu 3-Pyrrolidin-3-yl-indol (III) umgesetzt. Nach Schützen des Pyrrolidin-Stickstoffs mit einer an sich bekannten Schutzgruppe, vorzugsweise der Boc-Schutzgruppe, wird der Indol-Stickstoff durch Zugabe eines Alkylierungsmittels, vorzugsweise ein Alkylhalogenid oder ein Benzylhalogenid, beispielsweise 3-Fluorbenzylbromid, alkyliert. Nach Abspaltung der Schutzgruppe wird der Pyrrolidin-Stickstoff mit einem 2-Chloracetamid oder einem 2-Bromacetamid, beispielsweise 2-Chlor-N-(4-trifluormethylphenyl)-acetamid, einem Säurechlorid, beispielsweise 2-Chlorbenzoylchlorid, einem Isocyanat, beispielsweise1,2-Dichlor-4-isocyanatomethyl-benzol, oder einem Isothiocyanat, beispielsweise 1-Isothiocyanato-3-trifluormethyl-benzol, umgesetzt.
Für den Fall, dass R¹ H bedeutet, wird **IV** direkt durch Umsetzung mit einem 2-Chloracetamid oder einem 2-Bromacetamid, beispielsweise 2-Chlor-N-(4-trifluormethylphenyl)acetamid, einem Säurechlorid, beispielsweise 2-Chlorbenzoylchlorid, einem Isocyanat, beispielsweise1,2-Dichlor-4-isocyanatomethylbenzol, oder einem Isothiocyanat, beispielsweise 1-Isothiocyanato-3-trifluormethylbenzol, zu Verbindungen der allgemeinen Formel I umgesetzt.

### Beispiele

### Beispiel 1: Synthese von (2-Chlor-4-nitro-phenyl)-{3-[1-(3-fluor-benzyl)-1H-indol-3-yl]-pyrrolidin-1-yl}-methanon (Verbindung 20):

### 1. Stufe:

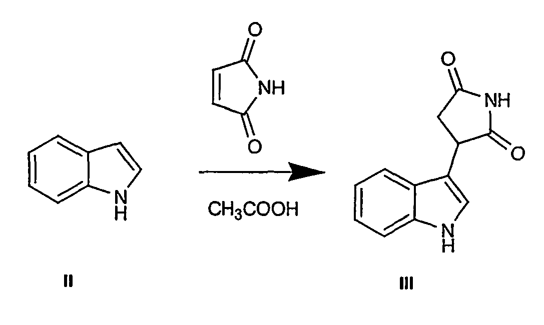

Zu einer Mischung von 29,5 g (252 mmol) Indol (II) und 24,4 g (251 mmol) Maleinimid wurden 220 ml Essigsäure zugegeben. Das Reaktionsgemisch wurde 3 Tage unter Stickstoffatmosphäre zum Rückfluss erhitzt. Nach Entfernen des Lösungsmittels wurden 500 ml Ethylacetat zugegeben und das Gemisch wurde 2x mit 250 ml wäßriger NaHCO₃-Lösung extrahiert. Die wäßrige Phase wurde mit 500 ml Ethylacetat extrahiert. Die vereinigten organischen Phasen wurden über Na₂SO₄ getrocknet. Das Rohprodukt wurde über Säulenchromatographie gereinigt (Kieselgel, Ethylacetat/Hexan 1:1 gefolgt durch Dichlormethan (DCM) gefolgt durch Et₃N/DCM/MeOH 1:89:10). Es wurden 25,7 g (48%) von III erhalten.

### 2. Stufe:

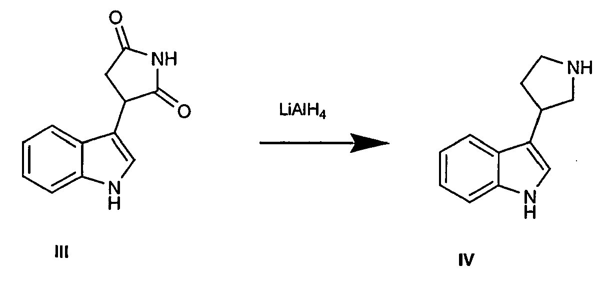

15,7 g (414 mmol) LiAIH₄ wurden in 100 ml trockenem Tetrehydrofuran (THF) gelöst. Eine Lösung von 17,7 g (82,6 mmol) von **III** in 250 ml trockenem THF wurde innerhalb von 30 min zugetropft. Das Reaktionsgemisch wurde über Nacht unter Stickstoffatmosphäre zum Rückfluss erhitzt. Unter Eiskühlung wurden vorsichtig 30 ml Ethylacetat zugegeben. Nach einer Stunde wurden 15 ml Wasser vorsichtig zugegeben, und nach weiterem Rühren bei Raumtemperatur wurden 15 ml einer wäßrigen 2 N NaOH-Lösung zugegeben. Das Reaktionsgemisch wurde zum Rückfluss erhitzt und mit weiteren 50 ml Wasser versetzt. Nach weiteren 2 Stunden wurde das Reaktionsgemisch auf Raumtemperatur abgekühlt und abfiltriert. Der Rückstand wurde mit THF gewaschen. Es wurden 16,3 g von IV erhalten, was ohne weitere Aufreinigung eingesetzt wurde.

### 3. Stufe:

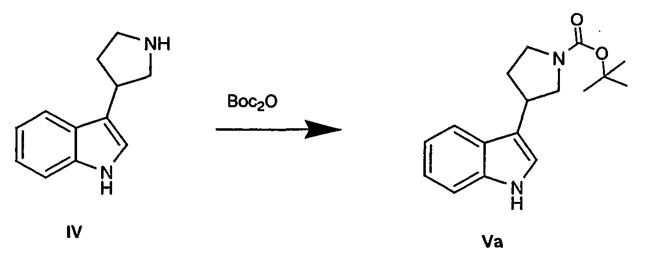

Unter Stickstoffatmosphäre bei 0°C wurde zu einer Lösung von 16,3 g **IV** in 500 ml DCM eine Lösung von 19,1 g (87,5 mmol) Boc₂O in 100 ml DCM innerhalb von 15 min getropft. Das Reaktionsgemisch wurde 2 h bei 0°C und daraufhin über Nacht bei Raumtemperatur gerührt. Nach Aufreinigung über Säulenchromatograpie (1. Kieselgel, Et₂O, 2. Kieselgel Ethylacetat/Heptan 1:2) erhielt man 4,1 g (17%) von **Va**.

### 4. Stufe:

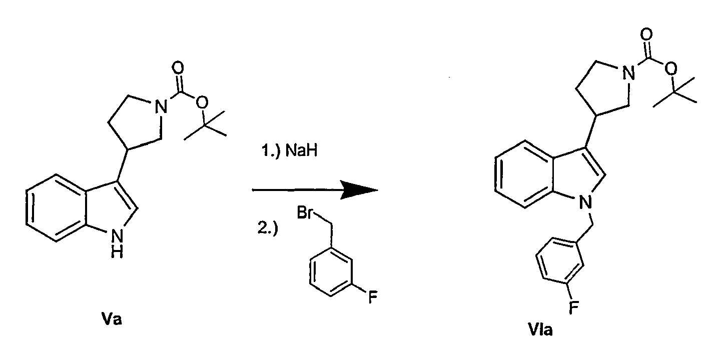

Unter Stickstoffatmosphäre wurden 126 mg (3,15 mmol) NaH (60% Dispersion in Mineralöl) in 6 ml trockenem THF suspendiert. Die Suspension wurde auf 0°C gekühlt und 750 mg **Va** (2,62 mmol) wurden hinzugegeben. Das Gemisch wurde 3 h bei 0°C und anschließend weitere 30 min bei Raumtemperatur gerührt. Nach Erneuter Kühlung auf 0°C wurden 595 mg (3,15 mmol) 3-Fluorbenzylbromid zugegeben, und das Reaktionsgemisch wurde 3 h bei 0°C und anschließend über Nacht bei Raumtemperatur gerührt. Wasser und gesättigte wäßrige NaCl-Lösung wurden zugegeben, und das Reaktionsgemisch wurde 3x mit DCM extrahiert. Die organischen Phasen wurden über Na₂SO₄ getrocknet und zur Trockne eingeengt. Das Produkt (1,08 g) wurde ohne weitere Aufreinigung eingesetzt.

### 5. Stufe

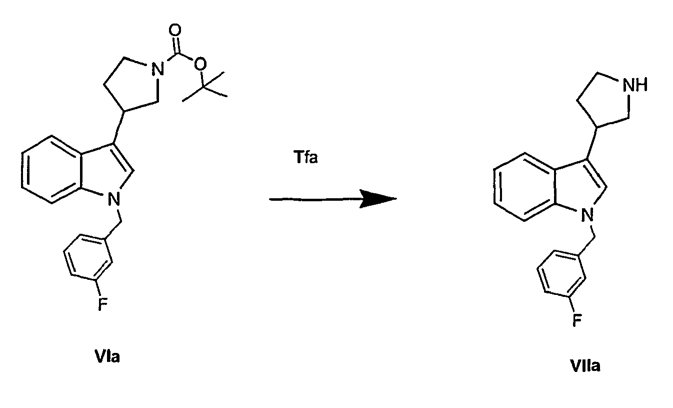

Verbindung **VIa** (1,08 g) wurde in 5 ml DCM gelöst. Nach Zugabe von 5 ml Trifluoressigsäure (Tfa) wurde über Nacht bei Raumtemperatur gerührt. Nach Entfernen des Lösungsmittels wurden Methanol und Heptan zugegeben. Nach Abdestillieren der Methanol-Schicht wurde Ethylacetat und wäßrige gesättigte NaHCO₃-Lösung zugegeben. Die organische Schicht wurde abgetrennt, über Na₂SO₄ getrocknet und zur Trockne eingeengt. Ausbeute: 870 mg **VIIa.**

### 6. Stufe:

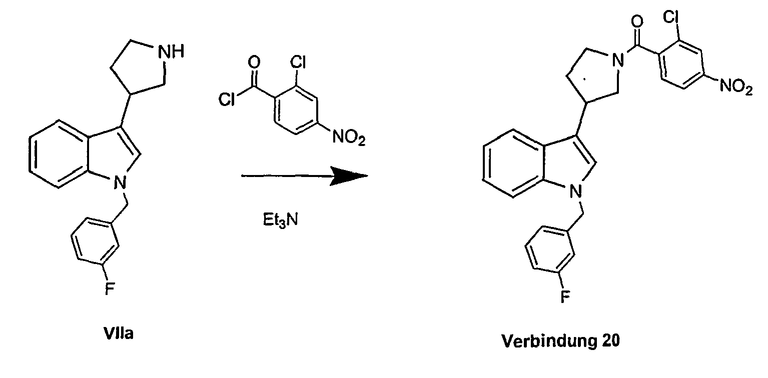

Verbindung **VIIa** (0,87 g) wurde in 10 ml DCM gelöst. 299 mg (2,95 mmol) und 650 mg (2,95 mmol) 2-Chlor-4-nitrobenzoylchlorid wurden zugegeben und das Reaktionsgemisch wurde unter Stickstoffatmosphäre über Nacht bei Raumtemperatur gerührt. Die Lösung wurde nacheinander mit 1 N NaOH, 1 N HCl und gesättigter wäßriger NaHCO₃-Lösung extrahiert. Die organische Phase wurde über Na₂SO₄ getrocknet und zur Trockne eingeengt. Das Rohprodukt wurde über Säulenchromatographie gereinigt (1. Kieselgel, MeOH/DCM 1:99, 2. Kieselgel, Ethylacetat/Hexan 1:2). Ausbeute: 373 mg **Verbindung 20**.

Die übrigen Beispielverbindungen wurden in analoger Weise hergestellt.

### Verbindungen:

| **Verbindung** | **R¹** | **R²** | **Name** |
|---|---|---|---|
| 1 | 4-Trifluormethyl benzyl | 4-Propyl-benzoyl | (4-Propyl-phenyl)-{3-[1-(4-trifluormethyl-benzyl)-1H-indol-3-yl]-pyrrolidin-1-yl}-methanon |
| 2 | 3-Methylbutyl | 3,4-Dimethoxy-benzoyl | (3,4-Dimethoxy-phenyl)-{3-[1-(3-methyl-butyl)-1H-indol-3-yl]-pyrrolidin-1-yl}-methanon |
| 3 | Butyl | 2-Methyl-6-trifluormethyl-nicotinoyl | [3-(1-Butyl-1H-indol-3-yl)-pyrrolidin-1-yl]-(2-methyl-6-trifluormethyl-pyridin-3-yl)-methanon |
| 4 | 3-Methoxy-benzyl | 3-Phenyl-acryloyl | 1-{3-[1-(3-Methoxy-benzyl)-1H-indol-3-yl]-pyrrolidin-1-yl}-3-phenyl-propenon |
| 5 | 4-Trifluormethyl benzyl | 2,3-Difluor-benzoyl | (2,3-Difluor-phenyl)-{3-[1-(4-trifluormethyl-benzyl)-1H-indol-3-yl]-pyrrolidin-1-yl}-methanon |
| 6 | 3-Methylbutyl | 4-Trifluormethyl-benzoyl | (4-tert-Butyl-phenyl)-{3-[1-(3-methyl-butyl)-1H-indol-3-yl]-pyrrolidin-1-yl}-methanon |
| 7 | 4-Brombenzyl | 4-Propyl-benzoyl | {3-[1-(4-Brom-benzyl)-1H-indol-3-yl]-pyrrolidin-1-yl}-(4-propyl-phenyl)-methanon |
| 8 | 3-Methoxy-benzyl | 2-Ethoxy-benzoyl | (2-Ethoxy-phenyl)-{3-[1-(3-methoxy-benzyl)-1H-indol-3-yl]-pyrrolidin-1-yl}-methanon |
| 9 | Ethyl | 4-Trifluormethyl -3-fluor-benzoyl | [3-(1-Ethyl-1H-indol-3-yl)-pyrrolidin-1-yl]-(3-fluor-4-trifluormethyl-phenyl)-methanon |
| 10 | Ethyl | 2,3-Dimethyl-benzoyl | (2,3-Dimethyl-phenyl)-[3-(1-ethyl-1H-indol-3-yl)-pyrrolidin-1-yl]-methanon |
| 11 | 3-Methoxy-benzyl | 2-Phenoxy-propionyl | 1-{3-[1-(3-Methoxy-benzyl)-1H-indol-3-yl]-pyrrolidin-1-yl}-2-phenoxy-propan-1-on |
| 12 | Ethyl | (3-Chlor-phenoxy)-acetyl | 2-(3-Chlor-phenoxy)-1-[3-(1-ethyl-1H-indol-3-yl)-pyrrolidin-1-yl]-ethanon |
| 13 | 4-Cyanobenzyl | 2-Phenoxy-propionyl | 4-{3-[1-(2-Phenoxy-propionyl)-pyrrolidin-3-yl]-indol-1-ylmethyl}-benzonitril |
| 14 | 2-*N,N*-Dimethyl-acetamid | 2,4,6-Trimethyl-benzyl-sulfonyl | N,N-Dimethyl-2-{3-[1-(2,4,6-trimethyl-benzensulfonyl)-pyrrolidin-3-yl]-indol-1-yl}-acetamid |
| 15 | Ethyl | 3,4-Dichlor-benzoyl | (3,4-Dichlor-phenyl)-[3-(1-ethyl-1H-indol-3-yl)-pyrrolidin-1-yl]-methanon |
| 16 | 2-*N,N*-Dimethyl-acetamid | N-(3-Trifluormethyl-phenyl)-thio-carbamoyl | N,N-Dimethyl-2-{3-[1-(3-trifluormethyl-phenylthiocarbamoyl)-pyrrolidin-3-yl]-indol-1-yl}-acetamid |
| 17 | *2-N,N*-Dimethyl-acetamid | 3,4-Dichlor-benzylamid | 3-(1-Dimethylcarbamoylmethyl-1H-indol-3-yl)-pyrrolidin-1-carbonsäure 3,4-dichlor-benzylamid |
| 18 | H | 4-(Trifluormethyl-sulfanyl)-benzoyl | [3-(1H-Indol-3-yl)-pyrrolidin-1-yl]-(4-trifluormethylsulfanyl-phenyl)-methanon |
| 19 | *2-N,N*-Dimethyl-acetamid | 2,5-Dimethoxy-phenylacetyl | 2-(3-{1-[2-(2,5-Dimethoxy-phenyl)-acetyl]-pyrrolidin-3-yl}-indol-1-yl)-N,N-dimethyl-acetamid |
| 20 | 3-Fluorbenzyl | 2-Chlor-4-nitrobenzoyl | (2-Chlor-4-nitro-phenyl)-{3-[1-(3-fluor-benzyl)-1H-indol-3-yl]-pyrrolidin-1-yl}-methanon |
| 21 | *2-N,N*-Dimethyl-acetamid | Pyridin-2-carbonyl | N,N-Dimethyl-2-{3-[1-(pyridin-2-carbonyl)-pyrrolidin-3-yl]-indol-1-yl}-acetamid |
| 22 | H | Cyclobutyl-carbonyl | Cyclobutyl-[3-(1H-indol-3-yl)-pyrrolidin-1-yl]-methanon |
| 23 | 2-*N,N*-Diethyl-acetamid | Pentanoyl | N,N-Diethyl-2-[3-(1-pentanoyl-pyrrolidin-3-yl)-indol-1-yl]-acetamid |
| 24 | H | 4-Nitro-benzyl-sulfonyl | 3-[1-(4-Nitro-benzensulfonyl)-pyrrolidin-3-yl]-1H-indol |
| 25 | 2-*N,N*-Diethyl-acetamid | Cyclopentyl-carbonyl | 2-[3-(1-Cyclopentanecarbonyl-pyrrolidin-3-yl)-indol-1-yl]-N,N-diethyl-acetamid |
| 26 | Methyl | 3-Phenyl-acryloyl | 1-[3-(1-Methyl-1H-indol-3-yl)-pyrrolidin-1-yl]-3-phenyl-propenon |
| 27 | H | 2-Chlorbenzoyl | (2-Chlor-phenyl)-[3-(1H-indol-3-yl)-pyrrolidin-1-yl]-methanon |
| 28 | Ethyl | 3-Pyridyl-carbonyl | [3-(1-Ethyl-1H-indol-3-yl)-pyrrolidin-1-yl]-pyridin-3-yl-methanon |
| 29 | 2*-N,N*-Dimethyl-acetamid | 3-Phenyl-2-(tolyl-4-sulfonyl-amino)-propionyl | N,N-Dimethyl-2-(3-{1-[3-phenyl-2-(toluene-4-sulfonylamino)-propionyl]-pyrrolidin-3-yl}-indol-1-yl)-acetamid |
| 30 | 2-*N,N*-Dimethyl-acetamid | Benzyl-sulfonyl | N,N-Dimethyl-2-[3-(1-phenylmethanesulfonyl-pyrrolidin-3-yl)-indol-1-yl]-acetamid |
| 31 | 4-Cyanobenzyl | 2-Chlor-6-fluor-3-methyl-benzoyl | 4-{3-[1-(2-Chlor-6-fluor-3-methyl-benzoyl)-pyrrolidin-3-yl]-indol-1-ylmethyl}-benzonitril |
| 32 | Ethyl | 3-(2-Chlor-phenyl)-5-methyl-isoxazol-4-carbonyl | [3-(2-Chlor-phenyl)-5-methyl-isoxazol-4-yl]-[3-(1-ethyl-1H-indol-3-yl)-pyrrolidin-1-yl]-methanon |
| 33 | 2-Cyanobenzyl | 5-*tert*-Butyl-2-methyl-furan-3-carbonyl | 2-{3-[1-(5-*tert*-Butyl-2-methyl-furan-3-carbonyl)-pyrrolidin-3-yl]-indol-1-ylmethyl}-benzonitril |
| 34 | 4-Cyanobenzyl | Benzo[1,2,5] oxadiazol-5-carbonyl | 4-{3-[1-(Benzo[1,2,5]oxadiazole-5-carbonyl)-pyrrolidin-3-yl]-indol-1-ylmethyl}-benzonitril |
| 35 | Ethyl | 3-Phenyl-propionyl | 1-[3-(1-Ethyl-1H-indol-3-yl)-pyrrolidin-1-yl]-3-phenyl-propan-1-on |
| 36 | 3-Methoxy-benzyl | 3-Phenyl-propionyl | 1-{3-[1-(3-Methoxy-benzyl)-1H-indol-3-yl]-pyrrolidin-1-yl}-3-phenyl-propan-1-on |
| 37 | Methyl | 2-Methyl-sulfanyl-nicotinoyl | [3-(1-Methyl-1H-indol-3-yl)-pyrrolidin-1-yl]-(2-methylsulfanyl-pyridin-3-yl)-methanon |
| 38 | Ethyl | 2-Chlor-5-trifluormethyl benzoyl | (2-Chlor-5-trifluormethyl-phenyl)-[3-(1-ethyl-1H-indol-3-yl)-pyrrolidin-1-yl]-methanon |
| 39 | 4-Trifluormethyl benzyl | 2-Chlor-4-nitrobenzoyl | (2-Chlor-4-nitro-phenyl)-{3-[1-(4-trifluormethyl-benzyl)-1H-indol-3-yl]-pyrrolidin-1-yl}-methanon |
| 40 | Methyl | 2-Chlor-nicotinoyl | (2-Chlor-pyridin-3-yl)-[3-(1-methyl-1H-indol-3-yl)-pyrrolidin-1-yl]-methanon |
| 41 | 3-Methoxy-benzyl | Methoxy-acetyl | 2-Methoxy-1-{3-[1-(3-methoxy-benzyl)-1H-indol-3-yl]-pyrrolidin-1-yl}-ethanon |
| 42 | 3-Fluorbenzyl | 4-Trifluormethyl-2-methyl-nicotinoyl | {3-[1-(3-Fluor-benzyl)-1H-indol-3-yl]-pyrrolidin-1-yl}-(2-methyl-6-trifluormethyl-pyridin-3-yl)-methanon |
| 43 | 3-Methoxy-benzyl | 4-Methyl-benzoyl | {3-[1-(3-Methoxy-benzyl)-1H-indol-3-yl]-pyrrolidin-1-yl}-p-tolyl-methanon |
| 44 | 3-Propinyl | 2-Methyl-sulfanyl-nicotinoyl | (2-Methylsulfanyl-pyridin-3-yl)-[3-(1-prop-2-inyl-1H-indol-3-yl)-pyrrolidin-1-yl]-methanon |
| 45 | Methyl | 4-Phenyl-benzoyl | Biphenyl-4-yl-[3-(1-methyl-1H-indol-3-yl)-pyrrolidin-1-yl]-methanon |
| 46 | 3-Methoxy-benzyl | (4-Chlor-phenyl)-acetyl | 2-(4-Chlor-phenyl)-1-{3-[1-(3-methoxy-benzyl)-1H-indol-3-yl]-pyrrolidin-1-yl}-ethanon |
| 47 | Methyl | 2,3,4,5,6-Pentafluor-benzoyl | [3-(1-Methyl-1H-indol-3-yl)-pyrrolidin-1-yl]-pentafluorphenyl-methanon |
| 48 | Ethyl | 4-Trifluormethyl-benzoyl | [3-(1-Ethyl-1H-indol-3-yl)-pyrrolidin-1-yl]-(4-trifluormethyl-phenyl)-methanon |
| 49 | 1-(2-Butinyl) | *N*-(2,5-Dimethyl-2H-pyrazol-3-yl)-acetamidyl | 2-[3-(1-But-2-ynyl-1H-indol-3-yl)-pyrrolidin-1-yl]-N-(2,5-dimethyl-2H-pyrazol-3-yl)-acetamid |
| 50 | 3,5-Dimethyl-benzyl | *N*-(3-Cyano- 4-methyl-thiophen-2-yl)-acetamidyl | N-(3-Cyano-4-methyl-thiophen-2-yl)-2-{3-[1-(3,5-dimethyl-benzyl)-1H-indol-3-yl]-pyrrolidin-1-yl}acetamid |
| 51 | 2-*N*,*N*-Diethyl-acetamid | *N*-(4-Trifluormethyl-phenyl)-acetamidyl | N,N-Diethyl-2-(3-{1-[(4-trifluormethyl-phenylcarbamoyl)-methyl]-pyrrolidin-3-yl}-indol-1-yl)-acetamid |
| 52 | Ethyl | *N*-(3-Cyano-4-methyl-thiophen-2-yl)-acetamidyl | N-(3-Cyano-4-methyl-thiophen-2-yl)-2-[3-(1-ethyl-1H-indol-3-yl)-pyrrolidin-1-yl]-acetamid |
| 53 | 3,4-Difluorbenzyl | *N*-(4-Phenyl-5-trifluor-methyl-thiophen-3-yl)-acetamidyl | 2-{3-[1-(3,4-Difluor-benzyl)-1H-indol-3-yl]-pyrrolidin-1-yl}-N-(4-phenyl-5-trifluormethyl-thiophen-3-yl)-acetamid |
| 54 | 2-*N,N*-Dimethyl-acetamid | 4-Propyl-benzyl-sulfonyl | *N,N*-Dimethyl-2-{3-[1-(4-propyl-benzensulfonyl)-pyrrolidin-3-yl]-indol-1-yl}-acetamid |
| 55 | 3-Methoxy-benzyl | 2-Chlor-4-nitrobenzoyl | (2-Chlor-4-nitro-phenyl)-(3-[1-(3-methoxy-benzyl)-1H-indol-3-yl]-pyrrolidin-1-yl}-methanon |
| 56 | 3-Cyanobenzyl | 4-Chlor-benzyl-sulfonyl | 3-{3-[1-(4-Chlor-benzen sulfonyl)-pyrrolidin-3-yl]-indol-1-ylmethyl}-benzonitril |
| 57 | 3-Methoxy-benzyl | 3,3-Dimethyl-butyryl | 1-{3-[1-(3-Methoxy-benzyl)-1H-indol-3-yl]-pyrrolidin-1-yl}-3,3-dimethyl-butan-1-on |
| 58 | Methyl | (4-Chlor-phenyl)-acetyl | 2-(4-Chlor-phenyl)-1-[3-(1-methyl-1H-indol-3-yl)-pyrrolidin-1-yl]-ethanon |
| 59 | 3-Methoxy-benzyl | 2,6-Difluor-3-methyl-benzoyl | (2,6-Difluor-3-methyl-phenyl)-{3-[1-(3-methoxy-benzyl)-1H-indol-3-yl]-pyrrolidin-1-yl}-methanon |
| 60 | Ethyl | 2-Methyl-benzoyl | [3-(1-Ethyl-1H-indol-3-yl)-pyrrolidin-1-yl]-o-tolyl-methanon |
| 61 | Ethyl | 4-Fluor-benzoyl | [3-(1-Ethyl-1H-indol-3-yl)-pyrrolidin-1-yl]-(4-fluor-phenyl)-methanon |
| 62 | n-Butyl | 6-Chlor-2H-chromen-3-carbonyl | [3-(1-Butyl-1H-indol-3-yl)-pyrrolidin-1-yl]-(6-chlor-2H-chromen-3-yl)-methanon |
| 63 | Naphtyl-methyl | 2-Chlor-6-fluor-benzoyl | (2-Chlor-6-fluor-phenyl)-[3-(1-naphthalen-2-ylmethyl-1H-indol-3-yl)-pyrrolidin-1-yl]-methanon |
| 64 | *2-N,N*-Dimethyl-acetamid | 3-Cyclopentyl-propionyl | 2-{3-[1-(3-Cyclopentyl-propionyl)-pyrrolidin-3-yl]-indol-1-yl}-N,N-dimethyl-acetamid |
| 65 | 3-Methoxy-benzyl | 3-(2-Chlor-phenyl)-5-methyl-isoxazol-4-carbonyl | [3-(2-Chlor-phenyl)-5-methyl-isoxazol-4-yl]-{3-[1-(3-methoxy-benzyl)-1H-indol-3-yl]-pyrrolidin-1-yl}-methanon |
| 66 | 3-Fluorbenzyl | 1-(4-Chlor-phenyl)-5-trifluormethyl-1H-pyrazol-4-carbonyl | [1-(4-Chlor-phenyl)-5-trifluormethyl-1H-pyrazol-4-yl]-{3-[1-(3-fluor-benzyl)-1H-indol-3-yl]-pyrrolidin-1-yl}-methanon |
| 67 | 2-Propenyl | 4-Methyl-benzoyl | [3-(1-Allyl-1H-indol-3-yl)-pyrrolidin-1-yl]-p-tolyl-methanon |
| 68 | 4-Fluorbenzyl | Pyridine-2-carbonyl | {3-[1-(4-Fluor-benzyl)-1H-indol-3-yl]-pyrrolidin-1-yl}-pyridin-2-yl-methanon |
| 69 | 4-Trifluormethyl benzyl | 3,4-Dichlor-benzoyl | (3,4-Dichlor-phenyl)-{3-[1-(4-trifluormethyl-benzyl)-1H-indol-3-yl]-pyrrolidin-1-yl}-methanon |
| 70 | *n*-Butyl | 2,5-Dimethoxy-benzoyl | 1-Butyl-3-[1-(2,5-dimethoxy-benzensulfonyl)-pyrrolidin-3-yl]-1H-indol |
| 71 | 2-Propenyl | 3,3-Dimethyl-butyryl | 1-[3-(1-Allyl-1H-indol-3-yl)-pyrrolidin-1-yl]-3,3-dimethyl-butan-1-on |
| 72 | 4-Cyano-benzyl | Methoxy-acetyl | 4-{3-[1-(2-Methoxy-acetyl)-pyrrolidin-3-yl]-indol-1-ylmethyl}-benzonitril |
| 73 | Ethyl | 2,4-Difluor-benzoyl | (2,4-Difluor-phenyl)-[3-(1-ethyl-1H-indol-3-yl)-pyrrolidin-1-yl]-methanon |
| 74 | 4-Trifluormethyl benzyl | 2,3-Dimethyl-benzoyl | (2,3-Dimethyl-phenyl)-{3-[1-(4-trifluormethyl-benzyl)-1H-indol-3-yl]-pyrrolidin-1-yl}-methanon |
| 75 | n-Butyl | 3-Nitro-4-methyl-benzoyl | [3-(1-Butyl-1H-indol-3-yl)-pyrrolidin-1-yl]-(4-methyl-3-nitro-phenyl)-methanon |
| 76 | 3-Methoxy-benzyl | 4-Brom-3-methyl-benzoyl | (4-Brom-3-methyl-phenyl)-{3-[1-(3-methoxy-benzyl)-1H-indol-3-yl]-pyrrolidin-1-yl}-methanon |
| 77 | n-Butyl | (4-Chlor-phenoxy)-acetyl | 1-[3-(1-Butyl-1H-indol-3-yl)-pyrrolidin-1-yl]-2-(4-chlor-phenoxy)-ethanon |
| 78 | 3-Fluorbenzyl | (4-Chlor-phenyl)-acetyl | 2-(4-Chlor-phenyl)-1-{3-[1-(3-fluor-benzyl)-1H-indol-3-yl]-pyrrolidin-1-yl}-ethanon |
| 79 | 4-Trifluormethyl benzyl | 4-Chlor-benzoyl | (4-Chlor-phenyl)-{3-[1-(4-trifluormethyl-benzyl)-1H-indol-3-yl]-pyrrolidin-1-yl}-methanon |
| 80 | Benzyl | 5-Methyl-isoxazol-3-carbonyl | [3-(1-Benzyl-1H-indol-3-yl)-pyrrolidin-1-yl]-(5-methyl-isoxazol-3-yl)-methanon |
| 81 | Benzyl | (4-Chlor-phenoxy)-acetyl | 1-[3-(1-Benzyl-1H-indol-3-yl}-pyrrolidin-1-yl]-2-(4-chlor-phenoxy)-ethanon |
| 82 | Methyl-naphtyl | 4-Trifluormethyl-benzoyl | [3-(1-Naphthalen-2-ylmethyl-1H-indol-3-yl)-pyrrolidin-1-yl]-(4-trifluormethyl-phenyl)-methanon |
| 83 | 4-Trifluormethyl benzyl | 3-Methoxy-benzoyl | (3-Methoxy-phenyl)-{3-[1-(4-trifluormethyl-benzyl)-1H-indol-3-yl]-pyrrolidin-1-yl}-methanon |
| 84 | n-Butyl | 3-(2-Chlor-phenyl)-acryloyl | 1-[3-(1-Butyl-1H-indol-3-yl)-pyrrolidin-1-yl]-3-(2-chlor-phenyl)-propenon |
| 85 | Methyl | 2-Phenyl-butyryl | 1-[3-(1-Methyl-1H-indol-3-yl)-pyrrolidin-1-yl]-2-phenyl-butan-1-on |
| 86 | Methyl | 2-Chlor-6-fluor-3-methyl-benzoyl | (2-Chlor-6-fluor-3-methyl-phenyl)-[3-(1-methyl-1H-indol-3-yl)-pyrrolidin-1-yl]-methanon |
| 87 | 2-*N,N*-Dimethyl-acetamid | 3-Methoxy-benzoyl | 2-{3-[1-(3-Methoxy-benzoyl)-pyrrolidin-3-yl]-indol-1-yl}-N,N-dimethyl-acetamid |
| 88 | 2-*N,N*-Dimethyl-acetamid | 2-Fluor-benzoyl | 2-{3-[1-(2-Fluor-benzoyl)-pyrrolidin-3-yl]-indol-1-yl}-N,N-dimethyl-acetamid |
| 89 | Methyl-naphtyl | 3-Difluor-methyl-sulfanyl-benzoyl | (3-Difluormethylsulfanyl-phenyl)-[3-(1-naphthalen-2-ylmethyl-1H-indol-3-yl)-pyrrolidin-1-yl]-methanon |
| 90 | 3-Fluorbenzyl | Benzo[1,2,5] oxadiazol-5-carbonyl | Benzo[1,2,5]oxadiazol-5-yl-{3-[1-(3-fluor-benzyl)-1H-indol-3-yl]-pyrrolidin-1-yl}-methanon |
| 91 | 4-Trifluormethyl -benzyl | 2-Methyl-benzoyl | o-Tolyl-{3-[1-(4-trifluormethyl-benzyl)-1H-indol-3-yl]-pyrrolidin-1-yl}-methanon |
| 92 | 2-*N,N*-Dimethyl-acetamid | 2-Trifluormethyl-benzyl-sulfonyl | N,N-Dimethyl-2-{3-[1-(2-trifluormethyl-benzensulfonyl)-pyrrolidin-3-y1]-indol-1-yl}-acetamid |
| 93 | n-Butyl | 2-Phenoxy-propionyl | 1-[3-(1-Butyl-1H-indol-3-yl)-pyrrolidin-1-yl]-2-phenoxy-propan-1-on |
| 94 | Methyl-naphtyl | 2-Chlor-6-fluor-3-methyl-benzoyl | (6-Chlor-2-fluor-3-methyl-phenyl)-[3-(1-naphthalen-2-ylmethyl-1H-indol-3-yl)-pyrrolidin-1-yl]-methanon |
| 95 | 2-Propenyl | 4-Trifluormethyl-2-methyl-nicotinoyl | [3-(1-Allyl-1H-indol-3-yl)-pyrrolidin-1-yl]-(2-methyl-6-trifluormethyl-pyridin-3-yl)-methanon |
| 96 | n-Butyl | 4-Propyl-benzoyl | [3-(1-Butyl-1H-indol-3-yl)-pyrrolidin-1-yl]-(4-propyl-phenyl)-methanon |
| 97 | 2-*N,N*- Dimethyl-acetamid | 4-Chlor-benzoyl | 2-{3-[1-(4-Chlor-benzoyl)-pyrrolidin-3-yl]-indol-1-yl}-N,N-dimethyl-acetamid |
| 98 | 3-Fluor-benzyl | 3-Phenyl-propionyl | 1-{3-[1-(3-Fluor-benzyl)-1H-indol-3-yl]-pyrrolidin-1-yl}-3-phenyl-propan-1-on |
| 99 | 2-Fluor-benzyl | 1-Phenyl-5-propyl-1H-pyrazol-4-carbonyl | {3-[1-(2-Fluor-benzyl)-1H-indol-3-yl]-pyrrolidin-1-yl}-(1-phenyl-5-propyl-1H-pyrazol-4-yl)-methanon |
| 100 | 3-Cyanobenzyl | 5-*tert*-Butyl-2-methyl-furan-3-carbonyl | 3-{3-[1-(5-*tert*-Butyl-2-methyl-furan-3-carbonyl)-pyrrolidin-3-yl]-indol-1-ylmethyl}-benzonitril |
| 101 | 2-Fluorbenzyl | 2,4-Difluor-sulfanyl | 3-[1-(2,4-Difluor-benzensulfonyl)-pyrrolidin-3-yl]-1-(2-fluor-benzyl)-1H-indol |
| 102 | 2-*N,N*-Dimethyl-acetamid | 2,6-Difluor- benzyl-sulfanyl | 2-{3-[1-(2,6-Difluor-benzoyl)-pyrrolidin-3-yl]-indol-1-yl}-N,N-dimethyl-acetamid |
| 103 | n-Butyl | Butyryl | 1-[3-(1-Butyl-1H-indol-3-yl)-pyrrolidin-1-yl]-butan-1-on |
| 104 | 4-Cyanobenzyl | 2-Propyl-pentanoyl | 4-{3-[1-(2-Propyl-pentanoyl)-pyrrolidin-3-yl]-indol-1-ylmethyl}-benzonitril |
| 105 | 2-Propinyl | (4-Chlor-phenoxy)-acetyl | 2-(4-Chlor-phenoxy)-1-[3-(1-prop-2-ynyl-1H-indol-3-yl)-pyrrolidin-1-yl]-ethanon |
| 106 | Methyl | Nicotinoyl | [3-(1-Methyl-1H-indol-3-yl)-pyrrolidin-1-yl]-pyridin-3-yl-methanon |
| 107 | 3-Cyanobenzyl | Benzo[1,3]di-oxol-5-carbonyl | 3-{3-[1-(Benzo[1,3]dioxole-5-carbonyl)-pyrrolidin-3-yl]-indol-1-ylmethyl}-benzonitril |
| 108 | n-Butyl | 2-Chlor-5-trifluor-methyl-benzoyl | [3-(1-Butyl-1H-indol-3-yl)-pyrrolidin-1-yl]-(2-chlor-5-trifluormethyl-phenyl)-methanon |
| 109 | 4-Trifluormethyl benzyl | 3-Chlor-benzoyl | (3-Chlor-phenyl)-{3-[1-(4-trifluormethyl-benzyl)-1H-indol-3-yl]-pyrrolidin-1-yl}-methanon |
| 110 | 2-*N,N*-Dimethyl-acetamid | 4-Brom-benzoyl | 2-{3-[1-(4-Brom-benzoyl)-pyrrolidin-3-yl]-indol-1-yl}-N,N-dimethyl-acetamid |
| 111 | 2-Propinyl | 3-Phenyl-acryloyl | 3-Phenyl-1-[3-(1-prop-2-ynyl-1H-indol-3-yl)-pyrrolidin-1-yl]-propenon |
| 112 | 3-Cyano-benzyl | 3-Chlor-2-fluor-benzoyl | 3-{3-[1-(3-Chlor-2-fluor-benzoyl)-pyrrolidin-3-yl]-indol-1-ylmethyl}-benzonitril |
| 113 | 3-Cyano-benzyl | 2-Phenyl-cyclopropan-carbonyl | 3-{3-[1-(2-Phenyl-cyclopropanecarbonyl)-pyrrolidin-3-yl]-indol-1-ylmethyl}-benzonitril |
| 114 | 3-Cyano-benzyl | 3-Chlor-thiophen-2-carbonyl | 3-{3-[1-(3-Chlor-thiophen-2-carbonyl)-pyrrolidin-3-yl]-indol-1-ylmethyl}-benzonitril |
| 115 | 2-Propinyl | 2-Trifluormethyl- benzyl-sulfanyl | 1-Prop-2-ynyl-3-[1-(2-trifluormethyl-benzensulfonyl)-pyrrolidin-3-yl]-1H-indol |
| 116 | 2-Fluorbenzyl | 3-Trifluormethyl-benzyl-sulfanyl | 1-(2-Fluor-benzyl)-3-[1-(3-trifluormethyl-benzensulfonyl)-pyrrolidin-3-yl]-1H-indol |
| 117 | Benzyl | 6-Chlor-2H-chromen-3-carbonyl | [3-(1-Benzyl-1H-indol-3-yl)-pyrrolidin-1-yl]-(6-chlor-2H-chromen-3-yl)-methanon |
| 118 | Benzyl | 3,4-Difluor-benzoyl | [3-(1-Benzyl-1H-indol-3-yl)-pyrrolidin-1-yl]-(3,4-difluor-phenyl)-methanon |

### Beispiel 2: Biologische Testung

### a) Untersuchungen zur Serotonin-Wiederaufnahmehemmung (5HT-Uptake-Hemmung)

Um diese in vitro Studien durchführen zu können, werden Synaptosomen aus Rattenhirnarealen frisch isoliert. Es findet jeweils eine sogenannte "P₂"-Fraktion Verwendung, die nach der Vorschrift von Gray und Whittaker (E.G. Gray und V.P. Whittaker (1962) J. Anat. 76, 79-88) präpariert wird. Für den 5HT-Uptake werden diese vesikulären Partikel aus dem Hypothalamus männlicher Rattengehirne isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

### b) Untersuchungen zur Noradrenalin-Wiederaufnahmehemmung (NA-Uptake-Hemmung)

Um diese in vitro Studien durchführen zu können, werden Synaptosomen aus Rattenhirnarealen frisch isoliert. Es findet jeweils eine sogenannte "P₂"-Fraktion Verwendung, die nach der Vorschrift von Gray und Whittaker (E.G. Gray und V.P. Whittaker (1962) J. Anat. 76, 79-88) präpariert wird. Für den NA-Uptake werden diese vesikulären Partikel aus dem Hypothalamus männlicher Rattengehirne isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

### c) Bindungsuntersuchungen am Natriumkanal

### Bindungsstelle 2 (BTX-Bindung):

Die Bindungsstelle 2 des Natriumkanals ist die sogenannte Batrachotoxin-(BTX) Bindungsstelle. Als Ligand wurde [³H]-Batrachotoxinin A20 α-Benzoat (10 nM im Ansatz) eingesetzt. Diese lonenkanal-Partikel (Synaptosomen) wurden aus dem Ratten Cerebrocortex nach Gray und Whittaker (E.G. Gray und V.P. Whittaker (1962) J. Anat. 76, 79-88) angereichert. Als unspezifische Bindung ist die Radioaktivität definiert, die in Gegenwart von Veratridin (0,3 mM im Ansatz) gemessen wird. Inkubation bei 25°C für 120 min. Die Assaybedingungen sind nach der Veröffentlichung von Pauwels, Leysen und Laduron (P.J. Pauwels, J.E. Leysen und P.M. Laduron (1986) Eur. J. Pharmacol. 124, 291-298) durchgeführt worden.

Der K_{D}-Wert für diese Bindungsstelle liegt bei 24,63 ± 1,56 nM. (N = 3, d.h. Mittelwerte ± SEM aus 3 unabhängigen Versuchsreihen, die in Dreifach-Parallelversuchen durchgeführt worden sind).
a) Hemmung der Serotonin-Wiederaufnahme

| **Verbindung** | **5HT-Uptake,** |
|---|---|
| | **% Hemmung [10µM]** |
| 17 | 43 |
| 18 | 49 |
| 19 | 86 |
| 20 | 89 |
| 21 | 84 |
| 22 | 58 |
| 23 | 49 |
| 24 | 53 |
| 25 | 80 |
| 26 | 62 |
| 27 | 70 |
| 28 | 41 |
| 29 | 63 |
| 30 | 47 |
| 31 | 62 |
| 33 | 87 |
| 34 | 57 |
| 35 | 68 |
| 36 | 42 |
| 37 | 64 |
| 39 | 43 |
| 40 | 66 |
| 41 | 44 |
| 42 | 82 |
| 43 | 48 |
| 44 | 60 |
| 45 | 80 |
| 46 | 46 |
| 47 | 46 |
| 48 | 43 |
| 49 | 68 |
| 50 | 83 |
| 52 | 79 |
| 53 | 55 |

b) Hemmung der Noradrenalin-Wiederaufnahme

| **Verbindung** | **NA-Uptake,** |
|---|---|
| | **% Hemmung [10µM]** |
| 16 | 50 |
| 18 | 49 |
| 20 | 43 |
| 24 | 59 |
| 26 | 44 |
| 28 | 52 |
| 31 | 41 |
| 32 | 44 |
| 34 | 49 |
| 35 | 48 |
| 37 | 43 |
| 38 | 49 |
| 39 | 41 |
| 40 | 43 |
| 41 | 43 |
| 42 | 58 |
| 44 | 49 |
| 45 | 43 |
| 46 | 20 |
| 48 | 40 |
| 49 | 100 |
| 51 | 57 |
| 52 | 54 |

*c) Bindung an die Bindungsstelle* 2 *des Natriumkanals*

| **Verbindung** | **BTX-Bindung,** |
|---|---|
| | **% Hemmung** |
| | **[10µM]** |
| 1 | 95 |
| 2 | 93 |
| 3 | 93 |
| 4 | 92 |
| 5 | 92 |
| 6 | 91 |
| 7 | 89 |
| 8 | 88 |
| 9 | 84 |
| 10 | 83 |
| 11 | 83 |
| 12 | 83 |
| 13 | 82 |
| 14 | 80 |
| 15 | 80 |
| 20 | 91 |
| 26 | 93 |
| 31 | 92 |
| 33 | 89 |
| 34 | 91 |
| 35 | 84 |
| 36 | 96 |
| 38 | 89 |
| 39 | 90 |
| 42 | 96 |
| 43 | 92 |
| 45 | 82 |
| 46 | 88 |

## Patentansprüche

1. Substituierte 3-Pyrrolidin-Indol-Derivate der allgemeinen Formel I 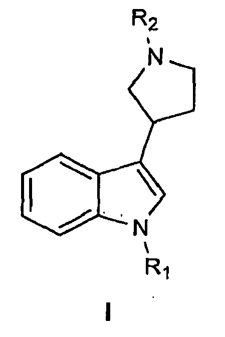 worin
der Rest R¹ für H; C₁₋₈-Alkyl, jeweils gesättigt oder ungesättigt, verzweigt oder unverzweigt, unsubstituiert oder einfach oder mehrfach substituiert; oderüber C₁₋₃-Alkyl gebundenes Aryl, C₃₋₈-Cycloalkyl oder Heteroaryl, jeweils einfach oder mehrfach substituiert oder unsubstituiert; CH₂CONR³R⁴ steht;
der Rest R² für COR⁵; SO2R⁶; CSNHR⁷; CONHR⁸ steht
die Reste R³ und R⁴ unabhängig voneinander für C₁₋₈-Alkyl, jeweils gesättigt oder ungesättigt, verzweigt oder unverzweigt, unsubstituiert oder einfach oder mehrfach substituiert stehen;
oder
die Reste R³ und R⁴ zusammen CH₂CH₂OCH₂CH₂, oder (CH₂)₃₋₆ bedeuten;
der Rest R⁵ für C₁₋₈-Alkyl, verzweigt oder unverzweigt, gesättigt oder ungesättigt, substituiert oder unsubstituiert; Aryl, substituiert oder unsubstituiert; Heteroaryl, substituiert oder unsubstituiert; Cycloalkyl oder Heterocyclyl, jeweils gesättigt oder ungesättigt, substituiert oder unsubstituiert;
oder 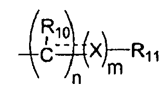 mitn=1,2,3;m=0, 1;X=O oder NH und 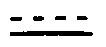 in der Bedeutung einer Einfachbindung oder einer Doppelbindung, steht
der Rest R⁶ für Aryl, jeweils substituiert oder unsubstituiert, Heteroaryl, jeweils substituiert oder unsubstituiert, C₁₋₈- Alkyl, jeweils gesättigt oder ungesättigt, verzweigt oder unverzweigt, unsubstituiert oder einfach oder mehrfach substituiert; C₃₋₈ Cycloalkyl, substituiert oder unsubstituiert, gesättigt oder ungesättigt; oder über C₁₋₃-Alkyl gebundenes Aryl, C₃₋₈-Cycloalkyl oder Heteroaryl, jeweils einfach oder mehrfach substituiert oder unsubstituiert steht
der Rest R⁷ für Aryl, jeweils substituiert oder unsubstituiert, Heteroaryl, jeweils substituiert oder unsubstituiert, C₁₋₈- Alkyl, jeweils gesättigt oder ungesättigt, verzweigt oder unverzweigt, unsubstituiert oder einfach oder mehrfach substituiert; C₃₋₈ Cycloalkyl, substituiert oder unsubstituiert, gesättigt oder ungesättigt; oder über C₁₋₃-Alkyl gebundenes Aryl, C₃₋₈-Cycloalkyl oder Heteroaryl, jeweils einfach oder mehrfach substituiert oder unsubstituiert steht,
der Rest R⁸ für C₁₋₈-Alkyl, verzweigt oder unverzweigt, substituiert oder unsubstituiert, gesättigt oder ungesättigt; C₃₋₈-Cycloalkyl, substituiert oder unsubstituiert, gesättigt oder ungesättigt; trifluormethyl- oder nitrosubstituiertes Phenyl, Pyrrolyl, Indolyl, Furyl, Benzofuranyl, Thienyl, Benzothienyl, Pyrazolyl, Imidazolyl, Thiazolyl, Oxazolyl, Isoxazoyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Oxadiazolyl, Chromenyl oder Phenothiazinyl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, steht;
R¹⁰ für H; C₁₋₈-Alkyl, verzweigt oder unverzweigt, substituiert oder unsubstituiert, gesättigt oder ungesättigt; Aryl oder Heteroaryl, jeweils substituiert oder unsubstituiert, Benzyl oder Phenethyl, jeweils substituiert oder unsubstituiert, steht
R¹¹ für H; C₁₋₈-Alkyl verzweigt oder unverzweigt, substituiert oder unsubstituiert, gesättigt oder ungesättigt; Aryl oder Heteroaryl, jeweils substituiert oder unsubstituiert, oder über C₁₋₃-Alkyl gebundenes Aryl, C₃₋₈-Cycloalkyl oder Heteroaryl, jeweils einfach oder mehrfach substituiert oder unsubstituiert steht,
worin Alkyl, Alkanyl, Alkenyl und Alkinyl substituiert sein können durch F, Cl, Br, I, -CN, -N=C, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, CO₂-Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, SO-Alkyl, SO₂-Alkyl, SO₂NH₂, SO₃H, PO(O-C₁₋₆-Alkyl)₂, Si(C₁₋₆-Alkyl)₃, Si(C₃₋₈-Cycloalkyl)₃, Si(CH₂-C₃₋₈-Cycloalkyl)₃, Si(Phenyl)₃, Cycloalkyl, Aryl, Heteroaryl oder Heterocyclyl,
und Aryl, Heterocyclyl, Heteroaryl sowie Cycloalkyl substituert sein können durch F, Cl, Br, I, CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Cycloalkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Cycloalkyl, O-Aryl, O-Heteroaryl, 0-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)-C₁₋₆-Alkyl-Aryl, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, CO₂Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, S(O)-Alkyl, S(O)-Aryl, SO₂-Alkyl, SO₂-Aryl, SO₂NH₂, SO₃H, CF₃, =O, =S; Alkyl, Cycloalkyl, Aryl, Heteroaryl und/oder Heterocyclyl;
mit der Maßgabe, dass wenn R² Cyclohexyl bedeutet, der Cyclohexylring nicht in 4-Position mit NR^{x}R^{y} und R^{z} substituiert sein darf,
wobei R^{x} und R^{y} unabhängig voneinander für H; C₁₋₈-Alkyl oder C₃₋₈-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, stehen,
oder die Reste R^{x} und R^{y} zusammen einen Ring bilden und CH₂CH₂OCH₂CH₂, CH₂CH₂NRⁿCH₂CH₂ oder (CH₂)₃₋₆ bedeuten,
wobei Rⁿ H; C₁₋₈-Alkyl oder C₃₋₈-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, bedeutet;
und
R^{Z} für C₁₋₈-Alkyl oder C₃₋₈-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte C₁₋₄-Alkyl-Gruppe gebundenem Aryl, C₃₋₈-Cycloalkyl oder Heteroaryl, jeweils unsubstituiert oder einfach oder mehrfach substituiert, steht;
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

2. Substituierte 3-Pyrrolidin-Indol-Derivate gemäß Anspruch 1, **dadurch gekennzeichnet, dass** der Rest R² für COR⁵, SO2R⁶ oder CSNHR⁷ steht.

3. Substituierte 3-Pyrrolidin-Indol-Derivate gemäß Anspruch 1, **dadurch gekennzeichnet, dass** der Rest R² für CONHR⁸ steht.

4. Substituierte 3-Pyrrolidin-Indol-Derivate gemäß den Ansprüchen 1-3, **dadurch gekennzeichnet, dass** der Rest R¹ für H; C₁₋₈-Alkyl, jeweils gesättigt oder ungesättigt, verzweigt oder unverzweigt, unsubstituiert oder einfach oder mehrfach substituiert; Benzyl oder Methylnaphthyl, jeweils einfach oder mehrfach substituiert oder unsubstituiert, insbesondere Benzyl unsubstituiert oder einfach oder mehrfach mit CF₃, Br, F, CN, OCH₃ oder CH₃ substituiert; CH₂CONR³R⁴ steht;

5. Substituierte 3-Pyrrolidin-Indol-Derivate gemäß den Ansprüchen 1, 2 oder 4, **dadurch gekennzeichnet, dass** der Rest R² für COR⁵ steht und R⁵ 4-Propylphenyl, 3,4-Dimethoxyphenyl, 2-Methyl-4-trifluormethylphenyl-3-pyridin, Ethenylphenyl, 2,3-Difluorphenyl, 4-tert.-Butylphenyl, 2-Ethoxyphenyl, 3-Fluor-4-trifluormethylphenyl, 2,3-Dimethylphenyl, Phenoxyethyl, Phenoxymethyl, 3,4-Dichlorphenyl, 4-Trifluormethylsulfanylphenyl, 2,5-Dimethoxyphenyl, 2-Chlor-4-nitrophenyl, 2-Chlorphenyl, 4-Methyl-N-phenethyl-benzylsulfonamid, 2-Chlor-5-fluor-3-methylphenyl, 3-(2-Chlorphenyl)-5-methyl-isoxazol, 5-tert.-Butyl-2-methylfuran, Benzo[1,2,5]oxadiazol, Phenylpropyl, 2-Methylsulfanyl-3-pyridin, 2-Chlor-5-trifluormethylphenyl, Methoxymethyl, 4-Methylphenyl, Biphenyl, 4-Chlorbenzyl, 2,3,4,5,6-Pentafluorphenyl, 3-Chlorphenyl, 4-Trifluormethylphenyl, 2,6-Dlfluor-3-methylphenyl, 2-Methylphenyl, 4-Fluorphenyl, 6-Chlorchromen, 2-Chlor-6-fluorphenyl, Ethylcyclopentyl, 1-(4-Chlorphenyl)-5-trifluormethylpyrazol, 2,4-Dichlorphenyl, 2,3-Dimethylphenyl, 3-Nitro-4-methylphenyl, 4-Brom-3-methylphenyl, (4-Chlorphenoxy)methyl, 4-Chlorphenyl, 5-Methylisoxazol, 3-Methoxyphenyl, 2-Chlorphenylethenyl, 2-Chlor-4-fluor-3-methylphenyl, 2-Fluorphenyl, 3-Difluormethylsulfanylphenyl, 2-Fluor-3-chlorphenyl, Cyclopropylphenyl, 1-Phenyl-5-propylpyrazol, 2,6-Difluorphenyl, Benzo[1,3]-dioxol, 4-Bromphenyl, 3-Chlorthiophenyl, 2-, 3- oder 4-Pyridin, Phenyl oder 3,4-Difluorphenyl bedeutet.

6. Substituierte 3-Pyrrolidin-Indol-Derivate gemäß den Ansprüchen 1, 2 oder 4, **dadurch gekennzeichnet, dass** der Rest R² für SO₂R⁶ steht und R⁶ 2,4,6-Dimethylphenyl, 4-Nitrophenyl, Benzyl, 4-Propylphenyl, 4-Chlorphenyl, 2-Trifluormethylphenyl, 2,4-Difluorphenyl oder 3-Trifluormethylphenyl bedeutet.

7. Substituierte 3-Pyrrolidin-Indol-Derivate gemäß den Ansprüchen 1, 2 oder 4, **dadurch gekennzeichnet, dass** der Rest R² für CSNHR⁷ steht und der Rest R⁷ 3-Trifluormethylphenyl bedeutet.

8. Substituierte 3-Pyrrolidin-Indol-Derivate gemäß den Ansprüchen 1, 3 oder 4, **dadurch gekennzeichnet, dass** der Rest R² für CONHR⁸ steht und der Rest R⁸ 3,4-Dichlorbenzyl bdeutet.

9. Substituierte 3-Pyrrolidin-Indol-Derivate gemäß den Ansprüchen 1-8, ausgewählt aus der Gruppe
• (4-Propyl-phenyl)-{3-[1-(4-triflüormethyl-benzyl)-1H-indol-3-yl]-pyrrolidin-1-yl}-methanen
• (3,4-Dimethoxy-phenyl)-{3-[1-(3-methyl-butyl)-9H-indol-3-yl]-pyrrolidin-1-yl}-methanon
• [3-(1-Butyl-1H-indol-3-yl)-pyrrolidin-1-yl]-(2-methyl-6-trifluormethyl-pyridin-3-yl)-methanon
• 1-{3-[1-(3-Methoxy-benzyl)-1H-indol-3-yl]-pyrrolidin-1-yl}-3-phenyl-propenon
• (2,3-Difluor-phenyl)-{3-[1-(4-trifluormethyl-benzyl)-1H-indol-3-yl]-pyrrolidin-1-yl}-methanon
• (4-tert-Butyl-phenyl)-{3-[1-(3-methyl-butyl)-1H-indol-3-yl]-pyrrolidin-1-yl}-methanon
• {3-[1-(4-Brom-benzyl)-1H-indof-3-yl]-pyrrolidin-1-yl}-(4-propyl-phenyl)-methanon
• (2-Ethoxy-phenyl)-{3-[1-(3-methoxy-benzyl)-1H-indol-3-yl]-pyrrolidin-1-yl}-methanon
• [3-(1-Ethyl-1H-indol-3-yl)-pyrrolidin-1-yl]-(3-fluor-4-trifluormethyl-phenyl)-methanon
• (2,3-Dimethyl-phenyl)-[3-(1-ethyl-1H-indol-3-yl)-pyrrolidin-1-yl]-methanon
• 1-{3-[1-(3-Methoxy-benzyl)-1H-indol-3-yl]-pyrrolidin-1-yl}-2-phenoxy-propan-1-on
• 2-(3-Chlor-phenoxy)-1-[3-(1-ethyl-1H-indol-3-yl)-pyrrolidin-1-yl]-ethanon
• 4-{3-[1-(2-Phenoxy-propionyl)-pyrrolidin-3-yl]-indol-1-ylmethyl}-benzonitril
• N,N-Dimethyl-2-{3-[1-(2,4,6-trimethyl-benzensulfonyl)-pyrrolidin-3-yl]-indol-1-yl}-acetamid
• (3,4-Dichlor-phenyl)-[3-(1-ethyl-1H-indol-3-yl)pyrrolidin-1-yl]-methanon
• N,N-Dimethyl-2-{3-[1-(3-trifluormethyl-phenylthiocarbamoyl)-pyrrolidin-3-yl]-indol-1-yl}-acetamid
• 3-(1-Dimethylcarbamoylmethyl-1H-indol-3-yl)-pyrrolidin-1-carbonsäure 3,4-dichlor-benzylamid
• [3-(1H-Indol-3-yl)-pyrrolidin-1-yl]-(4-trifluormethylsulfanyl-phenyl)-methanon
• 2-(3-{1-[2-(2,5-Dimethoxy-phenyl)-acetyl]-pyrrolidin-3-yl}-indol-1-yl)-N,N-dimethyl-acetamid
• (2-Chlor-4-nitro-phenyl)-{3-[1-(3-fluor-benzyl)-1H-indol-3-yl]-pyrrolidin-1-yl}-methanon
• N,N-Dimethyt-2-{3-[1-(pyridin-2-carbonyl)-pyrrolidin-3-yl]-indol-1-yl}-acetamid
• Cyclobutyl-[3-(1H-indol-3-yl)-pyrrolidin-1-yl]-methanon
• N,N-Diethyl-2-[3-(1-pentanoyl-pyrrolidin-3-yl)-indol-1-yl]-acetamid
• 3-[1-(4-Nitro-benzensulfonyl)-pyrrolidin-3-yl]-1H-indol
• 2-[3-(1-Cyclopentanecarbonyl-pyrrolidin-3-yl)-indol-1-yl]-N,N-diethyl-acetamid
• 1-[3-(1-Methyl-1H-indol-3-yl)-pyrrolidin-1-yl]-3-phenyl-propenon
• (2-Chlor-phenyl)-[3-(1H-indol-3-yl)-pyrrolidin-1-yl]-methanon
• [3-(1-Ethyl-1H-indol-3-yl)pyrrolidin-1-yl]-pyridin-3-yl-methanon
• N,N-Dimethyl-2-(3-{1-[3-phenyl-2-(toluene-4-sulfonylamino)-propionyl]-pyrrolidin-3-yl}-indol-1-yl)-acetamid
• N,N-Dimethyl-2-[3-(1-phenylmethanesulfonyl-pyrrolidin-3-yl)-indol-1-yl]-acetamid
• 4-{3-[1-(2-Chlor-6-fluor-3-methyl-benzoyl)-pyrrolidin-3-yl]-indol-1-ylmethyl}-benzonitril
• [3-(2-Chlor-phenyl)-5-methyl-isoxazol-4-yl]-[3-(1-ethyl-1H-indol-3-yl)-pyrrolidin-1-yl]-methanon
• 2-{3-[1-(5-tert-Butyl-2-methyl-furan-3-carbonyl)-pyrrolidin-3-yl]-indol-1-ylmethyl}-benzonitril
• 4-{3-[1-(Benzo[1,2,5]oxadiazole-5-carbonyl)-pyrrolidin-3-yl]-indol-1-ylmethyl}-benzonitril
• 1-[3-(1-Ethyl-1H-indol-3-yl)-pyrrolidin-1-yl]-3-phenyl-propan-1-on
• 1-{3-[1-(3-Methoxy-benzyl)-1H-indol-3-yl]-pyrrolidin-1-yl}-3-phenyl-propan-1-on
• [3-(1-Methyl-1H-indol-3-yl)-pyrrolidin-1-yl]-(2-methylsulfanyl-pyridin-3-yl)-methanon
• (2-Chlor-5-trifluormethyl-phenyl)-[3-(1-ethyl-1H-indol-3-yl)-pyrrolidin-1-yl]-methanon
• (2-Chlor-4-nitro-phenyl)-{3-[1-(4-trifluormethyl-benzyl)-1H-indol-3-yl]-pyrrolidin-1-yl}-methanon
• (2-Chlor-pyridin-3-yl)-[3-(1-methyl-1H-indol-3-yl)-pyrrolidin-1-yl]-methanon
• 2-Methoxy-1-{3-[1-(3-methoxy-benzyl)-1H-indol-3-yl]-pyrrolidin-1-yl}-ethanon
• {3-[1-(3-Fluor-benzyl)-1H-indol-3-yl]-pyrrolidin-1-yl}-(2-methyl-6-trifluormethyl-pyridin-3-yl)-methanon
• {3-[1-(3-Methoxy-benzyl)-1H-indol-3-yl]-pyrrolidin-1-yl}-p-tolyl-methanon
• (2-Methylsulfanyl-pyridin-3-yl)-[3-(1-prop-2-ynyl-1H-indol-3-yl)-pyrrolidin-1-yl]-methanon
• Biphenyl-4-yl-[3-(1-methyl-1H-indol-3-yl)-pyrrolidin-1-yl]-methanon
• 2-(4-Chlor-phenyl)-1-{3-[1-(3-methoxy-benzyl)-1H-indol-3-yl]-pyrrolidin-1-yl}-ethanon
• [3-(1-Methyl-1H-indol-3-yl)-pyrrolidin-1-yl]-pentafluorphenyl-methanon
• [3-(1-Ethyl-1H-indol-3-yl)-pyrrolidin-1-yl]-(4-trifluormethyl-phenyl)-methanon
• N,N-Dimethyl-2-{3-[1-(4-propyl-benzensulfonyl)-pyrrolidin-3-yl]-indol-1-yl}-acetamid
• (2-Chlor-4-nitro-phenyl)-{3-[1-(3-methoxy-benzyl)-1H-indol-3-yl]-pyrrolidin-1-yl}-methanon
• 3-{3-[1-(4-Chlor-benzensulfonyl)-pyrrolidin-3-yl]-indol-1-ylmethyl}-benzonitril
• 1-{3-[1-(3-Methoxy-benzyl)-1H-indol-3-yl]-pyrrolidin-1-yl}-3,3-dimethyl-butan-1-on
• 2-(4-Chlor-phenyl)-1-[3-(1-methyl-1H-indol-3-yl)-pyrrolidin-1-yl]-ethanon
• (2,6-Difluor-3-methyl-phenyl)-{3-[1-(3-methoxy-benzyl)-1H-indol-3-yl]-pyrrolidin-1-yl}-methanon
• [3-(1-Ethyl-1H-indol-3-yl)-pyrrolidin-1-yl]-o-tolyl-methanon
• [3-(1-Ethyl-1H-indol-3-yl)-pyrrolidin-1 -yl]-(4-tluor-phenyl)-methanon
• [3-(1-Butyl-1H-indol-3-yl)-pyrrolidin-1-yl]-(6-chlor-2H-chromen-3-yl)-methanon
• (2-Chlor-6-fluor-phenyl)-[3-(1-naphthalen-2-ylmethyl-1H-indol-3-yl)-pyrrolidin-1-yl]-methanon
• 2-{3-[1-(3-Cyclopentyl-propionyl)-pyrrolidin-3-yl]-indol-1-yl}-N,N-dimethyl-acetamid
• [3-(2-Chlor-phenyl)-5-methyl-isoxazol-4-yl]-{3-[1-(3-methoxy-benzyl)-1H-indol-3-yl]-pyrrolidin-1-yl}-methanon
• [1-(4-Chlor-phenyl)-5-trifluormethyl-1H-pyrazol-4-yl]-{3-[1-(3-fluor-benzyl)-1H-indol-3-yl]-pyrrolidin-1-yl}-methanon
• [3-(1-Allyl-1H-indol-3-yl)-pyrrolidin-1-yl]-p-tolyl-methanon
• {3-[1-(4-Fluor-benzyl)-1H-indol-3-yl]-pyrrolidin-1-yl}-pyridin-2-yl-methanon
• (3,4-Dichlor-phenyl)-{3-[1-(4-trifluormethyl-benzyl)-1H-indol-3-yl]-pyrrolidin-1-yl}-methanon
• 1-Butyl-3-[1-(2,5-dimethoxy-benzensulfonyl)-pyrrolidin-3-yl]-1H-indol
• 1-[3-(1-Allyl-1H-indol-3-yl)-pyrrolidin-1-yl]-3,3-dimethyl-butan-1-on
• 4-{3-[1-(2-Methoxy-acetyl)-pyrrolidin-3-yl]-indol-1-ylmethyl}-benzonitril
• (2,4-Difluor-phenyl)-[3-(1-ethyl-1H-indol-3-yl)-pyrrolidin-1-yl]-methanon
• (2,3-Dimethyl-phenyl)-{3-[1-(4-trifluormethyl-benzyl)-1H-indol-3-yl]-pyrrolidin-1-yl}-methanon
• [3-(1-Butyl-1H-indol-3-yl)-pyrrolidin-1-yl]-(4-methyl-3-nitro-phenyl)-methanon
• (4-Brom-3-methyl-phenyl)-{3-[1-(3-methoxy-benzyl)-1H-indol-3-yl]-pyrrolidin-1-yl}-methanon
• 1-[3-(1-Butyl-1H-indol-3-yl)-pyrrolidin-1-yl]-2-(4-chlor-phenoxy)-ethanon
• 2-(4-Chlor-phenyl)-1-{3-[1-(3-fluor-benzyl)-1H-indol-3-yl]-pyrrolidin-1-yl}-ethanon
• (4-Chlor-phenyl)-{3-[1-(4-trifluormethyl-benzyl)-1H-indol-3-yl]-pyrrolidin-1-yl}-methanon
• [3-(1-Benzyl-1H-indol-3-yl)-pyrrolidin-1-yl]-(5-methyl-isoxazol-3-yl)-methanon
• 1-[3-(1-Benzyl-1H-indol-3-yl)-pyrrolidin-1-yl]-2-(4-chlor-phenoxy)-ethanon
• [3-(1-Naphthalen-2-ylmethyl-1H-indol-3-yl)-pyrrolidin-1-yl]-(4-trifluormethylphenyl)-methanon
• (3-Methoxy-phenyl)-{3-[1-(4-trifluormethyl-benzyl)-1H-indol-3-yl]-pyrrolidin-1-yl}-methanon
• 1-[3-(1-Butyl-1H-indol-3-yl)-pyrrolidin-1-yl]-3-(2-chlor-phenyl)-propenon
• 1-[3-(1-Methyl-1H-indol-3-yl)-pyrrolidin-1-yl]-2-phenyl-butan-1-on
• (2-Chlor-6-fluor-3-methyl-phenyl)-[3-(1-methyl-1H-indol-3-yl)-pyrrolidin-1-yl]-methanon
• 2-{3-[1-(3-Methoxy-benzoyl)-pyrrolidin-3-yl]-indol-1-yl}-N,N-dimethyl-acetamid
• 2-{3-[1-(2-Fluor-benzoyl)-pyrrolidin-3-yl]-indol-1-yl}-N,N-dimethyl-acetamid
• (3-Difluormethylsulfanyl-phenyl)-[3-(1-naphthalen-2-ylmethyl-1H-indol-3-yl)-pyrrolidin-1-yl]-methanon
• Benzo[1,2,5]oxadiazol-5-yl-{3-[1-(3-fluor-benzyl)-1H-indol-3-yl]-pyrrolidin-1-yl}-methanon
• o-Tolyl-{3-[1-(4-trifluormethyl-benzyl)-1H-indol-3-yl]-pyrrolidin-1-yl}-methanon
• N,N-Dimethyl-2-{3-[1-(2-trifluormethyl-benzensulfonyl)-pyrrolidin-3-yl]-indol-1-yl}-acetamid
• 1-[3-(1-Butyl-1H-indol-3-yl)-pyrrolidin-1-yl]-2-phenoxy-propan-1-on
• (6-Chlor-2-fluor-3-methyl-phenyl)-[3-(1-naphthalen-2-ylmethyl-1H-indol-3-yl)-pyrrol idin-1-yl]-methanon
• [3-(1-Allyl-1H-indol-3-yl)-pyrrolidin-1-yl]-(2-methyl-6-trifluormethyl-pyrldin-3-yl)-methanon
• [3-(1-Butyl-1H-indol-3-yl)-pyrrolidin-1-yl]-(4-propyl-phenyl)-methanon
• 2-{3-[1-(4-Chlor-benzoyl)-pyrrolidin-3-yl]-indol-1-yl}-N,N-dimethyl-acetamid
• 1-{3-[1-(3-Fluor-benzyl)-1H-indol-3-yl]-pyrrolidin-1-yl}-3-phenyl-propan-1-on
• {3-[1-(2-Fluor-benzyl)-1H-indol-3-yl]-pyrrolidin-1-yl}-(1-phenyl-5-propyl-1H-pyrazol-4-yl)-methanon
• 3-{3-[1-(5-tert-Butyl-2-methyl-furan-3-carbonyl)-pyrrolidin-3-yl]-indol-1-ylmethyl}-benzonitril
• 3-[1-(2,4-Difluor-benzensulfonyl)-pyrrolidin-3-yl]-1-(2-fluor-benzyl)-1H-indol
• 2-{3-[1-(2,6-Difluor-benzoyl)-pyrrolidin-3-yl]-indol-1-yl}-N,N-dimethyl-acetamid
• 1-[3-(11-Butyl-1H-indol-3-yl)-pyrrolidin-1-yl]-butan-1-on
• 4-{3-[1-(2-Propyl-pentanoyl)-pyrrolidin-3-yl]-indol-1-ylmethyl}-benzonitril
• 2-(4-Chlor-phenoxy)-1-[3-(1-prop-2-ynyl-1H-indol-3-yl)-pyrrolidin-1-yl]-ethanon
• [3-(1-Methyl-1H-indol-3-yl)-pyrrolidin-1-yl]-pyridin-3-yl-methanon
• 3-{3-[1-(Benzo[1,3]dioxole-5-carbonyl)-pyrrolidin-3-yl]-indol-1-ylmethyl}-benzonitril
• [3-(1-Butyl-1H-indol-3-yl)-pyrrolidin-1-yl]-(2-chlor-5-trifluormethyl-phenyl)-methanon
• (3-Chlor-phenyl)-{3-[1-(4-trifluormethyl-benzyl)-1H-indol-3-yl]-pyrrolidin-1-yl}-methanon
• 2-{3-[1-(4-Brom-benzoyl)-pyrrolidin-3-yl]-indol-1-yl}-N,N-dimethyl-acetamid
• 3-Phenyl-1-[3-(1-prop-2-ynyl-1H-indol-3-yl)-pyrrolidin-1-yl]-propenon
• 3-{3-[1-(3-Chlor-2-fluor-benzoyl)-pyrrolidin-3-yl]-indol-1-ylmethyl}-benzonitril
• 3-{3-[1-(2-Phenyl-cyclopropanecarbonyl)-pyrrolidin-3-yl]-indol-1-ylmethyl}-benzonitril
• 3-{3-[1-(3-Chlor-thiophen-2-carbonyl)-pyrrolidin-3-yl]-indol-1-ylmethyl}-benzonitril
• 1-Prop-2-ynyl-3-[1-(2-trifluormethyl-benzensulfonyl)-pyrrolidin-3-yl]-1H-indol
• 1-(2-Fluor-benzyl)-3-[1-(3-trifluormethyl-benzensulfonyl)-pyrrolidin-3-yl]-1H-indol
• [3-(1-Benzyl-1H-indol-3-yl)-pyrrolidin-1-yl]-(6-chlor-2H-chromen-3-yl)-methanon
• [3-(1-Benzyl-1H-indol-3-yl)-pyrrolidin-1-yl]-(3,4-difluor-phenyl)-methanon
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

10. Arzneimittel enthaltend wenigstens ein substituiertes 3-Pyrrolidin-Indol-Derivat der allgemeinen Formel I, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weiterer Wirkstoffe.

11. Verwendung eines substituierten 3-Pyrrolidin-Indol-Derivates der allgemeinen Formel I, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate; zur Herstellung eines Arzneimittels zur Behandlung von Schmerz, insbesondere von akutem, neuropathischem oder chronischem Schmerz.

12. Verwendung eines substituierten 3-Pyrrolidin-Indol-Derivates der allgemeinen Formel I, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate; zur Herstellung eines Arzneimittels zur Behandlung von Depressionen und/oder zur Anxiolyse.

13. Verwendung eines substituierten 3-Pyrrolidin-Indol-Derivates der allgemeinen Formel I, gegebenenfalls in Form seines Razemats, der reinen Stereoisomeren, Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate; zur Herstellung eines Arzneimittels zur Behandlung von cardiovaskulären Erkrankungen, Haminkontinenz, Diarrhöe, Pruritus, Alkohol- und Drogenmißbrauch, Medikamentenabhängigkeit, Entzündungen, Antriebslosigkeit, Bulimie, Anorexie, Katalepsie, zur Verwendung als Lokalanästhetikum, Antiarrythmikum, Antiemetikum, Nootropikum, zur Vigilanz- und Libidosteigerung.

14. Verfahren zur Herstellung von substituierten 3-Pyrrolidin-Indol-Derivaten der allgemeinen Formel I gemäß einem oder mehreren der Ansprüche 1-9, ausgehend von 3-Pyrrolidin-3-yl-indol, **dadurch gekennzeichnet, dass**
a) der Pyrrolidin-Stickstoff von 3-Pyrrolidin-3-yl-indol mit einer Schutzgruppe versehen,
b) der Indol-Stickstoff alkyliert,
c) die Schutzgruppe wieder entfernt und
d) der Pyrrolidin-Stickstoff mit einem 2-Chloracetamid oder einem 2-Bromacetamid, einem Säurechlorid, einem Isocyanat oder einem Isothiocyanat umgesetzt wird.

## Claims

1. Substituted 3-pyrrolidine-indole derivatives of general formula I 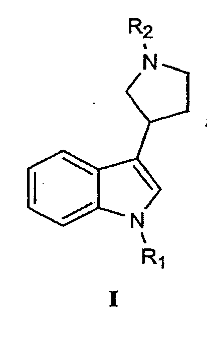 wherein
the radical R¹ represents H; respectively saturated or unsaturated, branched or unbranched, unsubstituted or singly or multiply substituted C₁₋₈-alkyl; or respectively singly or multiply substituted or unsubstituted aryl bound via C₁₋₃-alkyl, C₃₋₈-cycloalkyl or heteroaryl; CH₂CONR³R⁴;
the radical R² represents COR⁵; SO₂R⁶; CSNHR⁷; CONHR⁸,
the radicals R³ and R⁴ independently of one another represent respectively saturated or unsaturated, branched or unbranched, unsubstituted or singly or multiply substituted C₁₋₈-alkyl;
or
the radicals R³ and R⁴ together represent CH₂CH₂OCH₂CH₂, or (CH₂)₃₋₆;
the radical R⁵ represents branched or unbranched, saturated or unsaturated, substituted or unsubstituted C₁₋₈-alkyl; substituted or unsubstituted aryl; substituted or unsubstituted heteroaryl; respectively saturated or unsaturated, substituted or unsubstituted cycloalkyl or heterocyclyl;
or 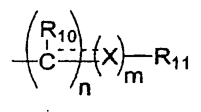 where n = 1, 2, 3; m = 0, 1; X = O or NH and 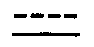 has the meaning of a single bond or a double bond
the radical R⁶ represents respectively substituted or unsubstituted aryl, respectively substituted or unsubstituted heteroaryl, respectively saturated or unsaturated, branched or unbranched, unsubstituted or singly or multiply substituted C₁₋₈-alkyl; substituted or unsubstituted, saturated or unsaturated C₃₋₈-cycloalkyl; or respectively singly or multiply substituted or unsubstituted aryl bound via C₁₋₃-alkyl, C₃₋₈-cycloalkyl or heteroaryl,
the radical R⁷ represents respectively substituted or unsubstituted aryl, respectively substituted or unsubstituted heteroaryl, respectively saturated or unsaturated, branched or unbranched, unsubstituted or singly or multiply substituted C₁₋₈-alkyl; substituted or unsubstituted, saturated or unsaturated C₃₋₈ cycloalkyl; or respectively singly or multiply substituted or unsubstituted aryl bound via C₁₋₃-alkyl, C₃₋₈-cycloalkyl or heteroaryl,
the radical R⁸ represents branched or unbranched, substituted or unsubstituted, saturated or unsaturated C₁₋₈-alkyl; substituted or unsubstituted, saturated or unsaturated C₃₋₈-cycloalkyl; respectively singly or multiply substituted or unsubstituted trifluoromethyl- or nitrosubstituted phenyl, pyrrolyl, indolyl, furyl, benzofuranyl, thienyl, benzothienyl, pyrazolyl, imidazolyl, thiazolyl, oxazolyl, isoxazoyl, pyridazinyl, pyrimidinyl, pyrazinyl, pyranyl, indazolyl, purinyl, indolizinyl, quinolinyl, isoquinolinyl, quinazolinyl, carbazolyl, phenazinyl, oxadiazolyl, chromenyl or phenothiazinyl; or respectively singly or multiply substituted or unsubstituted aryl bound via C₁₋₃-alkyl, C₃₋₈-cycloalkyl or heteroaryl;
R¹⁰ represents H; branched or unbranched, substituted or unsubstituted, saturated or unsaturated C₁₋₈-alkyl; respectively substituted or unsubstituted aryl or heteroaryl, respectively substituted or unsubstituted benzyl or phenethyl,
R¹¹ represents H; branched or unbranched, substituted or unsubstituted, saturated or unsaturated C₁₋₈-alkyl; respectively substituted or unsubstituted aryl or heteroaryl, or respectively singly or multiply substituted or unsubstituted aryl bound via C₁₋₃-alkyl, C₃₋₈-cycloalkyl or heteroaryl,
wherein alkyl, alkanyl, alkenyl and alkinyl may be substituted by F, Cl, Br, I, -CN, -N≡C, NH₂, NH-alkyl, NH-aryl, NH-heteroaryl, NH-alkyl-aryl, NH-alkyl-heteroaryl, NH-heterocyclyl, NH-alkyl-OH, N(alkyl)₂, N(alkyl-aryl)₂, N(alkyl-heteroaryl)₂, N(heterocyclyl)₂, N(alkyl-OH)₂, NO, NO₂, SH, S-alkyl, S-aryl, S-heteroaryl, S-alkyl-aryl, S-alkyl-heteroaryl, S-heterocyclyl, S-alkyl-OH, S-alkyl-SH, OH, O-alkyl, O-aryl, O-heteroaryl, O-alkyl-aryl, O-alkyl-heteroaryl, O-heterocyclyl, O-alkyl-OH, CHO, C(=O)C₁₋₆-alkyl, C(=S)C₁₋₆-alkyl, C(=O)aryl, C(=S)aryl, C(=O)C₁₋₆-alkyl-aryl, C(=S)C₁₋₆-alkyl-aryl, C(=O)-heteroaryl, C(=S)-heteroaryl, C(=O)-heterocyclyl, C(=S)-heterocyclyl, CO₂H, CO₂-alkyl, CO₂-alkyl-aryl, C(=O)NH₂, C(=O)NH-alkyl, C(=O)NH-aryl, C(=O)NH-heterocyclyl, C(=O)N(alkyl)₂, C(=O)N(alkyl-aryl)₂, C(=O)N(alkyl-heteroaryl)₂, C(=O)N(heterocyclyl)₂, SO-alkyl, SO₂-alkyl, SO₂NH₂, SO₃H, PO(O-C₁₋₆-alkyl)₂, Si(C₁₋₆-alkyl)₃, Si(C₃₋₈-cycloalkyl)₃, Si(CH₂-C₃₋₈-cycloalkyl)₃, Si(phenyl)₃, cycloalkyl, aryl, heteroaryl or heterocyclyl,
and aryl, heterocyclyl, heteroaryl and cycloalkyl may be substituted by F, Cl, Br, I, CN, NH₂, NH-alkyl, NH-aryl, NH-heteroaryl, NH-alkyl-aryl, NH-alkyl-heteroaryl, NH-heterocyclyl, NH-alkyl-OH, N(alkyl)₂, N(alkyl-aryl)₂, N(alkyl-heteroaryl)₂, N(heterocyclyl)₂, N(alkyl-OH)₂, NO, NO₂, SH, S-alkyl, S-cycloalkyl, S-aryl, S-heteroaryl, S-alkyl-aryl, S-alkyl-heteroaryl, S-heterocyclyl, S-alkyl-OH, S-alkyl-SH, OH, O-alkyl, O-cycloalkyl, O-aryl, O-heteroaryl, O-alkyl-aryl, O-alkyl-heteroaryl, O-heterocyclyl, O-alkyl-OH, CHO, C(=O)C₁₋₆-alkyl, C(=S)C₁₋₆-alkyl, C(=O)aryl, C(=S)aryl, C(=O)-C₁₋₆-alkyl-aryl, C(=S)C₁₋₆-alkyl-aryl, C(=O)-heteroaryl, C(=S)-heteroaryl, C(=O)-heterocyclyl, C(=S)-heterocyclyl, CO₂H, CO₂-alkyl, CO₂-alkyl-aryl, C(=O)NH₂, C(=O)NH-alkyl, C(=O)NH-aryl, C(=O)NH-heterocyclyl, C(=O)N(alkyl)₂, C(=O)N(alkyl-aryl)₂, C(=O)N(alkyl-heteroaryl)₂, C(=O)N(heterocyclyl)₂, S(O)-alkyl, S(O)-aryl, SO₂-alkyl, SO₂-aryl, SO₂NH₂, SO₃H, CF₃, =O, =S; alkyl, cycloalkyl, aryl, heteroaryl and/or heterocyclyl;
provided that when R² represents cyclohexyl, the cyclohexyl ring cannot be substituted in the 4-position by NR^{x}R^{y} and R^{z},
wherein R^{x} and R^{y} independently of one another represent H; respectively saturated or unsaturated, branched or unbranched, singly or multiply substituted C₁₋₈-alkyl or C₃₋₈-cycloalkyl; respectively singly or multiply substituted or unsubstituted aryl or heteroaryl; or respectively singly or multiply substituted or unsubstituted aryl bound via C₁₋₃-alkyl, C₃₋₈-cycloalkyl or heteroaryl,
or the radicals R^{x} and R^{y} together form a ring and represent CH₂CH₂OCH₂CH₂, CH_{z}CH₂NRⁿCH₂CH₂ or (CH₂)₃₋₆,
wherein Rⁿ represents H; respectively saturated or unsaturated, branched or unbranched, singly or multiply substituted or unsubstituted C₁₋₈-alkyl or C₃₋₈ cycloalkyl; respectively singly or multiply substituted or unsubstituted aryl or heteroaryl, or respectively singly or multiply substituted or unsubstituted aryl bound via C₁₋₃-alkyl, C₃₋₈-cycloalkyl or heteroaryl;
and
R^{z} represents respectively saturated or unsaturated, branched or unbranched, singly or multiply substituted or unsubstituted C₁₋₈-alkyl or C₃₋₈ cycloalkyl; respectively unsubstituted or singly or multiply substituted aryl or heteroaryl, respectively unsubstituted or singly or multiply substituted aryl bound via a saturated or unsaturated, branched or unbranched, substituted or unsubstituted C₁₋₄-alkyl group, C₃₋₈-cycloalkyl or heteroaryl;
in the form of the racemate; the enantiomers, diastereomers, blends of the enantiomers or diastereomers or of an individual enantiomer or diastereomer; the bases and/or salts of physiologically acceptable acids or cations.

2. Substituted 3-pyrrolidine-indole derivatives according to claim 1, **characterised in that** the radical R² represents COR⁵, SO₂R⁶ or CSNHR⁷.

3. Substituted 3-pyrrolidine-indole derivatives according to claim 1, **characterised in that** the radical R² represents CONHR⁸.

4. Substituted 3-pyrrolidine-indole derivatives according to claims 1 to 3, **characterised in that** the radical R¹ represents H; respectively saturated or unsaturated, branched or unbranched, unsubstituted or singly or multiply substituted C₁₋₈-alkyl; respectively singly or multiply substituted or unsubstituted benzyl or methylnaphthyl, in particular benzyl which is unsubstituted or singly or multiply substituted by CF₃, Br, F, CN, OCH₃ or CH₃; CH₂CONR³R⁴.

5. Substituted 3-pyrrolidine-indole derivatives according to claims 1, 2 or 4, **characterised in that** the radical R² represents COR⁵ and R⁵ represents 4-propylphenyl, 3,4-dimethoxyphenyl, 2-methyl-4-trifluoromethylphenyl-3-pyridine, ethenylphenyl, 2,3-difluorophenyl, 4-tert.-butylphenyl, 2-ethoxyphenyl, 3-fluoro-4-trifluoromethylphenyl, 2,3-dimethylphenyl, phenoxyethyl, phenoxymethyl, 3,4-dichlorophenyl, 4-trifluoromethylsulphanylphenyl, 2,5-dimethoxyphenyl, 2-chloro-4-nitrophenyl, 2-chlorophenyl, 4-methyl-N-phenethyl-benzylsulphonamide, 2-chloro-5-fluoro-3-methylphenyl, 3-(2-chlorophenyl)-5-methyl-isoxazole, 5-tert.-butyl-2-methylfuran,-benzo[1,2,5]oxadiazole, phenylpropyl, 2-methylsulphanyl-3-pyridine, 2-chloro-5 trifluoromethylphenyl, methoxymethyl, 4-methylphenyl, biphenyl, 4-chlorobenzyl, 2,3,4,5,6-pentafluorophenyl, 3-chlorophenyl, 4-trifluoromethylphenyl, 2,6-difluoro-3-methylphenyl, 2-methylphenyl, 4-fluorophenyl, 6-chlorochromene, 2-chloro-6-fluorophenyl, ethyl cyclopentyl, 1-(4-chlorophenyl)-5-trifluoromethylpyrazole, 2,4-dichlorophenyl, 2,3-dimethylphenyl, 3-nitro-4-methylphenyl, 4-bromo-3-methylphenyl, (4-chlorophenoxy)methyl, 4-chlorophenyl, 5-methylisoxazole, 3-methoxyphenyl, 2-chlorophenylethenyl, 2-chloro-4-fluoro-3-methylphenyl, 2-fluorophenyl, 3-difluoromethylsulphanylphenyl, 2-fluoro-3-chlorophenyl, cyclopropylphenyl, 1-phenyl-5-propylpyrazole, 2,6-difluorophenyl, benzo[1,3]-dioxole, 4-bromophenyl, 3-chlorothiophenyl, 2-,3-or 4-pyridine, phenyl or 3,4-difluorophenyl.

6. Substituted 3-pyrrolidine-indole derivatives according to claims 1, 2 or 4, **characterised in that** the radical R² represents SO₂R⁶ and R⁶ represents 2,4,6-dimethylphenyl, 4-nitrophenyl, benzyl, 4-propylphenyl, 4-chlorophenyl, 2-trifluoromethylphenyl, 2,4-difluorophenyl or 3-trifluoromethylphenyl.

7. Substituted 3-pyrrolidine-indole derivatives according to claims 1, 2 or 4, **characterised in that** the radical R² represents CSNHR⁷ and the radical R⁷ represents 3-trifluoromethylphenyl.

8. Substituted 3-pyrrolidine-indole derivatives according to claims 1, 3 or 4, **characterised in that** the radical R² represents CONHR⁸ and the radical R⁸ represents 3,4-dichlorobenzyl.

9. Substituted 3-pyrrolidine-indole derivatives according to claims 1 to 8, selected from the group comprising
• (4-propyl-phenyl)-{3-[1-(4-trifluoromethyl-benzyl)-1H-indol-3-yl]-pyrrolidin-1-yl}-methanone
• (3,4-dimethoxy-phenyl)-{3-[1-(3-methyl-butyl)-1H-indol-3-yl]-pyrrolidin-1-yl)}-methanone
• [3-(1-butyl-1H-indol-3-yl)-pyrrolidin-1-yl]-(2-methyl-6-trifluoromethyl-pyridin-3-yl)-methanone
• 1-{3-[1-(3-methoxy-benzyl)-1H-indol-3-yl]-pyrrolidin-1-yl}-3-phenyl-propenone
• (2,3-difluoro-phenyl)-{3-[1-(4-trifluoromethyl-benzyl)-1H-indol-3-yl]-pyrrolidin-1-yl}-methanone
• (4-tert-butyl-phenyl)-{3-[1-(3-methyl-butyl)-1H-indol-3-yl]-pyrrolidin-1-yl}-methanone
• {3-[1-(4-bromo-benzyl)-1H-indol-3-yl]-pyrrolidin-1-yl}-(4-propyl-phenyl)-methanone
• (2-ethoxy-phenyl)-{3-[1-(3-methoxy-benzyl)-1H-indol-3-yl]-pyrrolidin-1-yl}-methanone
• [3-(1-ethyl-1H-indol-3-yl)-pyrrolidin-1-yl]-(3-fluoro-4-trifluoromethylphenyl)-methanone
• (2,3-dimethyl-phenyl)-[3-(1-ethyl-1H-indol-3-yl)-pyrrolidin-1-yl]-methanone
• 1-{3-[1-(3-methoxy-benzyl)-1H-indol-3-yl]-pyrrolidin-1-yl}-2-phenoxy-propan-1-one
• 2-(3-chloro-phenoxy)-1-[3-(1-ethyl-1H-indol-3-yl)-pyrrolidin-1-yl]-ethanone
• 4-{3-[1-(2-phenoxy-propionyl)-pyrrolidin-3-yl]-indol-1-ylmethyl}-benzonitrile
• N,N-dimethyl-2-{3-[1-(2,4,6-trimethyl-benzenesulphonyl)-pyrrolidin-3-yl]-indol-1-yl}-acetamide
• (3,4-dichloro-phenyl)-[3-(1-ethyl-1H-indol-3-yl)-pyrrolidin-1-yl]-methanone
• N,N-dimethyl-2-{3-[1-(3-trifluoromethyl-phenylthiocarbamoyl)-pyrrolidin-3-yl]-indol-1-yl}-acetamide
• 3-(1-dimethylcarbamoylmethyl-1H-indol-3-yl)-pyrrolidin-1-carboxylic acid 3,4-dichloro-benzylamide
• [3-(1H-indol-3-yl)-pyrrolidin-1-yl]-(4-trifluoromethylsulphanyl-phenyl)-methanone
• 2-(3-{1-[2-(2,5-dimethoxy-phenyl)-acetyl]-pyrrolidin-3-yl}-indol-1-yl)-N,N-dimethyl-acetamide
• (2-chloro-4-nitro-phenyl)-{3-[1-(3-fluoro-benzyl)-1H-indol-3-yl]-pyrrolidin-1-yl}-methanone
• N,N-dimethyl-2-{3-[1-(pyridin-2-carbonyl)-pyrrolidin-3-yl]-indol-1-yl}-acetamide
• cyclobutyl-[3-(1H-indol-3-yl)-pyrrolidin-1-yl]-methanone
• N,N-diethyl-2-[3-(1-pentanoyl-pyrrolidin-3-yl)-indol-1-yl]-acetamide
• 3-[1-(4-nitrobenzenesulphonyl)-pyrrolidin-3-yl]-1H-indole
• 2-[3-(1-cyclopentanecarbonyl-pyrrolidin-3-yl)-indol-1-yl]-N,N-diethylacetamide
• 1-[3-(1-methyl-1H-indol-3-yl)-pyrrolidin-1-yl]-3-phenyl-propenone
• (2-chloro-phenyl)-[3-(1H-indol-3-yl)-pyrrolidin-1-yl]-methanone
• [3-(1-ethyl-1H-indol-3-yl)-pyrrolidin-1-yl]-pyridin-3-yl-methanone
• N,N-dimethyl-2-(3-{1-[3-phenyl-2-(toluene-4-sulphonylamino)-propionyl]-pyrrolidin-3-yl}-indol-1-yl)-acetamide
• N,N-dimethyl-2-[3-(1-phenylmethanesulphonyl-pyrrolidin-3-yl)-indol-1-yl]-acetamide
• 4-{3-[1-(2-chloro-6-fluoro-3-methyl-benzoyl)-pyrrolidin-3-yl]-indol-1-ylmethyl}-benzonitrile
• [3-(2-chloro-phenyl)-5-methyl-isoxazol-4-yl]-[3-(1-ethyl-1H-indol-3-yl)-pyrrolidin-lyl]-methanone
• 2-{3-[1-(5-tert-butyl-2-methyl-furan-3-carbonyl)-pyrrolidin-3-yl]-indol-1-ylmethyl}-benzonitrile
• 4-{3-[1-(benzo[1,2,5]oxadiazole-5-carbonyl)-pyrrolidin-3-yl]-indol-1-ylmethyl}-benzonitrile
• 1-[3-(1-ethyl-1H-indol-3-yl)-pyrrolidin-1-yl]-3-phenyl-propan-1-one
• 1-{3-[1-(3-methoxy-benzyl)-1H-indol-3-yl]-pyrrolidin-1-yl}-3-phenyl-propan-1-one
• [3-(1-methyl-1H-indol-3-yl)-pyrrolidin-1-yl]-(2-methylsulphanyl-pyridin-3-yl)-methanone
• (2-chloro-5-trifluoromethyl-phenyl)-[3-(1-ethyl-1H-indol-3-yl)-pyrrolidin-1-yl]-methanone
• (2-chloro-4-nitro-phenyl)-{3-[1-(4-trifluoromethyl-benzyl)-1H-indol-3-yl]-pyrrolidin-lyl}-methanone
• (2-chloro-pyridin-3-yl)-[3-(1-methyl-1H-indol-3-yl)-pyrrolidin-1-yl]-methanone
• 2-methoxy-1-{3-[1-(3-methoxy-benzyl)-1H-indol-3-yl]-pyrrolidin-1-yl}-ethanone
• {3-[1-(3-fluoro-benzyl)-1H-indol-3-yl]-pyrrolidin-1-yl}-(2-methyl-6-trifluoromethylpyridin-3-yl)-methanone
• {3-[1-(3-methoxy-benzyl)-1H-indol-3-yl]-pyrrolidin-1-yl}-p-tolyl-methanone
• (2-methylsulphanyl-pyridin-3-yl)-[3-(1-prop-2-ynyl-1H-indol-3-yl)-pyrrolidin-1-yl]-methanone
• biphenyl-4-yl-[3-(1-methyl-1H-indol-3-yl)-pyrrolidin-1-yl]-methanone
• 2-(4-chloro-phenyl)-1-{3-[1-(3-methoxy-benzyl)-1H-indol-3-yl]-pyrrolidin-1-yl}-ethanone
• [3-(1-methyl-1H-indol-3-yl)-pyrrolidin-1-yl]-pentafluorophenyl-methanone
• [3-(1-ethyl-1H-indol-3-yl)-pyrrolidin-1-yl]-(4-trifluoromethyl-phenyl)-methanone
• 2-[3-(1-but-2-ynyl-1H-indol-3-yl)-pyrrolidin-1-yl]-N-(2,5-dimethyl-2H-pyrazol-3-yl)-acetamide
• N-(3-cyano-4-methyl-thiophen-2-yl)-2-{3-[1-(3,5-dimethyl-benzyl)-1H-indol-3-yl] pyrrolidin-1-yl}acetamide
• N,N-diethyl-2-(3-{1-[(4-trifluoromethyl-phenyl carbamoyl)-methyl]-pyrrolidin-3-yl}-indol-1-yl)-acetamide
• N-(3-cyano-4-methyl-thiophen-2-yl)-2-[3-(1-ethyl-1H-indol-3-yl)-pyrrolidin-1-yl]-acetamide
• 2-{3-[1-(3,4-difluoro-benzyl)-1H-indol-3-yl]-pyrrolidin-1-yl}-N-(4-phenyl-5-trifluoromethyl-thiophen-3-yl)-acetamide
• N,N-dimethyl-2-{3-[1-(4-propyl-benzenesulphonyl)-pyrrolidin-3-yl]-indol-1-yl}-acetamide
• (2-chloro-4-nitro-phenyl)-{3-[1-(3-methoxy-benzyl)-1H-indol-3-yl]-pyrrolidin-1-yl}-methanone
• 3-{3-[1-(4-chloro-benzenesulphonyl)-pyrrolidin-3-yl]-indol-1-ylmethyl}-benzonitrile
• 1-{3-[1-(3-methoxy-benzyl)-1H-indol-3-yl]-pyrrolidin-1-yl}-3,3-dimethyl-butan-1-one
• 2-(4-chloro-phenyl)-1-[3-(1-methyl-1H-indol-3-yl)-pyrrolidin-1-yl]-ethanone
• (2,6-difluoro-3-methyl-phenyl)-{3-[1-(3-methoxy-benzyl)-1H-indol-3-yl]-pyrrolidin-1-yl}-methanone
• [3-(1-ethyl-1H-indol-3-yl)-pyrrolidin-1-yl]-o-tolyl-methanone
• [3-(1-ethyl-1H-indol-3-yl)-pyrrolidin-1-yl]-(4-fluoro-phenyl)-methanone
• [3-(1-butyl-1H-indol-3-yl)-pyrrolidin-1-yl]-(6-chloro-2H-chromen-3-yl)-methanone
• (2-chloro-6-fluoro-phenyl)-[3-(1-naphthalen-2-ylmethyl-1H-indol-3-yl)-pyrrolidin-1-yl]-methanone
• 2-{3-[1-(3-cyclopentyl-propionyl)-pyrrolidin-3-yl]-indol-1-yl}-N,N-dimethylacetamide
• [3-(2-chloro-phenyl)-5-methyl-isoxazol-4-yl]-{3-[1-(3-methoxy-benzyl)-1H-indol-3-yl]-pyrrolidin-1-yl}-methanone
• [1-(4-chloro-phenyl)-5-trifluoromethyl-1H-pyrazol-4-yl]-{3-[1-(3-fluorobenzyl)-1H-indol-3-yl]-pyrrolidin-1-yl}-methanone
• [3-(1-allyl-1H-indol-3-yl)-pyrrolidin-1-yl]-p-tolyl-methanone
• {3-[1-(4-fluoro-benzyl)-1H-indol-3-yl]-pyrrolidin-1-yl}-pyridin-2-yl-methanone
• (3,4-dichloro-phenyl)-{3-[1-(4-trifluoromethyl-benzyl)-1H-indol-3-yl]-pyrrolidin-1-yl}-methanone
• 1-butyl-3-[1-(2,5-dimethoxy-benzenesulphonyl)-pyrrolidin-3-yl]-1H-indole
• 1-[3-(1-allyl-1H-indol-3-yl)-pyrrolidin-1-yl]-3,3-dimethyl-butan-1-one
• 4-{3-[1-(2-methoxy-acetyl)-pyrrolidin-3-yl]-indol-1-ylmethyl}-benzonitrile
• (2,4-difluoro-phenyl)-[3-(1-ethyl-1H-indol-3-yl)-pyrrolidin-1-yl]-methanone
• (2,3-dimethyl-phenyl)-{3-[1-(4-trifluoromethyl-benzyl)-1H-indol-3-yl]-pyrrolidin-1-yl}-methanone
• [3-(1-butyl-1H-indol-3-yl)-pyrrolidin-1-yl]-(4-methyl-3-nitro-phenyl)-methanone
• (4-bromo-3-methyl-phenyl)-{3-[1-(3-methoxy-benzyl)-1H-indol-3-yl]-pyrrolidin-1-yl}-methanone
• 1-[3-(1-butyl-1H-indol-3-yl)-pyrrolidin-1-yl]-2-(4-chloro-phenoxy)-ethanone
• 2-(4-chloro-phenyl)-1-{3-[1-(3-fluoro-benzyl)-1H-indol-3-yl]-pyrrolidin-1-yl}-ethanone
• (4-chloro-phenyl)-{3-[1-(4-trifluoromethyl-benzyl)-1H-indol-3-yl]-pyrrolidin-1-yl}-methanone
• [3-(1-benzyl-1H-indol-3-yl)-pyrrolidin-1-yl]-(5-methyl-isoxazol-3-yl)-methanone
• 1-[3-(1-benzyl-1H-indol-3-yl)-pyrrolidin-1-yl]-2-(4-chloro-phenoxy)-ethanone
• [3-(1-naphthalen-2-ylmethyl-1H-indol-3-yl)-pyrrolidin-1-yl]-(4-trifluoromethylphenyl)-methanone
• (3-methoxy-phenyl)-(3-[1-(4-trifluoromethyl-benzyl)-1H-indol-3-yl]-pyrrolidin-1-yl}-methanone
• 1-[3-(1-butyl-1H-indol-3-yl)-pyrrolidin-1-yl]-3-(2-chloro-phenyl)-propenone
• 1-[3-(1-methyl-1H-indol-3-yl)-pyrrolidin-1-yl]-2-phenyl-butane-1-one
• (2-chloro-6-fluoro-3-methyl-phenyl)-[3-(1-methyl-1H-indol-3-yl)-pyrrolidin-1-yl]-methanone
• 2-{3-[1-(3-methoxy-benzoyl)-pyrrolidin-3-yl]-indol-1-yl}-N,N-dimethylacetamide
• 2-{3-[1-(2-fluoro-benzoyl)-pyrrolidin-3-yl]-indol-1-yl}-N,N-dimethylacetamide
• (3-difluoromethylsulphanyl-phenyl)-[3-(1-naphthalen-2-ylmethyl-1H-indol-3-yl)pyrrolidin-1-yl]-methanone
• benzo[1,2,5]oxadiazol-5-yl-{3-[1-(3-fluoro-benzyl)-1H-indol-3-yl]-pyrrolidin-1-yl}-methanone
• o-tolyl-{3-[1-(4-trifluoromethyl-benzyl)-1H-indol-3-yl]-pyrrolidin-1-yl}-methanone
• N,N-dimethyl-2-{3-[1-(2-trifluoromethyl-benzenesulphonyl)-pyrrolidin-3-yl]-indol-1-yl}-acetamide
• 1-[3-(1-butyl-1H-indol-3-yl)-pyrrolidin-1-yl]-2-phenoxy-propan-1-one
• (6-chloro-2-fluoro-3-methyl-phenyl)-[3-(1-naphthalen-2-ylmethyl-1H-indol-3-yl)-pyrrolidin-1-yl]-methanone
• [3-(1-allyl-1H-indol-3-yl)-pyrrolidin-1-yl]-(2-methyl-6-trifluoromethyl-pyridin-3-yl)-methanone
• [3-(1-butyl-1H-indol-3-yl)-pyrrolidin-1-yl]-(4-propyl-phenyl)-methanone
• 2-{3-[1-(4-chloro-benzoyl)-pyrrolidin-3-yl]-indol-1-yl)-N,N-dimethylacetamide
• 1-{3-[1-(3-fluoro-benzyl)-1H-indol-3-yl]-pyrrolidin-1-yl}-3-phenyl-propan-1-one
• {3-[1-(2-fluoro-benzyl)-1H-indol-3-yl]-pyrrolidin-1-yl}-(1-phenyl-5-propyl-1H-pyrazol-4-yl)-methanone
• 3-{3-[1-(5-tert-butyl-2-methyl-furan-3-carbonyl)-pyrrolidin-3-yl]-indol-1-ylmethyl}-benzonitrile
• 3-[1-(2,4-difluoro-benzenesulphonyl)-pyrrolidin-3-yl]-1-(2-fluoro-benzyl)-1H-indole
• 2-{3-[1-(2,6-difluoro-benzoyl)-pyrrolidin-3-yl]-indol-1-yl}-N,N-dimethylacetamide
• 1-[3-(1-butyl-1H-indol-3-yl)-pyrrolidin-1-yl]-butane-1-one
• 4-{3-[1-(2-propyl-pentanoyl)-pyrrolidin-3-yl]-indol-1-ylmethyl}-benzonitrile
• 2-(4-chloro-phenoxy)-1-[3-(1-prop-2-ynyl-1H-indol-3-yl)-pyrrolidin-1-yl]-ethanone
• [3-(1-methyl-1H-indol-3-yl)-pyrrolidin-1-yl]-pyridin-3-yl-methanone
• 3-{3-[1-(benzo[1,3]dioxole-5-carbonyl)-pyrrolidin-3-yl]-indol-1-ylmethyl}-benzonitrile
• [3-(1-butyl-1H-indol-3-yl)-pyrrolidin-1-yl]-(2-chloro-5-trifluoromethylphenyl)-methanone
• (3-chloro-phenyl)-{3-[1-(4-trifluoromethyl-benzyl)-1H-indol-3-yl]-pyrrolidin-1-yl}-methanone
• 2-{3-[1-(4-bromo-benzoyl)-pyrrolidin-3-yl]-indol-1-yl}-N,N-dimethylacetamide
• 3-phenyl-1 -[3-(1-prop-2-ynyl-1H-indol-3-yl)-pyrrolidin-1-yl]-propenone
• 3-{3-[1-(3-chloro-2-fluoro-benzoyl)-pyrrolidin-3-yl]-indol-1-ylmethyl}-benzonitrile
• 3-{3-[1-(2-phenyl-cyclopropanecarbonyl)-pyrrolidin-3-yl]-indol-1-ylmethyl}-benzonitrile
• 3-{3-[1-(3-chloro-thiophene-2-carbonyl)-pyrrolidin-3-yl]-indol-1-ylmethyl}-benzonitrile
• 1-prop-2-ynyl-3-[1-(2-trifluoromethyl-benzenesulphonyl)-pyrrolidin-3-yl]-1H-indole
• 1-(2-fluoro-benzyl)-3-[1-(3-trifluoromethyl-benzenesulphonyl)-pyrrolidin-3-yl]-1H-indole
• [3-(1-benzyl-1H-indol-3-yl)-pyrrolidin-1-yl]-(6-chloro-2H-chromen-3-yl)-methanone
• [3-(1-benzyl-1H-indol-3-yl)-pyrrolidin-1-yl]-(3,4-difluoro-phenyl)-methanone
in the form of the racemate; the enantiomers, diastereomers, blends of the enantiomers or diastereomers or of an individual enantiomer or diastereomer; the bases and/or salts of physiologically acceptable acids or cations.

10. Pharmaceutical composition containing at least one substituted 3-pyrrolidine-indole derivative of general formula I, optionally in the form of its racemate, the pure stereoisomers, in particular enantiomers or diastereomers, in any mixing ratio; in the form of its acids or its bases or in the form of its salts, in particular the physiologically acceptable salts or salts of physiologically acceptable acids or cations; or in the form of its solvates, in particular the hydrates, and optionally containing suitable additives and/or auxiliaries and/or optionally further active ingredients.

11. Use of a substituted 3-pyrrolidine-indole derivative of general formula I, optionally in the form of its racemate, the pure stereoisomers, in particular enantiomers or diastereomers, in any mixing ratio; in the form of its acids or its bases or in the form of its salts, in particular the physiologically acceptable salts or salts of physiologically acceptable acids or cations; or in the form of its solvates, in particular the hydrates; for producing a pharmaceutical composition for treating pain, in particular acute, neuropathic or chronic pain.

12. Use of a substituted 3-pyrrolidine-indole derivative of general formula I, optionally in the form of its racemate, the pure stereoisomers, in particular enantiomers or diastereomers, in any mixing ratio; in the form of its acids or its bases or in the form of its salts, in particular the physiologically acceptable salts or salts of physiologically acceptable acids or cations; or in the form of its solvates, in particular the hydrates; for producing a pharmaceutical composition for treating depression and/or for anxiolysis.

13. Use of a substituted 3-pyrrolidine-indole derivative of general formula I, optionally in the form of its racemate, the pure stereoisomers, in particular enantiomers or diastereomers, in any mixing ratio; in the form of its acids or its bases or in the form of its salts, in particular the physiologically acceptable salts or salts of physiologically acceptable acids or cations; or in the form of its solvates, in particular the hydrates; for producing a pharmaceutical composition for treating cardiovascular diseases, urinary incontinence diarrhoea, pruritus, alcohol and drug abuse, medicine dependency, inflammations, lethargy, bulimia, anorexia, catalepsy, for use as a local anaesthetic, an anti-arrhythmic, an anti-emetic, a nootropic, for increasing alertness and libido.

14. Method for producing substituted 3-pyrrolidine-indole derivatives of general formula I according to one or more of claims 1 to 10, starting from 3-pyrrolidin-3-yl-indole, **characterised in that**
a) the pyrrolidine nitrogen of 3-pyrrolidin-3-yl-indole is provided with a protecting group,
b) the indole nitrogen is alkylated
c) the protecting group is removed again and
d) the pyrrolidine nitrogen is reacted with a 2-chloroacetamide or a 2-bromoacetamide, an acid chloride, an isocyanate or an isothiocyanate.

## Revendications

1. Dérivés substitués de 3-pyrrolidine-indole de formule générale I 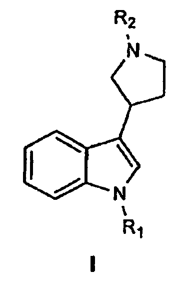 dans laquelle
le reste R¹ représente H ; un reste alkyle en C₁ à C₈, dans chaque cas saturé ou non saturé, ramifié ou non ramifié, non substitué ou substitué une ou plusieurs fois ; ou un reste hétéroaryle, cycloalkyle en C₃ à C₈ ou aryle lié par l'intermédiaire d'un radical alkyle en C₁ à C₃, chacun substitué une ou plusieurs fois ou non substitué ;
un reste CH₂CONR³R⁴ ;
le reste R² représente COR⁵ ; SO2R⁶ ; CSNHR⁷ ; CONHR⁸ ;
les restes R³ et R⁴ représentent indépendamment l'un de l'autre un reste alkyle en C₁ à C₈, dans chaque cas saturé ou non saturé, ramifié ou non ramifié, non substitué ou substitué une ou plusieurs fois ;
ou bien
les restes R³ et R⁴ forment ensemble un groupe CH₂CH₂OCH₂CH₂ ou (CH₂)₃₋₆ ;
le reste R⁵ représente un reste alkyle en C₁ à C₈, ramifié ou non ramifié, saturé ou non saturé, substitué ou non substitué; un reste aryle, substitué ou non substitué ; un reste hétéroaryle, substitué ou non substitué ; un reste cycloalkyle ou hétérocyclyle, chacun saturé ou non saturé, substitué ou non substitué ;
ou bien
un groupe 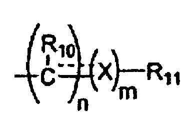 dans lequel n = 1, 2 ou 3 ; m = 0, 1 ; X = O ou NH et 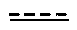 désignent une liaison simple ou une double liaison,
le reste R⁶ désigne un reste aryle, dans chaque cas substitué ou non substitué, un reste hétéroaryle, dans chaque cas substitué ou non substitué, un reste alkyle en C₁ à C₈, dans chaque cas saturé ou non saturé, ramifié ou non ramifié, non substitué ou substitué une ou plusieurs fois ; un reste cycloalkyle en C₃ à C₈, substitué ou non substitué, saturé ou non saturé ; ou un reste hétéroaryle, cycloalkyle en C₃ à C₈ ou aryle lié par l'intermédiaire d'un radical alkyle en C₁ à C₃, chaque reste substitué ou non substitué une ou plusieurs fois,
le reste R⁷ désigne un reste aryle, dans chaque cas substitué ou non substitué, un reste hétéroaryle, dans chaque cas substitué ou substitué, un reste alkyle en C₁ à C₈, dans chaque cas saturé ou non saturé, ramifié ou non ramifié, non substitué ou substitué une ou plusieurs fois ; un reste cycloalkyle en C₃ à C₈, substitué ou non substitué, saturé ou non saturé ; ou un reste hétéroaryle, cycloalkyle en C₃ à C₈ ou aryle lié par l'intermédiaire d'un radical alkyle en C₁ à C₃, chaque reste substitué une ou plus fois ou non substitué,
le reste R⁸ représente un reste alkyle en C₁ à C₈, ramifié ou non ramifié, substitué ou non substitué, saturé ou non saturé ; un reste cycloalkyle en C₃ à C₈, substitué ou non substitué, saturé ou non saturé ; un reste à substituant trifluorométhyle ou nitro, phényle, pyrrolyle, indolyle, furyle, benzofurannyle, thiényle, benzothiényle, pyrazolyle, imidazolyle, thiazolyle, oxazolyle, isoxazolyle, pyridazinyle, pyrimidinyle, pyrazinyle, pyrannyle, indazolyle, purinyle, indolizinyle, quinolinyle, isoquinolinyle, quinazolinyle, carbazolyle, phénazinyle, oxadiazolyle, chroményle ou phénothiazinyle, chacun substitué une ou plusieurs fois ou non substitué ; ou un reste hétéroaryle, cycloalkyle en C₃ à C₈ ou aryle lié par l'intermédiaire d'un radical alkyle en C₁ à C₃, chacun substitué une ou plusieurs fois ou non substitué ;
R¹⁰ représentent H ; un reste alkyle en C₁ à C₈, ramifié ou non ramifié, substitué ou non substitué, saturé ou non saturé ; un reste aryle ou hétéroaryle, chacun substitué ou non substitué, un reste benzyle ou phénétyle, chacun substitué ou non substitué,
R¹¹ représente H ; un reste alkyle en C₁ à C₈, ramifié ou non ramifié, substitué ou non substitué, saturé ou non saturé ; un reste aryle ou hétéroaryle, chacun substitué ou non substitué, ou un reste hétéroaryle, cycloalkyle en C₃ à C₈ ou aryle lié par l'intermédiaire d'un radical alkyle en C₁ à C₃, chaque reste substitué une ou plusieurs fois ou non substitué,
les restes alkyle, alcanyle, alcényle et alcynyle, pouvant être substitués par un radical F, Cl, Br, I, -CN, -N≡C, NH₂, NH-alkyle, NH-aryle, NH-hétéroaryle, NH-alkyl-aryle, NH-alkyl-hétéroaryle, NH-hétérocyclyle, NH-alkyl-OH, N(alkyl)₂, N(alkyl-aryl)₂, N(alkyl-hétéroaryl)₂, N(hétérocyclyl)₂, N(alkyl-OH)₂, NO, NO₂, SH, S-alkyle, S-aryle, S-hétéroaryle, S-alkyl-aryle, S-alkyl-hétéroaryle, S-hétérocyclyle, S-alkyl-OH, S-alkyl-SH, OH, O-alkyle, O-aryle, O-hétéroaryle, O-alkyl-aryle, O-alkyl-hétéroaryle, 0-hétérocyclyle, O-alkyl-OH, CHO, C(=O)alkyle en C₁ à C₆, C(=S)alkyle en C₁ à C₆, C(=O)aryle, C(=S)aryle, C(=O)(alkyle en C₁ à C₆)aryle, C(=S)(alkyle en C₁ à C₆)-aryle, C(=O)hétéroaryle, C(=S)hétéroaryle, C(=O)-hétérocyclyle, C(=S)hétérocyclyle, CO₂H, CO₂-alkyle, CO₂-alkyl-aryle, C(=O)NH₂, C(=O)NH-alkyle, C(=O)NH-aryle, C(=O)NH-hétérocyclyle, C(=O)N(alkyl)₂, C(=O)N(alkyl-aryl)₂, C(=O)N(alkyl-hétéroaryl)₂, C(=O)N(hétérocyclyl)₂, SO-alkyle, SO₂-alkyle, SO₂NH₂, SO₃H, PO(O-alkyle en C₁ à C₆)₂, Si(alkyle en C₁ à C₆)₃, Si(cycloalkyle en C₃ à C₈)₃, Si(CH₂-(cycloalkyle en C₃ à C₈))₃, Si(phényl)₃, cycloalkyle, aryle, hétéroaryle ou hétérocyclyle, et aryle, hétérocyclyle, hétéroaryle ainsi que cycloalkyle peuvent être substitués par un radical F, Cl, Br, I, CN, NH₂, NH-alkyle, NH-aryle, NH-hétéroaryle, NH-alkyl-aryle, NH-alkyl-hétéroaryle, NH-hétérocyclyle, NH-alkyl-OH, N(alkyl)₂, N(alkyl-aryl)₂, N(alkyl-hétéroaryl)₂, N(hétérocyclyl)₂, N(alkyl-OH)₂, NO, NO₂, SH, S-alkyle, S-cycloalkyle, S-aryle, S-hétéroaryle, S-alkyl-aryle, S-alkyl-hétéroaryle, S-hétérocyclyle, S-alkyl-OH, S-alkyl-SH, OH, O-alkyle, O-cycloalkyle, O-aryle, O-hétéroaryle, O-alkyl-aryle, O-alkyl-hétéroaryle, O-hétérocyclyle, O-alkyl-OH, CHO, C(=O)alkyle en C₁ à C₆, C (=S) alkyle en C₁ à C₆, C(=O)aryle, C (=S) aryle, C(=O)(alkyle en C₁ à C₆) aryle, C(=S)(alkyle en C₁ à C₆)aryle, C(=O)hétéroaryle, C(=S)hétéroaryle, C(=O)hétérocyclyle, C(=S)hétérocyclyle, CO₂H, CO₂-alkyle, CO₂-alkyl-aryle, C(=O)NH₂, C(=O)NH-alkyle, C(=O)NH-aryle, C(=O)NH-hétérocyclyle, C(=O)N(alkyl)₂, C(=O)N(alkyl-aryl)₂, C(=O)N(alkyl-hétéroaryl)₂, C(=O)N(hétérocyclyl)2, S(O)-alkyle, S(O)-aryle, SO₂-alkyle, SO₂-aryle, SO₂NH₂, SO₃H, CF₃, =O, =S ; alkyle, cycloalkyle, aryle, hétéroaryle et/ou hétérocyclyle ;
sous réserve que lorsque R² désigne un reste cyclohexyle, le noyau cyclohexyle ne puisse pas être substitué en position 4 avec NR^{x}R^{y} et R^{z},
R^{x} et R^{y} désignant, indépendamment l'un de l'autre, H ; un reste alkyle en C₁ à C₈ ou cycloalkyle en C₃ à C₈, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste hétéroaryle, cycloalkyle en C₃ à C₈ ou aryle lié par l'intermédiaire d'un radical alkyle en C₁ à C₃, chaque reste étant substitué une ou plusieurs fois ou non substitué,
ou bien les restes R^{x} et R^{y} forment ensemble un noyau et représentent CH₂CH₂OCH₂CH₂, CH₂CH₂NRⁿCH₂CH₂ ou (CH₂)₃₋₆,
Rⁿ désignant H ; un reste alkyle en C₁ à C₈ ou cycloalkyle en C₃ à C₈, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste hétéroaryle, cycloalkyle en C₃ à C₈ ou aryle lié par l'intermédiaire d'un radical alkyle en C₁ à C₃, chaque reste étant substitué une ou plusieurs fois ou non substitué ;
et
R^{z} désigne un reste alkyle en C₁ à C₈ ou un reste cycloalkyle en C₃ à C₈, chacun saturé ou non saturé, ramifié ou non ramifié, substitué une ou plusieurs fois ou non substitué ; un reste aryle, hétéroaryle, chacun non substitué ou substitué une ou plusieurs fois ; un reste hétéroaryle, cycloalkyle en C₃ à C₈ ou aryle lié par l'intermédiaire d'un groupe alkyle en C₁ à C₄ saturé ou non saturé, ramifié ou non ramifié, substitué ou non substitué, chaque reste étant non substitué ou substitué une ou plusieurs fois ;
sous forme du racémate ; des énantiomères, des diastéréoisomères, de mélanges des énantiomères ou des diastéréoisomères ou d'un énantiomère ou diastéréoisomère individuel ; des bases ou de sels d'acides ou de cations physiologiquement acceptables.

2. Dérivés substitués de 3-pyrrolidine-indole suivant la revendication 1, **caractérisés en ce que** le reste R² représente un groupe COR⁵, SO2R⁶ ou CSNHR⁷.

3. Dérivés substitués de 3-pyrrolidine-indole suivant la revendication 1, **caractérisés en ce que** le reste R² est un groupe CONHR⁸.

4. Dérivés substitués de 3-pyrrolidine-indole suivant les revendications 1 à 3, **caractérisés en ce que** le reste R¹ représente H ; un reste alkyle en C₁ à C₈, dans chaque cas saturé ou non saturé, ramifié ou non ramifié, non substitué ou substitué une ou plusieurs fois ; un reste benzyle ou méthylnaphtyle, chacun substitué une ou plusieurs fois ou non substitué, en particulier un reste benzyle non substitué ou substitué une ou plusieurs fois avec un radical CF₃, Br, F, CN, OCH₃ ou CH₃ ; un groupe CH₂CONR³R⁴.

5. Dérivés substitués de 3-pyrrolidine-indole suivant les revendications 1, 2 ou 4, **caractérisés en ce que** le reste R² est un reste COR⁵ et R⁵ désigne un reste 4-propylphényle, 3,4-diméthoxyphényle, 2-méthyl-4-trifluorométhylphényl-3-pyridine, éthénylphényle, 2,3-difluorophényle, 4-tertiobutylphényle, 2-éthoxyphényle, 3-fluoro-4-trifluorométhylphényle, 2,3-diméthylphényle, phénoxyéthyle, phénoxyméthyle, 3,4-dichlorophényle, 4-trifluorométhylsulfanylphényle, 2,5-diméthoxyphényle, 2-chloro-4-nitrophényle, 2-chlorophényle, 4-méthyl-N-phénéthyl-benzylsulfonamide, 2-chloro-5-fluoro-3-méthylphényle, 3-(2-chlorophényl)-5-méthyl-isoxazole, 5-tertiobutyl-2-méthylfuranne, benzo[1,2,5]oxadiazole, phénylpropyle, 2-méthylsulfanyl-3-pyridine, 2-chloro-5-trifluorométhylphényle, méthoxyméthyle, 4-méthylphényle, biphényle, 4-chlorobenzyle, 2,3,4,5,6-pentafluorophényle, 3-chlorophényle, 4-trifluorométhylphényle, 2,6-difluoro-3-méthylphényle, 2-méthylphényle, 4-fluorophényle, 6-chlorochromène, 2-chloro-6-fluorophényle, éthylcyclopentyle, 1-(4-chlorophényl)-5-trifluorométhylpyrazole, 2,4-dichlorophényle, 2,3-diméthylphényle, 3-nitro-4-méthylphényle, 4-bromo-3-méthylphényle, (4-chlorophénoxy)-méthyle, 4-chlorophényle, 5-méthylisoxazole, 3-méthoxyphényle, 2-chlorophényl-éthényle, 2-chloro-4-fluoro-3-méthylphényle, 2-fluorophényle, 3-difluorométhylsulfanylphényle, 2-fluoro-3-chlorophényle, cyclopropylphényle, 1-phényl-5-propylpyrazole, 2,6-difluorophényle, benzo[1,3]-dioxole, 4-bromophényle, 3-chlorothiophényle, 2-, 3- ou 4-pyridine, phényle ou 3,4-difluorophényle.

6. Dérivés substitués de 3-pyrrolidine-indole suivant les revendications 1, 2 ou 4, **caractérisés en ce que** le reste R² représente SO₂R⁶ et R⁶ désigne un reste 2,4,6-diméthylphényle, 4-nitrophényle, benzyle, 4-propylphényle, 4-chlorophényle, 2-trifluorométhylphényle, 2,4-difluorophényle ou 3-trifluorométhylphényle.

7. Dérivés substitués de 3-pyrrolidine-indole suivant les revendications 1, 2 ou 4, **caractérisés en ce que** le reste R² représente CSNHR⁷ et le reste R⁷ représente un reste 3-trifluorométhylphényle.

8. Dérivés substitués de 3-pyrrolidine-indole suivant les revendications 1, 3 ou 4, **caractérisés en ce que** le reste R² est un groupe CONHR⁸ et le reste R⁸ est un reste 3,4-dichlorobenzyle.

9. Dérivés substitués de 3-pyrrolidine-indole suivant les revendications 1 à 8, choisis dans le groupe suivant
• (4-propyl-phényl)-{3-[1-(4-trifluorométhyl-benzyl)-1H-indole-3-yl]-pyrrolidine-1-yl}-méthanone
• (3,4-diméthoxy-phényl)-{3-[1-(3-méthyl-butyl)-1H-indole-3-yl]-pyrrolidine-1-yl}-méthanone
• [3-(1-butyl-1H-indole-3-yl)-pyrrolidine-1-yl]-(2-méthyl-6-trifluorométhyl-pyridine-3-yl)-méthanone
• -1-{3-[1-(3-méthoxy-benzyl)-1H-indole-3-yl]-pyrrolidine l-yl}-3-phényl-propénone
• (2,3-difluoro-phényl)-{3-[1-(4-trifluorométhyl-benzyl)-1H-indole-3-yl]-pyrrolidine-1-yl}-méthanone
• (4-tertiobutyl-phényl)-{3-[1-(3-méthyl-butyl)-1H-indole-3-yl]-pyrrolidine-1-yl}-méthanone
• {3-[1-(4-bromo-benzyl)-1H-indole-3-yl]-pyrrolidine-1-yl}-(4-propyl-phényl)-méthanone
• (2-éthoxy-phényl)-{3-[1-(3-méthoxy-benzyl)-1H-indole-3-yl]-pyrrolidine-1-yl}-méthanone
• [3-(1-éthyl-1H-indole-3-yl)-pyrrolidine-1-yl]-(3-fluoro-4-trifluorométhyl-phényl)-méthanone
• (2, 3-diméthyl-phényl) - [3- (1-éthyl-1H-indole-3-yl)-pyrrolidine-1-yl]-méthanone
• 1-{3-[1-(3-méthoxy-benzyl)-1H-indole-3-yl]-pyrrolidine-1-yl}-2-phénoxy-propane-1-one
• 2-(3-chloro-phénoxy)-1-[3-(1-éthyl-1H-indole-3-yl)-pyrrolidine-1-yl]-éthanone
• 4-{3-[1-(2-phénoxy-propionyl)-pyrrolidine-3-yl]-indole-1-ylméthyl}-benzonitrile
• N,N-diméthyl-2-{3-[1-(2,4,6-triméthyl-benzènesulfonyl)-pyrrolidine-3-yl]-indole-1-yl}-acétamide
• (3,4-dichloro-phényl)-[3-(1-éthyl-1H-indole-3-yl)-pyrrolidine-1-yl]-méthanone
• N,N-diméthyl-2-{3-[1-(3-trifluorométhyl-phénylthiocarbamoyl)-pyrrolidine-3-yl]-indole-1-yl}-indole-1-yl}-acétamide
• 3,4-dichloro-benzylamide d'acide 3-(1-diméthylcarbamoylméthyl-1H-indole-3-yl)-pyrrolidine-1-carboxylique
• [3-(1H-indole-3-yl)-pyrrolidine-1-yl]-(4-trifluorométhylsulfanyl-phényl)-méthanone
• 2-(3-{1-[2-(2,5-diméthoxy-phényl)-acétyl]-pyrrolidine-3-yl}-indole-1-yl)-N,N-diméthyl-acétamide
• (2-chloro-4-nitro-phényl)-{3-[1-(3-fluoro-benzyl)-1H-indole-3-yl]-pyrrolidine-1-yl}-méthanone
• N,N-diméthyl-2-{3-[1-(pyridine-2-carbonyl)-pyrrolidine-3-yl]-indole-1-yl}-acétamide
• cyclobutyl-[3-(1H-indole-3-yl)-pyrrolidine-1-yl]-méthanone,
• N,N-diéthyl-2-[3-(1-pentanoyl-pyrrolidine-3-yl)-indole-1-yl]-acétamide
• 3-[1-(4-nitro-benzènesulfonyl)-pyrrolidine-3-yl]-1H-indole
• 2-[3-(1-cyclopentanecarbonyl-pyrrolidine-3-yl)-indole-1-yl]-N,N-diéthyl-acétamide
• 1-[3-(1-méthyl-1H-indole-3-yl)-pyrrolidine-l-yl]-3-phénylpropénone
• (2-chloro-phényl)-[3-(1H-indole-3-yl)-pyrrolidine-1-yl]-méthanone
• [3-(1-éthyl-1H-indole-3-yl)-pyrrolidine-1-yl]-pyridine-3-yl-méthanone
• N,N-diméthyl-2-(3-{1-[3-phényl-2-(toluène-4-sulfonylamino)-propionyl]-pyrrolidine-3-yl}-indole-1-yl)-acétamide
• N,N-diméthyl-2-[3-(1-phénylméthanesulfonyl-pyrrolidine-3-yl)-indole-1-yl]-acétamide
• 4-{3-[1-(2-chloro-6-fluoro-3-méthyl-benzoyl)-pyrrolidine-3-yl]-indole-1-ylméthyl}-benzonitrile
• [3-(2-chloro-phényl)-5-méthyl-isoxazole-4-yl]-[3-(1-éthyl-1H-indole-3-yl)-pyrrolidine-1-yl]-méthanone
• 2-{3-[1-(5-tertiobutyl-2-méthyl-furanne-3-carbonyl)-pyrrolidine-3-yl]-indole-1-ylméthyl}-benzonitrile
• 4-{3-[1-(benzo[l,2,5]oxadiazole-5-carbonyl)-pyrrolidine-3-yl]-indole-1-ylméthyl}-benzonitrile
• 1-[3-(1-éthyl-1H-indole-3-yl)-pyrrolidine-1-yl]-3-phényl-propane-1-one
• 1-{3-[1-(3-méthoxy-benzyl)-1H-indole-3-yl]-pyrrolidine-1-yl}-3-phényl-propane-1-one
• [3-(1-méthyl-1H-indole-3-yl)-pyrrolidine-1-yl]-(2-méthylsulfanyl-pyridine-3-yl)-méthanone
• (2-chloro-5-trifluorométhyl-phényl)-[3-(1-éthyl-1H-indole-3-yl)-pyrrolidine-1-yl]-méthanone
• (2-chloro-4-nitro-phényl)-{3-[1-(4-trifluorométhyl-benzyl)-1H-indole-3-yl]-pyrrolidine-1-yl}-méthanone
• (2-chloro-pyridine-3-yl)-[3-(1-méthyl-1H-indole-3-yl)-pyrrolidine-1-yl]-méthanone
• 2-méthoxy-1-{3-[1-(3-méthoxy-benzyl)-1H-indole-3-yl]-pyrrolidine-1-yl}-éthanone
• {3-[1-(3-fluoro-benzyl)-1H-indole-3-yl]-pyrrolidine-1-yl}-(2-méthyl-6-trifluorométhyl-pyridine-3-yl)-méthanone
• {3-[1-(3-méthoxy-benzyl)-1H-indole-3-yl]-pyrrolidine-1-yl}-p-tolyl-méthanone
• (2-méthanesulfanyl-pyridine-3-yl)-[3-(1-prop-2-ynyl-1H-indole-3-yl)-pyrrolidine-1-yl]-méthanone
• biphényl-4-yl-[3-(1-méthyl-1H-indole-3-yl)-pyrrolidine-1-yl]-méthanone
• 2-(4-chloro-phényl)-1-{3-[1-(3-méthoxy-benzyl)-1H-indole-3-yl]-pyrrolidine-1-yl}-éthanone
• [3-(1-méthyl-1H-indole-3-yl)-pyrrolidine-1-yl]-pentafluorophényl-méthanone
• [3-(1-éthyl-1H-indole-3-yl)-pyrrolidine-1-yl]-(4-trifluorométhyl-phényl-méthanone
• N,N-diméthyl-2-{3-[1-(4-propyl-benzènesulfonyl)-pyrrolidine-3-yl]-indole-1-yl}-acétamide
• (2-chloro-4-nitro-phényl)-{3-[1-(3-méthoxy-benzyl)-1H-indole-3-yl]-pyrrolidine-1-yl}-méthanone
• 3-{3-[1-(4-chloro-benzènesulfonyl)-pyrrolidine-3-yl]-indole-1-ylméthyl}-benzonitrile
• 1-{3-[1-(3-méthoxy-benzyl)-1H-indole-3-yl]-pyrrolidine-1-yl}-3,3-diméthyl-butane-1-one
• 2-(4-chloro-phényl)-1-[3-(1-méthyl-1H-indole-3-yl)-pyrrolidine-1-yl]-éthanone
• (2,6-difluoro-3-méthyl-phényl)-{3-[1-(3-méthoxy-benzyl)-1H-indole-3-yl]-pyrrolidine-1-yl}-méthanone
• [3-(1-éthyl-1H-indole-3-yl)-pyrrolidine-1-yl]-o-tolyl-méthanone
• [3-(1-éthyl-1H-indole-3-yl)-pyrrolidine-1-yl]-(4-fluoro-phényl)-méthanone
• [3-(1-butyl-1H-indole-3-yl)-pyrrolidine-1-yl]-(6-chloro-2H-chromène-3-yl)-méthanone
• (2-chloro-6-fluoro-phényl)-[3-(1-naphtalène-2-ylméthyl-1H-indole-3-yl)-pyrrolidine-1-yl]-méthanone
• 2-{3-[1-(3-cyclopentyl-propionyl)-p yrrolidine-3-yl]-indole-1-yl}-N,N-diméthyl-acétamide
• [3-(2-chloro-phényl)-5-méthyl-isoxazole-4-yl]-{3-[1-(3-méthoxy-benzyl)-1H-indole-3-yl]-pyrrolidine-1-yl}-méthanone
• [1-(4-chloro-phényl)-5-fluorométhyl-1H-pyrazole-4-yl]-{3-[1-(3-fluoro-benzyl)-1H-indole-3-yl]-pyrrolidine-1-yl}-méthanone
• [3-(1-allyl-1H-indole-3-yl)-pyrrolidine-1-yl]-p-tolyl-méthanone
• {3-[1-(4-fluoro-benzyl)-1H-indole-3-yl]-pyrrolidine-1-yl}-pyridine-2-yl-méthanone
• (3,4-dichloro-phényl)-{3-[1-(4-trifluorométhyl-benzyl)-1H-indole-3-yl]-pyrrolidine-1-yl}-méthanone
• 1-butyl-3-[1-(2,5-diméthoxy-benzènesulfonyl)-pyrrolidine-3-yl]-1H-indole
• 1-[3-(1-allyl-1H-indole-3-yl) -pyrrolidine-1-yl]-3,3-diméthyl-butane-1-one
• 4-{3-[1-(2-méthoxy-acétyl)-pyrrolidine-3-yl]-indole-1-ylméthyl}-benzonitrile
• (2,4-difluoro-phényl)-[3-(1-éthyl-1H-indole-3-yl)-pyrrolidine-1-yl]-méthanone
• (2,3-diméthyl-phényl)-{3-[1-(4-trifluorométhyl-benzyl)-1H-indole-3-yl]-pyrrolidine-1-yl}-méthanone
• [3-(1-butyl-1H-indole-3-yl)-pyrrolidine-1-yl]-(4-méthyl-3-nitro-phényl)-méthanone
• 4-bromo-3-méthyl-phényl)-{3-[1-(3-méthoxy-benzyl)-1H-indole-3-yl]-pyrrolidine-1-yl}-méthanone
• 1-[3-(1-butyl-1H-indole-3-yl)-pyrrolidine-1-yl]-2-(4-chloro-phénoxy)-éthanone
• 2-(4-chloro-phényl)-1-{3-[1-(3-fluoro-benzyl)-1H-indole-3-yl]-pyrrolidine-1-yl}-éthanone
• (4-chloro-phényl)-{3-[1-(4-trifluorométhyl-benzyl)-1H-indole-3-yl]-pyrrolidine-1-yl}-méthanone
• [3-(1-benzyl-1H-indole-3-yl}-pyrrolidine-1-yl]-(5-méthylisoxazole-3-yl)-méthanone
• 1-[3-(1-benzyl-1H-indole-3-yl)-pyrrolidine-1-yl]-2-(4-chloro-phénoxy) -éthanone
• [3-(1-naphtalène-2-ylméthyl-1H-indole-3-yl)-pyrrolidine-1-yl]-(4-trifluorométhyl-phényl)-méthanone
• (3-méthoxy-phényl)-{3-[1-(4-trifluorométhyl-benzyl)-1H-indole-3-yl]-pyrrolidine-1-yl}-méthanone
• 1-[3-(1-butyl-1H-indole-3-yl)-pyrrolidine-1-yl]-3-(2-chloro-phényl)-propénone
• 1-[3-(1-méthyl-1H-indole-3-yl)-pyrrolidine-1-yl]-2-phényl-butane-1-one
• (2-chloro-6-fluoro-3-méthyl-phényl)-[3-(1-méthyl-1H-indole-3-yl)-pyrrolidine-1-yl]-méthanone
• 2-{3-[1-(3-méthoxy-benzoyl)-pyrrolidine-3-yl]-indole-1-yl}-N,N-diméthyl-acétamide
• 2-{3-[1-(2-fluoro-benzoyl)-pyrrolidine-3-yl]-indole-1-yl}-N,N-diméthyl-acétamide
• (3-difluorométhylsulfanyl-phényl)-[3-(1-naphtalène-2-ylméthyl-1H-indole-3-yl)-pyrrolidine-1-yl]-méthanone
• benzo[1,2,5]oxadiazole-5-yl-{3-[1-(3-fluoro-benzyl)-1H-indole-3-yl]-pyrrolidine-1-yl}-méthanone
• o-tolyl-13-[1-(4-trifluorométhyl-benzyl)-1H-indole-3-yl]-pyrrolidine-1-yl}-méthanone
• N,N-diméthyl-2-{3-[1-(2-trifluorométhyl-benzène-sulfonyl)-pyrrolidine-3-yl]-indole-1-yl}-acétamide
• 1-[3-(1-butyl-1H-indole-3-yl)-pyrrolidine-1-yl]-2-phénoxy-propane-1-one
• (6-chloro-2-fluoro-3-méthyl-phényl)-[3-(1-naphtalène-2-ylméthyl-1H-indole-3-yl)-pyrrolidine-1-yl]-méthanone
• [3-(1-allyl-1H-indole-3-yl)-pyrrolidine-1-yl]-(2-méthyl-6-trifluorométhyl-pyridine-3-yl)-méthanone
• [3-(1-butyl-1H-indole-3-yl)-pyrrolidine-1-yl]-(4-propyl-phényl)-méthanone
• 2-{3-[1-(4-chloro-benzoyl)-pyrrolidine-3-yl]-indole-1-yl}-N,N-diméthyl-acétamide
• 1-{3-[1-(3-fluoro-benzyl)-1H-indole-3-yl]-pyrrolidine-1-yl}-3-phényl-propane-1-one
• {3-[1-(2-fluoro-benzyl)-1H-indole-3-yl]-pyrrolidine-1-yl}-(1-phényl-5-propyl-1H-pyrazole-4-yl)-méthanone
• 3-{3-[1-(5-tertiobutyl-2-méthyl-furanne-3-carbonyl)-pyrrolidine-3-yl]-indole-1-ylméthyl}-benzonitrile
• 3-[1-(2,4-difluoro-benzènesulfonyl)-pyrrolidine-3-yl]-1-(2-fluoro-benzyl)-1H-indole
• 2-{3-[1-(2,6-difluoro-benzoyl)-pyrrolidine-3-yl]-indole-1-yl}-N,N-diméthyl-acétamide
• 1-[3-(1-butyl-1H-indole-3-yl)-pyrrolidine-1-yl]-butane-1-one
• 4-{3-[1-(2-propyl-pentanoyl)-pyrrolidine-3-yl]-indole-1-ylméthyl}-benzonitrile
• 2-(4-chloro-phénoxy)-1-[3-(1-prop-2-ynyl-1H-indole-3-yl)-pyrrolidine-1-yl]-éthanone
• [3-(1-méthyl-1H-indole-3-yl)-pyrrolidine-1-yl]-pyridine-3-yl-méthanone
• 3-{3-[1-(benzo[1,3]dioxole-5-carbonyl)-pyrrolidine-3-yl]-indole-1-ylméthyl}-benzonitrile
• [3-(1-butyl-1H-indole-3-yl)-pyrrolidine-1-yl]-(2-chloro-5-trifluorométhyl-phényl)-méthanone
• (3-chloro-phényl)-{3-[1-(4-trifluorométhyl-benzyl)-1H-indole-3-yl]-pyrrolidine-l-yl}-méthanone
• 2-{3-[1-(4-bromo-benzoyl)-pyrrolidine-3-yl]-indole-1-yl}-N,N-diméthyl-acétamide
• 3-phényl-1-[3-(1-prop-2-ynyl-1H-indole-3-yl)-pyrrolidine-1-yl]-propénone
• 3-{3-[1-(3-chloro-2-fluoro-benzoyl)-pyrrolidine-3-yl]-indole-1-ylméthyl}-benzonitrile
• 3-{3-[1-(2-phényl-cyclopropanecarbonyl)-pyrrolidine-3-yl]-indole-1-ylméthyl}-benzonitrile
• 3-{3-[1-(3-chloro-thiophène-2-carbonyl)-pyrrolidine-3-yl]-indole-1-ylméthyl}-benzonitrile
• 1-prop-2-ynyl-3-[1-(2-trifluorométhyl-benzènesulfonyl)-pyrrolidine-3-yl]-1H-indole
• 1-(2-fluoro-benzyl)-3-[1-(3-trifluorométhyl-benzène-sulfonyl)-pyrrolidine-3-yl]-1H-indole
• [3-(1-benzyl-1H-indole-3-yl)-pyrrolidine-1-yl]-(6-chloro-2H-chromène-3-yl)-méthanone
• [3-(1-benzyl-1H-indole-3-yl)-pyrrolidine-1-yl]-(3,4-difluoro-phényl)-méthanone
sous forme du racémate; des énantiomères, des diastéréoisomères, de mélanges des énantiomères ou des diastéréoisomères ou sous forme d'un énantiomère ou diastéréoisomère individuel ; des bases et/ou de sels d'acides ou cations physiologiquement acceptables.

10. Médicament contenant au moins un dérivé substitué de 3-pyrrolidine-indole de formule générale I, éventuellement sous forme d'un racémate, des stéréoisomères purs, en particulier des énantiomères et des diastéréoisomères, dans un rapport de mélange quelconque ; sous forme de ses acides ou de ses bases ou sous forme de ses sels, en particulier des sels physiologiquement acceptables ou de sels d'acides ou de cations physiologiquement acceptables ; ou sous forme de ses produits de solvatation, en particulier des hydrates, contenant aussi le cas échéant des additifs et/ou des substances auxiliaires convenables et/ou éventuellement d'autres substances actives.

11. Utilisation d'un dérivé substitué de 3-pyrrolidine-indole de formule générale I, le cas échéant sous forme de son racémate, des stéréoisomères purs, en particulier des énantiomères et des diastéréoisomères, dans un rapport de mélange quelconque ; sous forme de ses acides ou de ses bases ou sous forme de ses sels, en particulier des sels physiologiquement acceptables ou de sels d'acides ou de cations physiologiquement acceptables ; ou sous forme de ses produits de solvatation, en particulier des hydrates ; pour la préparation d'un médicament destiné au traitement de la douleur, en particulier d'une douleur aiguë, neuropathique ou chronique.

12. Utilisation d'un dérivé substitué de 3-pyrrolidine-indole de formule générale I, éventuellement sous forme de son racémate, des stéréoisomères purs, en particulier des énantiomères et des diastéréoisomères, dans un rapport de mélange quelconque ; sous forme de ses acides ou de ses bases ou sous forme de ses sels, en particulier des sels physiologiquement acceptables ou de sels d'acides ou de cations physiologiquement acceptables ; ou sous forme de ses produits de solvatation, en particulier des hydrates ; pour la préparation d'un médicament destiné au traitement de dépressions et/ou pour l'anxiolyse.

13. Utilisation d'un dérivé substitué de 3-pyrrolidine-indole de formule générale I, éventuellement sous forme de son racémate, des stéréoisomères purs, des énantiomères et des diastéréoisomères, dans un rapport de mélange quelconque ; sous forme de ses acides ou de ses bases ou sous forme de ses sels, en particulier des sels physiologiquement acceptables ou de sels d'acides ou de cations physiologiquement acceptables ; ou sous forme de ses produits de solvatation, en particulier des hydrates ; pour la préparation d'un médicament destiné au traitement de maladies cardio-vasculaires, de l'incontinence urinaire, de la diarrhée, du prurit, de l'abus d'alcool et de drogues, de la dépendance à des médicaments, d'inflammations, du manque de tonus, de la boulimie, de l'anorexie, de catalepsie ; destiné à être utilisé comme anesthésique local, antiarythmique, antiémétique, nootropique, et pour accroître la vigilance et la libido.

14. Procédé de production de dérivés substitués de 3-pyrrolidine-indole de formule générale I suivant une ou plusieurs des revendications 1 à 9, à partir du 3-pyrrolidine-3-yl-indole, **caractérisé en ce que**
a) on fixe un groupe protecteur sur l'azote de pyrrolidine du 3-pyrrolidine-3-yl-indole,
b) on alkyle l'azote de l'indole,
c) on élimine le groupe protecteur, et
d) on fait réagir l'azote de pyrrolidine avec un 2-chloracétamide ou un 2-bromacétamide, un chlorure d'acide, un isocyanate ou un isothiocyanate.
